(19) Europäisches Patentamt / European Patent Office / Office européen des brevets

(11) **EP 4 692 332 A1**

(12) **EUROPEAN PATENT APPLICATION**
published in accordance with Art. 153(4) EPC

(43) Date of publication:
**11.02.2026  Bulletin 2026/07**

(21) Application number: **24784910.2**

(22) Date of filing: **02.04.2024**

(51) International Patent Classification (IPC):
*C12N 5/10* (2006.01)    *C12N 5/078* (2010.01)
*C12N 15/12* (2006.01)    *C12N 15/19* (2006.01)
*C12N 15/63* (2006.01)

(52) Cooperative Patent Classification (CPC):
**C07K 14/435; C07K 14/52; C12N 5/06; C12N 5/10; C12N 15/63**

(86) International application number:
**PCT/JP2024/013612**

(87) International publication number:
**WO 2024/210126 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.04.2023  JP 2023060124**
**17.01.2024  JP 2024005230**

(71) Applicants:
• **Otsuka Pharmaceutical Co., Ltd.**
**Tokyo 101-8535 (JP)**
• **Kyoto University**
**Kyoto-shi, Kyoto 606-8501 (JP)**

(72) Inventors:
• **ETO, Koji**
**Kyoto-shi, Kyoto 606-8501 (JP)**

• **NAKAMURA, Sou**
**Kyoto-shi, Kyoto 606-8501 (JP)**
• **FUJIO, Kosuke**
**Osaka-shi, Osaka 540-0021 (JP)**
• **HARADA, Yasuo**
**Osaka-shi, Osaka 540-0021 (JP)**
• **HAYASHI, Hideki**
**Osaka-shi, Osaka 540-0021 (JP)**
• **TAGA, Ryosuke**
**Osaka-shi, Osaka 540-0021 (JP)**
• **KUWANO, Nozomi**
**Osaka-shi, Osaka 540-0021 (JP)**

(74) Representative: **Hoffmann Eitle Patent- und Rechtsanwälte PartmbB Arabellastraße 30 81925 München (DE)**

Remarks:
The complete document including Reference Table(s) and the Sequence Listing(s) can be downloaded from the EPO website

(54) **PRODUCTION METHOD FOR MEGAKARYOCYTES**

(57)    Provided is a method for producing megakaryocytes from hematopoietic progenitor cells, which comprises the step of culturing the hematopoietic progenitor cells while forcibly expressing in the hematopoietic progenitor cells an oncogene, a homeobox gene, and an apoptosis inhibitor gene. The application also provides a kit for producing megakaryocytes, mature megakaryocytes or platelets which comprising a vector having an oncogene, a homeobox gene and an apoptosis inhibitor gene.

**Description**

[Technical Field]

The present application relates to a method for producing megakaryocytes.

[Background Art]

Each platelet transfusion uses approximately 200 billion platelets. Platelets generally have a short lifespan of only a few days in the body, and therefore, repeated transfusions of platelets are often necessary. Despite the increasing number of elderly people who need repeated platelet transfusions in the aging societies, the population of young donors is on the decline.

In response to the social demand for platelets, the inventors have established a technology for *in vitro* stable platelet production from human iPS cells (Non-Patent Literatures 1, 2). The inventors introduced three factors (MYC/BMI1/BCL-XL) into hematopoietic cells derived from human iPS cells to establish an immortalized megakaryocyte line (the cells that give rise to platelets). In addition, they have also established a technology for mass production of platelets (human iPS cell-derived artificial platelet preparations) from the immortalized megakaryocytes by just changing the culture medium for the cells. (Patent Literature 1 to 4).

[Citation List]

[Patent Literature]

Patent Literature 1: WO2011/034073
Patent Literature 2: WO2012/157586
Patent Literature 3: WO2014/123242
Patent Literature 4: WO2021/075568

[Non-Patent Literature]

[Non-Patent Literature 1]: Cell Stem Cell. April 3, 2014; 144: 535-48
[Non-Patent Literature 2]: Cell. July 26, 2018; 174(3): 636-648

[Summary of Invention]

[Technical Problem]

An object of this application is to provide a method for the production of megakaryocytes.

[Solution to Problem]

The present application provides a method for producing megakaryocytes from hematopoietic progenitor cells, which comprises the step of culturing the hematopoietic progenitor cells while forcibly expressing an oncogene, a homeobox gene, and an apoptosis inhibitor gene in the hematopoietic progenitor cells. The present application also provides a method for producing megakaryocytes from hematopoietic progenitor cells, which comprises the step of culturing the hematopoietic progenitor cells a medium comprising a pyrimido-indole derivative.

The present application also provides a method for producing mature megakaryocytes, which comprises the steps of: producing megakaryocytes by the method provided herein and culturing the obtained megakaryocytes while stopping the forced expression of all or a part of the oncogene, the homeobox gene, and the apoptosis inhibitor gene.

The present application also provides a method for producing platelets, which comprises the steps of: producing mature megakaryocytes by the method provided herein, and culturing the obtained mature megakaryocytes.

The present application also provides megakaryocytes or mature megakaryocytes, wherein an exogenous oncogene, an exogenous homeobox gene, and an exogenous apoptosis inhibitor gene are integrated in their chromosome.

The present application also provides a method for producing megakaryocytes from hematopoietic progenitor cells, characterized by forcibly expressing a gene encoding a cytokine receptor or a cell growth promoting factor under the control of a promoter specific to megakaryocytes or platelets in the hematopoietic progenitor cells.

The present application also provides a kit for producing megakaryocytes, mature megakaryocytes or platelets, which comprises a vector having an oncogene, a homeobox gene, and an apoptosis inhibitor gene.

The present application also provides a kit for producing megakaryocytes, mature megakaryocytes or platelets, which comprises a vector having a gene that encodes a cytokine receptor or a cell growth promoting factor under the control of a promoter specific to megakaryocytes or platelets.

The present application also provides a kit for producing megakaryocytes, mature megakaryocytes or platelets, which comprises a vector having a gene that encodes a cytokine receptor or a cell growth promoting factor under the control of a promoter specific to megakaryocytes or platelets.

[Advantageous Effects of Invention]

By the methods presented herein, it becomes possible to produce megakaryocytes capable of generating functional platelets.

[Brief Description of Drawings]

[Figure 1A] Proliferative capacity of the megakaryocyte cell line derived from iPS cell line 585A1 in which an instability domain fused c-MYC gene (MYCdd gene), HOXA2 gene, and BCL2L1 gene were introduced.

[Figure 1B] Flow cytometry analysis of megakaryocyte cell line derived from iPS cell line 585A1. The percentages of the dots in each quadrant (from upper left to upper right to lower right to lower left) are as follows:

Day 32: 0.69, 79.6, 16.6, 3.12,
Day 66: 2.03, 79.3, 11.6, 7.10

[Figure 2A] Number of platelets produced by megakaryocyte cell line derived from iPS cell line 585A1.

[Figure 2B] The ratio of activated platelets in platelets generated from megakaryocyte cell line derived from iPS cell line 585A1. X-axis: PAC1-FITC, Y-axis: CD62P-PE.

[Figure 3] Proliferation rate of megakaryocyte cell line derived from the iPS cell line Ff-I01s01 in which MYCdd gene, HOXA2 gene, and BCL2L1 gene were introduced.

[Figure 4] Platelet production efficiency of megakaryocyte cell line derived from iPS cell line Ff-I01s01.

[Figure 5] The vector map of a vector having the human MPL gene under the control of the human CD41 promoter.

[Figure 6] The effect of introducing the MPL gene under the control of the CD41 promoter on the proliferation of megakaryocyte cell line derived from the iPS cell line Ff-101s01 in which MYCdd, BCL2L1 and HOXA2 genes were introduced.

[Figure 7] The efficiencies of platelet production of two megakaryocyte cell lines with different expression promoters for the BCL2L1 gene. The megakaryocyte cell line was derived from the iPS cell line Ff-101s01 in which MYCdd, BCL2L1 and HOXA2 genes were introduced.

[Figure 8A] The effect of UM729 on the proliferation of megakaryocyte cell lines derived from the iPS cell line Ff-I01s01. The iPS cell line Ff-I01s01 was introduced with MYCdd, BCL2L1, and HOXA2 genes, and with or without MPL gene under the control of CD41 promoter.

[Figure 8B] The effect of UM729 on the proliferation of megakaryocyte cell lines derived from the iPS cell line Ff-I01s01. The iPS cell line Ff-101s01 was introduced with MYCdd and BCL2L1 genes, and with or without MPL gene under the control of CD41 promoter.

[Figure 9A] The effect of UM729 on the platelet production efficiency of megakaryocyte cell lines derived from the iPS cell line Ff-I01s01. The iPS cell line Ff-I01s01 was introduced with MYCdd, BCL2L1, and HOXA2 genes, and with or without MPL gene under the control of CD41 promoter.

[Figure 9B] The effect of UM729 on the platelet production efficiency of megakaryocyte cell lines derived from the iPS cell line Ff-I01s01. The iPS cell line Ff-I01s01 was introduced with MYCdd and BCL2L1 genes, and with or without MPL gene under the control of CD41 promoter.

[Figure 10] Schematic diagram showing the induction of hematopoietic progenitor cells and megakaryocytes from iPS cells, along with a schematic diagram of the constructs introduced into the cells.

[Figure 11] Flow cytometry analysis of the cells collected on days 7, 11, 15, 19, and 36 of culture. The cells were stained with CD41a-APC and CD34-FITC antibodies. The number of dots (%) in each quadrant (from top left to top right to bottom right to bottom left) were as follows:

[UM729- (upper row)]

DoxON day 7: 0.096, 3.74, 87.1, 9.02,
DoxON day 11: 0.068, 7.07, 80.1, 12.8,
DoxON day 15: 0.084, 3.95, 87.0, 9.00,
DoxON day 19: 0.11, 4.14, 79.6, 16.2,
DoxON day 36: 0.019, 1.81, 87.1, 11.1

[UM729+ (lower row)]

DoxON day 7: 0.26, 17.1, 76.1, 6.57,
DoxON day 11: 0.019, 52.2, 46.2, 1.53,
DoxON day 15: 0.14, 55.8, 43.3, 0.69,
DoxON day 19: 0.25, 62.7, 34.7, 2.29,
DoxON day 36: 0.45, 29.9, 68.1, 1.61

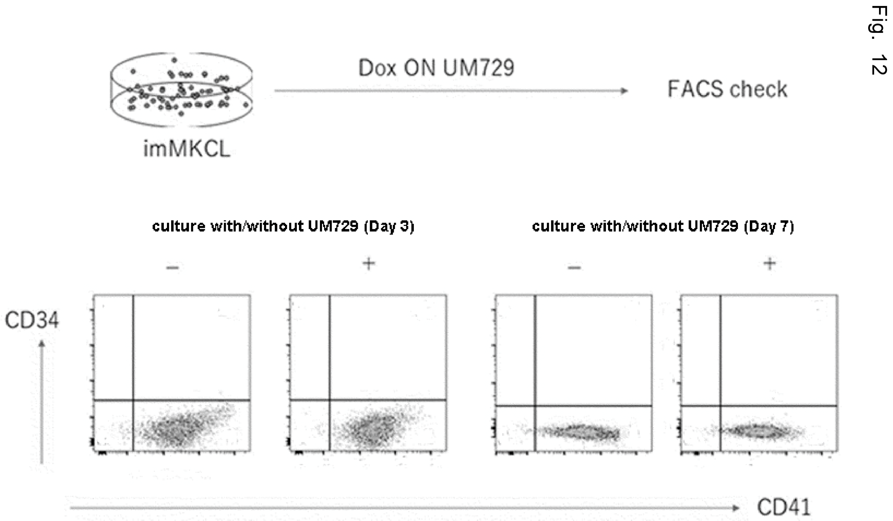

[Figure 12] Flow cytometry analysis of the cells collected on day 3 and day 7 of culture. The cells were stained with CD41a-APC and CD34-FITC antibodies. The number of dots (%) in each quadrant (from top left to top right to bottom right to bottom left) were as follows:

UM729 day 3-: 0, 0.020, 99.0, 1.00,
UM729 day 3+: 0, 0.040, 99.6, 0.36,
UM729 day 7-: 0, 0, 99.4, 0.61,
UM729 day 7+: 0, 0.020, 99.2, 0.80

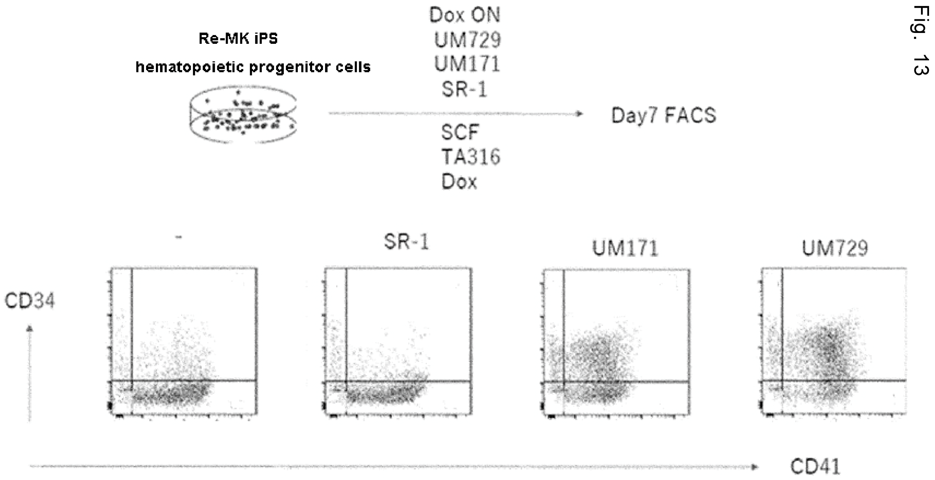

[Figure 13] Flow cytometry analysis of the cells collected on day 7 of culture. Cells were stained with CD41a-APC and CD34-FITC antibodies. The number of dots (%) in each quadrant (from top left to top right to bottom right to bottom left) were as follows:

-: 1.16, 9.04, 73.8, 16.1,
SR-1: 2.07, 5.26, 72.9, 19.7,
UM-171: 5.85, 44.5, 38.7, 10.9,
UM729: 5.1, 64.1, 21.9, 8.87

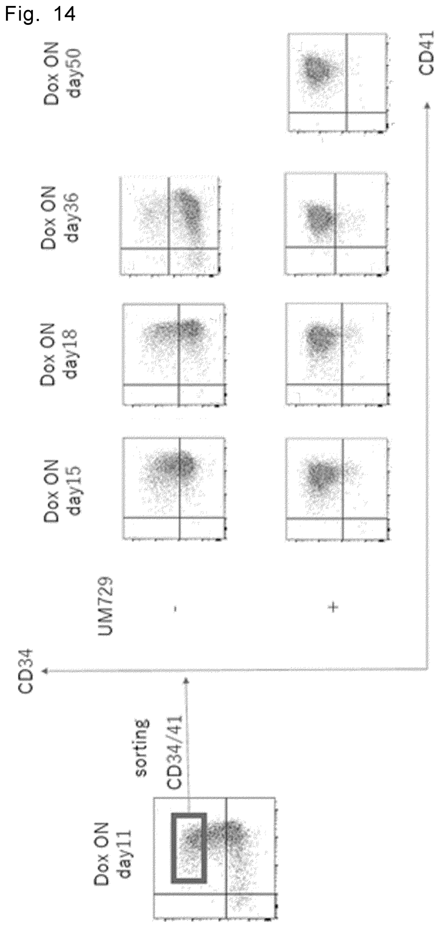

[Figure 14] Flow cytometry analysis of the cells treated with UM729. The cells were collected on day 11 of culture, stained with CD41a-APC and CD34-FITC antibodies, and CD34/41 positive population was sorted by FACS. The CD34/41 positive cells were then cultured in the presence of 1 $\mu$M of UM729. The dot counts (%) for each quadrant (from top left to top right to bottom right to bottom left) were as follows:
DoxON day 11 (leftmost): 0.019, 52.2, 46.2, 1.53

[UM729 - (upper row)]

DoxON day 15: 0.021, 46.9, 53.1, 0.041,
DoxON day 18: 0.025, 30.7, 69.1, 0.12,
DoxON day 36: 0.16, 8.58, 87.7, 3.56

[UM729 + (lower row)]

DoxON day 15: 0.061, 96.7, 3.15, 0.081,
DoxON day 18: 0.25, 97.4, 2.32, 0.021,
DoxON day 36: 0.17, 96.9, 2.88, 0.038,
DoxON day 50: 0.038, 99.6, 0.32, 0

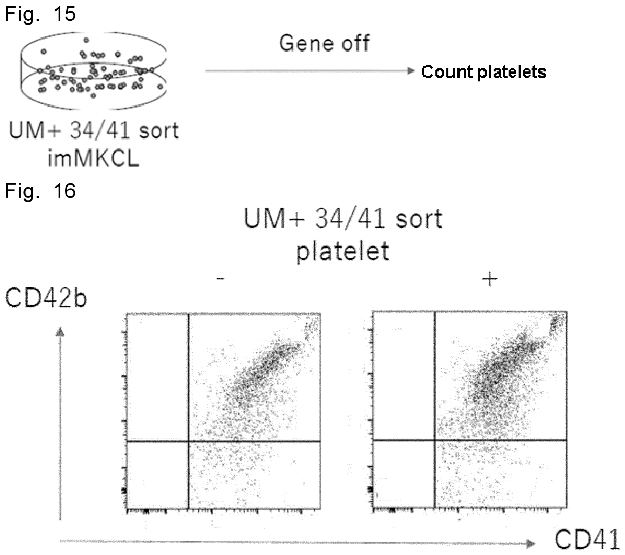

[Figure 15] Schematic representation of the procedures. The culture supernatant was collected on day 6 of megakaryocyte maturation culture and stained with CD41a-APC and CD42b-PE, and the number of platelets was counted by flow cytometry.

[Figure 16] Flow cytometry analysis to count the number of platelets in the culture supernatant obtained on day 6 megakaryocyte maturation culture stained with CD41a-APC and CD42b-PE. The dot counts (%) for each quadrant (from top left to top right to bottom right to bottom left) were as follows:

- (left): 0, 91.5, 8.34, 0.14,

    + (right): 0.018, 95.9, 4.01, 0.037

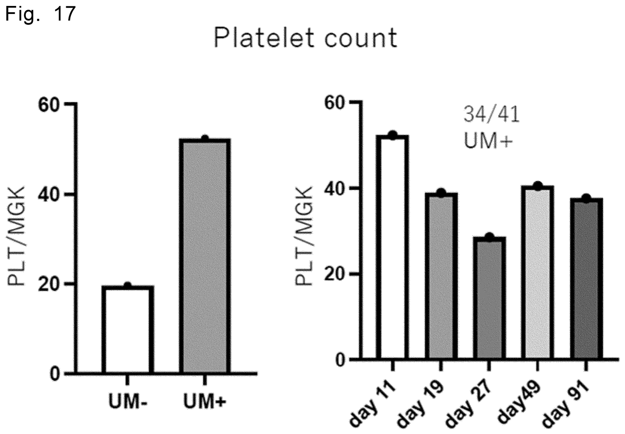

[Figure 17] The number of platelets produced and the number of platelets produced per single megakaryocyte in the culture supernatant obtained on day 6 megakaryocyte maturation culture stained with CD41a-APC and CD42b-PE.

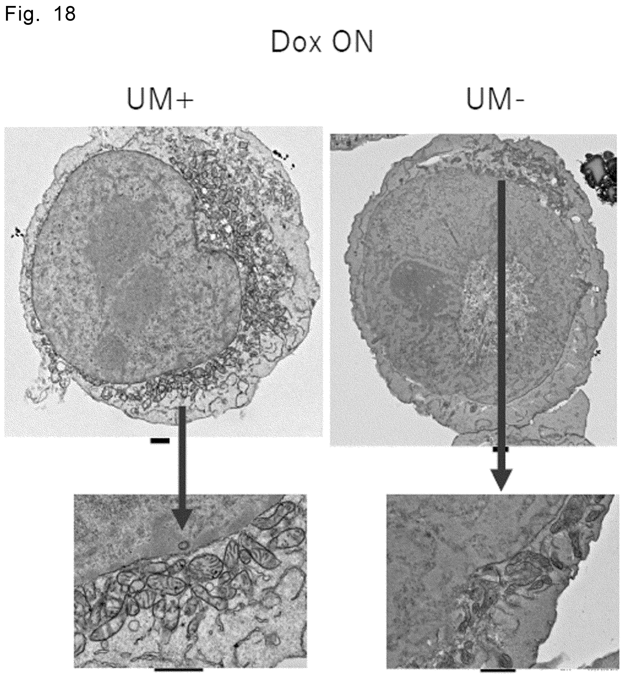

[Figure 18] Electron microscopic image of the megakaryocytes collected during the proliferation phase.

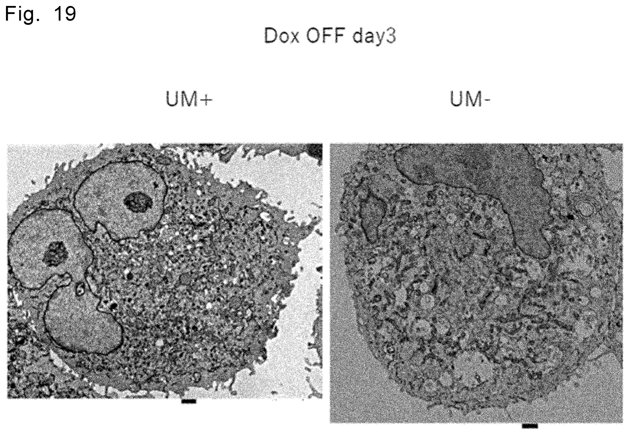

[Figure 19] Electron microscopic image of the megakaryocytes collected on day 3 of megakaryocyte maturation culture.

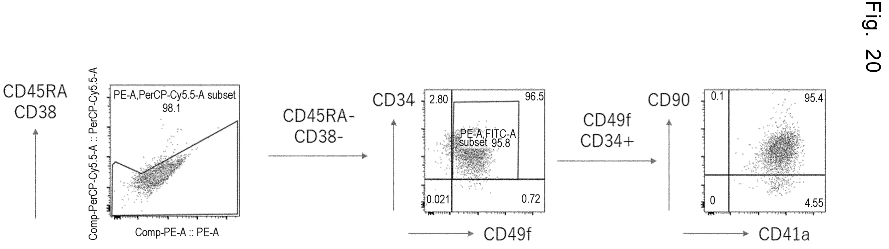

[Figure 20] Flow cytometry analysis of CD34/41 positive megakaryocytes stained with CD45RA-PerCP, CD38-PerCP, CD34-FITC, CD90-BV420, CD49f-PE, and CD41a APC.

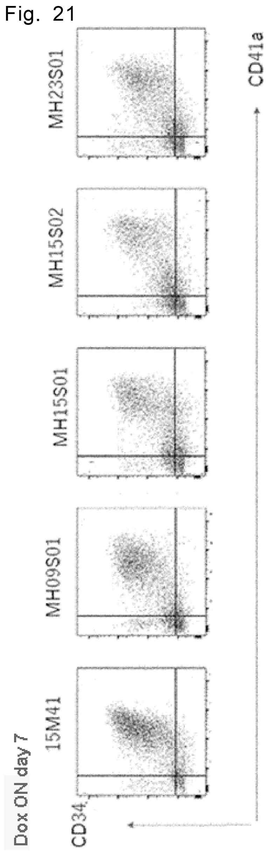

[Figure 21] Flow cytometry analysis of the cells on day 7 of megakaryocyte induction from each iPS cell line. The cells were stained with CD41a-APC and CD34-FITC antibodies. The number of dots (%) in each quadrant (from top left to top right to bottom right to bottom left) was as follows:

    15M41: 3.67, 78.7, 9.37, 8.28,
    MH09S01: 14.0, 58.7, 8.13, 19.2,
    MH15S01: 10.8, 49.3, 15.8, 24.1,
    MH15S02: 18.5, 50.4, 8.90, 22.2,
    MH23S01: 11.4, 48.0, 15.7, 24.9

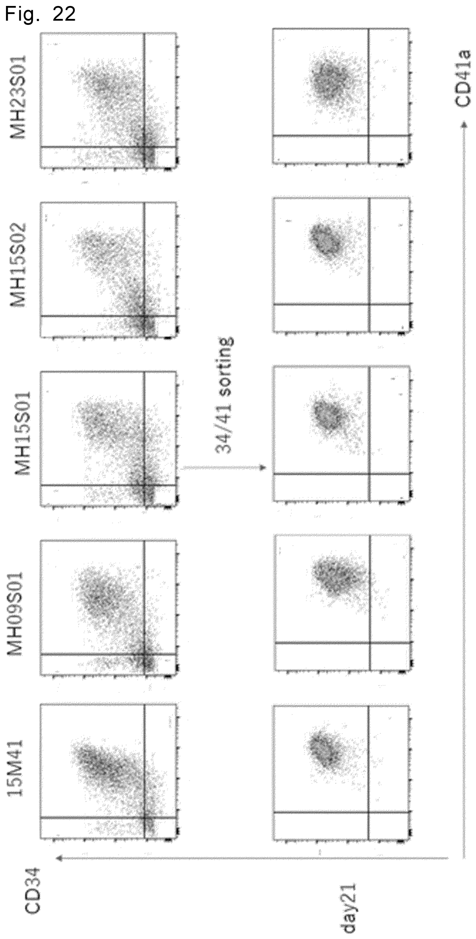

[Figure 22] Flow cytometry analysis of surface markers of the cells collected on day 21 of megakaryocyte induction from each iPS cell line. The cells were stained with CD41a-APC and CD34-FITC antibodies. The number of dots (%) in each quadrant (from top left to top right to bottom right to bottom left) was as follows:

    Top row (day 7): see Figure 21;
    Bottom row (day 21):
    15M41: 0, 99.8, 0.22, 0,
    MH09S01: 0, 99.3, 0.74, 0,
    MH15S01: 0, 99.9, 0.067, 0,
    MH15S02: 0, 99.9, 0.065, 0,
    MH23S01: 0, 99.7, 0.34, 0

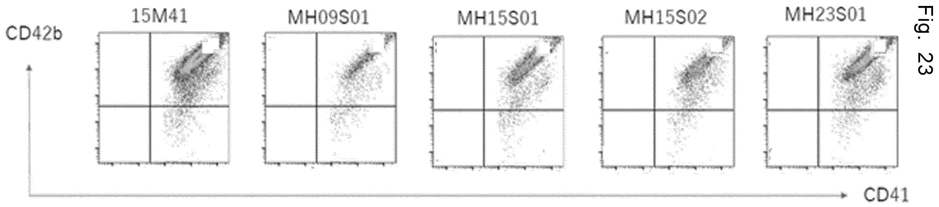

[Figure 23] Flow cytometry analysis to count the number of platelets in the culture supernatant collected on day 6 of megakaryocyte maturation culture from each iPS cell line. The culture supernatant was stained with CD41a-APC and CD42b-PE. The number of dots (%) in each quadrant (from top left to top right to bottom right to bottom left) was as follows:

    15M41: 0, 98.5, 1.49, 0,
    MH09S01: 0, 93.9, 6.11, 0,
    MH15S01: 0, 97.8, 2.25, 0,
    MH15S02: 0, 97.1, 2.89, 0,
    MH23S01: 0, 98.8, 1.20, 0)

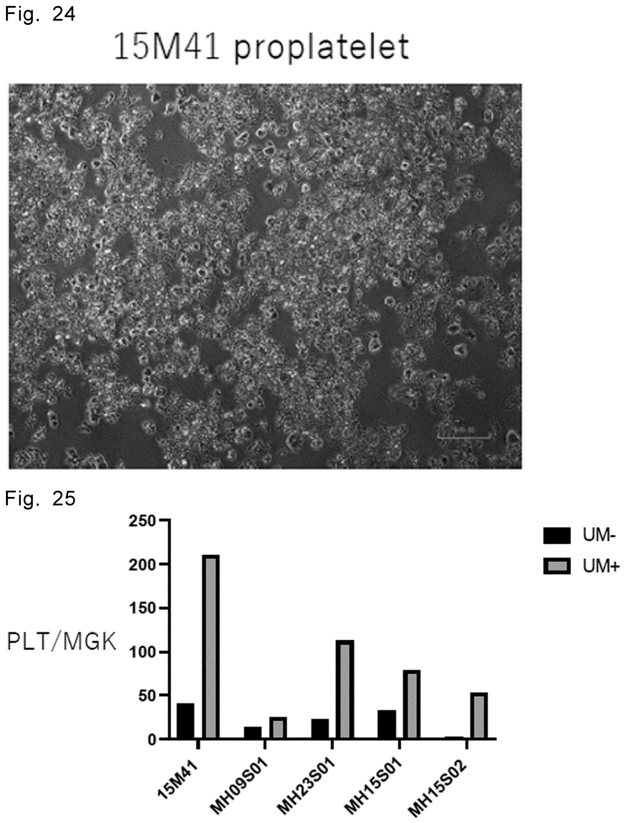

[Figure 24] Microscopic image of the platelet-producing precursors on day 6 of megakaryocytes maturation culture derived from 15M41 cells. Almost all of the cells formed platelets.
[Figure 25] The number of platelets produced per megakaryocyte in the culture supernatant stained with CD41a-APC and CD42b-PE, collected on day 6 of megakaryocyte maturation culture from each iPS cell line.

[Description of Embodiments]

In the present disclosure, when a numerical value is accompanied by the term "about", it is intended to include a range of ± 10% of that value. For example, "about 20" includes "18 to 22". The numerical range includes all numbers between and including the endpoints. The "about" relating to a range applies to the endpoints of that range. For example, "about 20 to 30" includes "18 to 33".

In the specification and claims of the present application, the expression "a certain type of cells" means a group of cells in which the specified type of cells are included, unless otherwise stated, and cells of types other than those of the specified types may be included in the group. For example, the expression "a culture of a certain type of cells" means a culture of a group of cells in which the specified type of cells are included, and cells other than those of the specified types may be included. In a similar manner, the expression "a certain type of cell population" means a cell population in which the specified type of cells is included, unless otherwise stated, and cells other than those of the specified types may be included in the cell population.

In the present application, a medium may be prepared by appropriately adding necessary factors to a basal medium used for culturing animal cells. Examples of the basal media include MEM zinc option medium, IMEM zinc option medium, IMDM medium, Medium 199 medium, Eagle's minimum essential medium (EMEM) medium, αMEM medium, Dulbecco's modified Eagle's medium (DMEM) medium, DMEM/F12 medium, Ham's F12 medium, RPMI 1640 medium, Fischer's medium, and a mixed medium thereof. The basal medium may contain serum (e.g., fetal bovine serum (FBS)), or may be serum-free. The basal medium may contain one or more serum alternatives such as albumin, transferrin, KnockOut Serum Replacement (KSR) (serum alternative in ES cell culture) (Thermo Fisher Scientific, Inc, N2 supplement (Thermo Fisher Scientific, Inc.), B27 supplement (Thermo Fisher Scientific, Inc.), fatty acids, insulin, collagen precursors, microelements, 2-mercaptoethanol, and 3'-thiol glycerol, one or more substances such as lipids, amino acids, L-glutamine, GlutaMax (Thermo Fisher Scientific, Inc.), non-essential amino acids (NEAA), vitamins, growth factors, antibiotics, antioxidants, pyruvic acid, buffer agents, inorganic salts, and equivalents thereof, or one or more other substances to be generally added to a medium for animal culture, as needed. The basal medium used in the present application may be, for example, IMDM medium.

Method for the production of megakaryocytes

The present application provides a method for producing megakaryocytes, which comprises the step of culturing hematopoietic progenitor cells while forcibly expressing an oncogene, a homeobox gene and an apoptosis.

The megakaryocyte may be characterized as a cell that is, for example, CD41a-positive/CD42a-positive/CD42b-positive. In addition to these markers, the megakaryocyte may further express one or more markers selected from the group consisting of CD9, CD34, CD61, CD62p, CD42c, CD42d, CD49f, CD51, CD110, CD123, CD131, and CD203c. The megakaryocyte may also express GATA1, FOG1, NF-E2, and β1-tubulin. The megakaryocyte may be a multinucleated cell, or it may be a mononuclear or binuclear cell. Furthermore, the megakaryocyte may be immortalized as a megakaryocyte cell line, or it may be a clonal cell population.

In this application, the production of the target cells may be confirmed by the expression of their markers, size and/or shape of the cells. The expression of the markers may be verified using known methods such as immunostaining, RT-qPCR, and flow cytometry.

Hematopoietic progenitor cells are cells that can differentiate into blood cell lineages such as lymphocytes, eosinophils, neutrophils, basophils, erythrocytes, and megakaryocytes. Hematopoietic progenitor cells may be recognized, for example, by being positive for surface antigens CD34 and/or CD43. Hematopoietic progenitor cells may be isolated from umbilical cord blood, bone marrow blood, or peripheral blood, or they may be induced by differentiating pluripotent stem cells such as embryonic stem (ES) cells or induced pluripotent stem (iPS) cells. For example, hematopoietic progenitor cells may be obtained from a sac-like structure prepared from ES cells or iPS cells (also referred to as ES-sac or iPS-sac) (Takayama N., et al. J Exp Med. 2817-2830(2010)). Here, a "sac-like structure" refers to a three-dimensional sac-like structure (one that contains internal space) derived from pluripotent stem cells, formed by a population of endothelial cells, and containing hematopoietic progenitor cells within. Additionally, methods for producing hematopoietic progenitor cells from pluripotent stem cells may comprise the formation of embryoid bodies and the addition of cytokines (Chadwick et al. Blood 2003, 102: 906-15, Vijayaragavan et al. Cell Stem Cell 2009, 4: 248-62, Saeki et al. Stem Cells 2009, 27: 59-67) or co-culture methods with stromal cells of different origins (Niwa A et al. J Cell Physiol. 2009 Nov;221(2):367-77).

In one embodiment, hematopoietic progenitor cells are differentiated from pluripotent stem cells by culturing them in a medium comprising vascular endothelial growth factor (VEGF) in the presence of feeder cells. In this process, the concentration of VEGF may range from 2 to 200 ng/mL, for example, about 20 ng/mL. The culture period may be from 10 to 20 days, for example, about 14 days. During this process, it is also possible to forcibly express an oncogene, a homeobox gene, and an apoptosis-inhibiting gene described later.

Pluripotent stem cells may be cultured by means of two-dimensional culture, three-dimensional culture, or a culture for the formation of organoids or cystic structures. For example, feeder cells may be pre-seeded on the bottom surface of a culture vessel or on a culture substrate and proliferated to the extent that the feeder cells cover the bottom surface of the culture vessel or the surface of the culture substrate. After this, pluripotent stem cells may be seeded on these feeder cells and cultured.

In this specification, "feeder cells" refer to cells other than the target cells that are co-cultured with the target cells during cultivation. The feeder cells are not specifically limited, and may be, for example, fibroblasts (such as C3H/10T1/2 cells, NIH3T3 cells, MEF cells, STO cells and SNL cells), fetal kidney cells (such as HEK293 cells), stromal cells (such as ST2 cells, OP9 cells, PA6 cells and MS-5 cells), epithelial cells (such as HeLa cells), placental cells, bone marrow cells, and endometrial cells. In this method, the feeder cells may be, for example, C3H/10T1/2 cells. The feeder cells may be derived from the same species as the target cells or from different species. Additionally, the feeder cells may be treated to inhibit cell proliferation, for example, using a proliferation inhibitor (such as mitomycin C) or by irradiation (such as X-ray or gamma ray radiation).

In the specification and claims of the present application, "pluripotent stem cells" refer to stem cells which have pluripotency, that is the ability of cells to differentiate into all types of the cells in the living body, as well as proliferative capacity. Examples of the pluripotent stem cells include embryonic stem (ES) cells (J. A. Thomson et al., (1998), Science 282: 1145-1147; J. A. Thomson et al., (1995), Proc. Natl. Acad. Sci. USA, 92: 7844-7848; J. A. Thomson et al., (1996), Biol. Reprod., 55: 254-259; J.A. Thomson and V.S. Marshall (1998), Curr. Top. Dev. Biol., 38: 133-165), embryonic stem cells derived from cloned embryo obtained by nuclear transfer (ntES cells) (T. Wakayama et al., (2001), Science, 292: 740-743; S. Wakayama et al., (2005), Biol. Reprod., 72: 932-936; J. Byrne et al., (2007), Nature, 450: 497-502), germline stem cells ("GS cells") (M. Kanatsu-Shinohara et al., (2003) Biol. Reprod., 69: 612-616; K. Shinohara et al., (2004), Cell, 119: 1001-1012), embryonic germ cells ("EG cells") (Y. Matsui et al., (1992), Cell, 70: 841-847; J. L. Resnick et al., (1992), Nature, 359: 550-551), induced pluripotent stem (iPS) cells (K. Takahashi and S. Yamanaka (2006) Cell, 126: 663-676; K. Takahashi et al., (2007), Cell, 131:861-872; J. Yu et al., (2007), Science, 318: 1917-1920; Nakagawa, M. et al., Nat. Biotechnol. 26: 101-106 (2008); WO 2007/069666), pluripotent cells derived from cultured fibroblasts and bone marrow stem cells (Muse cells) (WO 2011/007900). Mouse or human pluripotent stem cells, particularly ES cells and iPS cells are preferably used.

iPS cells may be generated by introducing specific reprogramming factors in the form of DNAs or proteins in somatic cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al, Nat. Biotechnol. 26:101-106 (2008); WO2007/069666). When iPS cells are employed, the iPS cells may be generated from somatic cells by a known method or obtained from a previously established iPS cell-stock. The somatic cells from which the iPS cells were induced are not particularly limited. For example, iPS cells induced from peripheral blood cells or umbilical code blood cells may be used.

As used herein, "oncogene" is a gene that causes the transformation of normal cells into cancerous cells due to differences in its expression, structure, or function compared to normal cells. Examples of oncogene may comprise MYC family genes, Src family genes, Ras family genes, Raf family genes, and protein kinase family genes such as c-Kit and PDGFR, as well as Abl. MYC family genes including c-MYC, N-MYC, and L-MYC. c-MYC gene may preferably be employed. c-MYC gene consists of a nucleotide sequence represented by NCBI accession number NM_002467. The c-MYC gene may be its homolog, wherein the c-MYC gene homolog is defined as a gene whose cDNA sequence is substantially identical to the nucleotide sequence represented by NCBI accession number NM_002467. The cDNA that is substantially identical to the nucleotide sequence represented by NCBI accession number NM_002467 consists of DNA that has about 60% or more, preferably 70% or more, more preferably 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity to the sequence represented by NCBI accession number NM_002467, or DNA that can hybridize under stringent conditions with a sequence complementary to the nucleotide sequence represented by NCBI accession number NM_002467. The proteins encoded by these DNAs contribute to the amplification of differentiated cells, such as hematopoietic progenitor cells.

As used herein, stringent condition refers to a hybridization condition that may be easily determined by practitioners, which is generally empirical experimental conditions dependent on the probe length, washing temperature, and salt concentration. In general, as the probe length increases, the temperature for appropriate annealing becomes higher, while as the probe length decreases, the temperature becomes lower. The formation of the hybrid generally depends on the re-annealing capability of the complementary strands in an environment slightly below their melting temperature.

An example of a low-stringent condition may comprise the step of washing the filter after hybridization at a temperature of 37°C to 42°C in a 0.1× SSC and 0.1% SDS solution. An example of a high-stringent condition may comprise the step of washing the filter at 65°C in 5× SSC and 0.1% SDS solution. By increasing the stringency of the conditions, it is possible to obtain highly homologous polynucleotides.

As used herein, "sequence identity" refers to the percentage of identical bases or amino acids at corresponding positions in two or more sequences (nucleotide sequences or amino acid sequences) when aligned considering gaps and insertions, such that the degree of match is maximized. The method for determining identity is designed to provide the maximum degree of match between the aligned sequences. Methods for determining identity between two sequences may comprise, but are not limited to, BLASTP, BLASTN, and FASTA. Additionally, it can also be determined using DNASIS (Hitachi Software Engineering Co., Ltd.) and GENETYX (Genetics Co., Ltd.). Alternatively, for short peptides, identity may be simply determined by comparing the sequences. A person skilled in the art can determine the identity between sequences using the aforementioned methods.

The c-MYC gene may be a c-MYC gene that encodes the protein fused with an instability domain. The "instability domain" refers to a domain that destabilizes a protein that is operably linked to the domain. The instability domain may be operably linked to the N-terminus or C-terminus of the protein. Examples of instability domains include ubiquitin, PEST sequences (rich in proline, glutamic acid, serine, and threonine), cyclin destruction boxes, hydrophobic stretches of amino acids, Escherichia coli dihydrofolate reductase (ecDHFR), human estrogen receptor ligand-binding domain (ERLBD), as well as FK506 binding protein (FKBP12) and its variants. The instability domain may be, for example, FKBP12 or its variants. Examples of FKBP12 variants used as instability domains include F15S, V24A, H25R, E60G, L106P, M66T, R71G, D100G, D100N, E102G, and K105I variants (Banaszynski et al., Cell 126:995 (2006)). The instability domain may utilize commercially available products, such as those sold by TAKARA Bio (Clontech ProteoTuner™ Shield System C, #631072).

The "homeobox gene" refers to a gene that contains a homeobox domain within its sequence, for example, a HOX gene. The HOX gene may be HOXA1, HOXA2, HOXA3, HOXA4, HOXA5, HOXA6, HOXA7, HOXA9, HOXA10, HOXA11, or HOXA12 gene. HOXA2 is preferably employed. HOXA2 gene is a gene composed of a nucleotide sequence represented by the NCBI accession number NM_006735. The HOXA2 gene may also be its homolog, where a homolog of HOXA2 gene is defined as a gene whose cDNA sequence is substantially identical to the nucleotide sequence represented by the NCBI accession number NM_006735. The cDNA that is substantially identical to the nucleotide sequence represented by the NCBI accession number NM_006735 may have DNA that has about 60% or more, preferably 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity to the sequence represented by the NCBI accession number NM_006735, or a DNA that can hybridize under a stringent condition with a sequence complementary to the nucleotide sequence represented by the NCBI accession number NM_006735, where the protein encoded by that DNA has effects equivalent to those of the protein encoded by the HOXA2 gene in hematopoietic progenitor cells.

The "apoptosis inhibitor gene" refers to any gene that inhibits apoptosis, without specific limitation. Examples include the BCL2 gene, the BCL2L1 gene (protein name: Bcl-xL), Survivin, and MCL1. Preferably, it is the BCL2L1 gene. The BCL2L1 gene consists of a nucleic acid sequence represented by, for example, NCBI accession numbers NM_001191 or NM_138578. The BCL2L1 gene may be its homolog, where a homolog of the BCL2L1 gene is defined as a gene whose cDNA sequence is substantially identical to the nucleic acid sequence represented by NCBI accession numbers NM_001191 or NM_138578. The cDNA that consists of a sequence substantially identical to the nucleic acid sequence represented by NCBI accession numbers NM_001191 or NM_138578 is DNA that has about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity with the sequence represented by NCBI accession numbers NM_001191 or NM_138578, or DNA that can hybridize under stringent conditions with a sequence complementary to the nucleic acid sequence represented by NCBI accession numbers NM_001191 or NM_138578, where the protein encoded by that DNA has the effect of inhibiting apoptosis.

Two or more of the oncogene, the homeobox gene, and the apoptosis inhibitor gene may be forcibly expressed simultaneously, or the genes may be forcibly expressed sequentially. In one embodiment, the forced expression of the oncogene, the homeobox gene, and the apoptosis inhibitor gene is performed simultaneously.

The procedures for forcibly expressing the aforementioned genes in hematopoietic progenitor cells may be procedures known to those skilled in the art. For example, a vector that expresses the aforementioned genes, proteins pr RNAs encoded by the genes may be introduced into the hematopoietic progenitor cells. Alternatively, a low molecular weight compound that induces the forced expression of the aforementioned genes may be brought into contact with the hematopoietic progenitor cells. The expression vector, proteins, RNAs, or low molecular weight compounds may be introduced into the hematopoietic progenitor cells multiple times.

In one embodiment, the aforementioned genes may be introduced into hematopoietic progenitor cells using a genome editing technology. Examples of genome editing technologies may comprise CRISPR system (such as CRISPR/Cas9), TALEN, and ZFN. In genome editing techniques, a targeting vector having a target gene is generally used. In a specific site of a genome in the cell, the DNA is cleaved by site-specific DNA cleavage with a nuclease, and then, an exogenous DNA may be inserted there by homologous recombination repair (homology-directed repair: HDR). The introduction of a gene into cells using genome editing technology may be performed using commercially available genome editing kits, enzymes, vectors, and so on.

In the CRISPR system, an endonuclease such as Cas9 or Cpf1 recognizes a specific nucleotide sequence known as the PAM sequence and cleaves the double-stranded target DNA through the action of the endonuclease. The gRNA contains a sequence about 20 nucleotides upstream of the PAM sequence (the target sequence) or a complementary sequence at the 5' end, serving to recruit the endonuclease to the target sequence. In the gRNA, the sequence of the portion outside the target sequence may be determined as appropriate by the practitioner depending on the endonuclease used. The gRNA may comprise the crRNA (CRISPR RNA), which contains the target sequence or a complementary sequence and is responsible for the sequence specificity of the gRNA, and the tracrRNA (Trans-activating crRNA), which forms a double strand and contributes to the formation of a complex with Cas9. The crRNA and tracrRNA may exist as separate molecules. The sequence of the gRNA may be designed using a tool available for selecting target sequences and designing gRNAs, such as CRISPRdirect (https://crispr.dbcls.jp/).

It is possible to introduce a vector having a nucleic acid sequence that encodes the gRNA and a nucleic acid sequence that encodes the endonuclease into the cells, or to introduce the gRNA and the endonuclease proteins produced outside the cells into the cells. The nucleic acid sequence that encodes the gRNA and the nucleic acid sequence that encodes the endonuclease may exist on separate vectors. Various methods may be utilized for the introduction of the vector, the gRNA, and the endonuclease, including lipofection, liposome methods, electroporation, nucleofection, calcium phosphate co-precipitation, DEAE-dextran method, microinjection, and gene gun methods.

In one embodiment, the above gene may be introduced into hematopoietic precursor cells by a transposon system. Transposon is a general term for short gene sequences that cause gene transposition and have been conserved throughout the course of evolution. The transposon system induces gene transposition through a pair of gene enzymes (transposases) and their specific recognition sequences. As a transposon system, for example, the PiggyBac™ transposon system may be used. The PiggyBac™ transposon system utilizes transposons isolated from insects (Fraser MJ et al., Insect Mol Biol. May 1996; 5(2): 141-51.; Wilson MH et al., Mol Ther. January 2007; 15(1): 139-45.), enabling high-efficiency integration into mammalian chromosomes. The PiggyBac™ transposon system is actually used for gene introduction (see, for example, Nakazawa Y, et al., J Immunother 32: 826-836, 2009; Nakazawa Y et al., J Immunother 6: 3-10, 2013). The transposon systems applicable to The present application are not limited to those utilizing PiggyBac™, and systems utilizing transposons such as Sleeping Beauty (Ivics Z, Hackett PB, Plasterk RH, Izsvak Z (1997) Cell 91: 501-510.), Frog Prince (Miskey C, Izsvak Z, Plasterk RH, Ivics Z (2003) Nucleic Acids Res 31: 6873-6881.), Toll (Koga A, Inagaki H, Bessho Y, Hori H. Mol Gen Genet. December 10, 1995; 249(4): 400-5.; Koga A, Shimada A, Kuroki T, Hori H, Kusumi J, Kyono-Hamaguchi Y, Hamaguchi S. J Hum Genet. 2007; 52(7): 628-35. Epub June 7, 2007.), and Tol2 (Koga A, Hori H, Sakaizumi M (2002) Mar Biotechnol 4: 6-11.; Johnson Hamlet MR, Yergeau DA, Kuliyev E, Takeda M, Taira M, Kawakami K, Mead PE (2006) Genesis 44: 438-445.; Choo BG, Kondrichin I, Parinov S, Emelyanov A, Go W, Toh WC, Korzh V (2006) BMC Dev Biol 6: 5.) may also be adopted.

The operation using the transposon system may be performed according to standard methods with reference to publications. For the PiggyBac™ transposon system, Nakazawa Y, et al., "J Immunother" 32:826-836, 2009; Nakazawa Y et al., "J Immunother" 6:3-10, 2013; and Saha S, Nakazawa Y, Huye LE, Doherty JE, Galvan DL, Rooney CM, Wilson MH. "J Vis Exp." 2012 Nov 5;(69):e4235, for example, describe the procedures. In one embodiment, the above gene is introduced into hematopoietic precursor cells by the PiggyBac™ transposon system. The typical procedure using the PiggyBac™ transposon system comprises preparing a vector (transposase plasmid) that carries a gene encoding the PiggyBac™ transposase and a vector (transposon plasmid; PiggyBac™ vector) that has a structure where the gene encoding the target protein is flanked by PiggyBac™ inverted repeat sequences, and introducing these two vectors into the target cells. Various methods may be utilized for the introduction, including lipofection, liposome methods, electroporation, nucleofection, calcium phosphate co-precipitation, DEAE-dextran method, microinjection, and gene gun methods.

In one embodiment, a vector expressing the aforementioned gene is introduced into hematopoietic progenitor cells. Examples of vectors expressing the aforementioned gene include, for example, viral vectors (such as retroviruses, lentiviruses, adenoviruses, adeno-associated viruses, herpesviruses, and Sendai viruses), transposon vectors, plasmid vectors, or episomal vectors. For example, a transposon vector such as the PiggyBac™ vector may be used.

To introduce multiple genes simultaneously, a polycistronic vector with genes linked vertically may be used. To enable polycistronic expression, for example, the 2A self-cleaving peptide or IRES sequence from the Thosea asigna virus, porcine teschovirus, or foot-and-mouth disease virus may be ligated between the genes that are to be expressed forcibly.

Known methods for introducing an expression vector into hematopoietic progenitor cells may comprise the use of viral vectors through infection methods, and non-viral vectors through lipofection, liposome methods, electroporation, nucleofection, calcium phosphate co-precipitation, DEAE-dextran methods, microinjection, and gene gun methods.

In one embodiment, the expression of the oncogene, the homeobox gene, and the apoptosis inhibitor gene is controlled by a system that can regulate the on/off states of gene expression. The expression of each gene may be controlled individually or collectively by a single system. Examples of such systems may be the one where expression is reversibly controlled by the presence or absence of an inducer. For example, the tetracycline (Tet) expression induction system, the cumate repressor protein CymR system, and the coumermycin/novobiocin regulatory system may be employed.

As a tetracycline (Tet) expression induction system, the Tet-On/Tet-Off ™ Gene Expression System (TAKARA Bio) is exemplified. In the Tet-On™ system, a Tet-On™ regulatory plasmid expressing the reverse tetracycline-controlled transactivator (rtTA) is used along with a plasmid encoding the Tetracycline Response Element (TRE) that has tetO repeat sequences. The rtTA is a fusion protein composed of a mutant Tet repressor protein (rTetR) and the VP16 Activation Domain (AD), which binds to the Tetracycline Response Element (TRE) upon the addition of doxycycline (Dox) to the medium, thereby inducing downstream gene expression. In the Tet-Off ™ system, gene expression is induced in the absence of Dox (Urlinger, S. et al. (2000) Proc. Natl. Acad. Sci. USA 97(14):7963-7968).

The Cumate repressor protein CymR system is a system that allows for the reversible induction of target gene expression using a lentiviral vector. This system involves the introduction of a vector for target gene expression and a vector for CymR repressor expression into cells, enabling induction through the addition of the expreinducer substance Cumate. The SparQ Cumate Switch Inducible System (from System Biosciences) may be utilized as the Cumate repressor protein CymR system.

The regulated mammalian expression system (Promega) may be used as the coumermycin/novobiocin regulation system.

Among the systems that can control the on and off of the expression of above genes, the tetracycline (Tet) expression induction system may be suitably used. In one embodiment, the expression of the oncogene, the homeobox gene, and the apoptosis inhibitor gene is controlled by the tetracycline expression induction system, and the medium for culturing hematopoietic precursor cells further comprises doxycycline. Doxycycline may be obtained commercially, for example, from LKT Labs. In this embodiment, the concentration of doxycycline may range from 1 ng/mL to 100 μg/mL, from 10 ng/mL to 100 μg/mL, or from 100 ng/mL to 10 μg/mL, for example, about 1 μg/mL.

When the introduced gene is no longer needed for expression, it may be removed from the cell. In the case of using the PiggyBac™ vector, the gene that has been integrated into the genome may be removed by the PiggyBac™ transposase. For example, the method of the present application may further include the step of removing the oncogene, the homeobox gene, and the apoptosis inhibitor gene from megakaryocytes. In one embodiment, the method of the present application may further include the step of removing the oncogene, the homeobox gene, and the apoptosis inhibitor gene that are under the control of a tetracycline expression induction system in megakaryocytes.

In one embodiment, the hematopoietic progenitor cells have an oncogene, a homeobox gene, and an apoptosis inhibitor gene under the control of a tetracycline expression induction system. In another embodiment, the hematopoietic progenitor cells have an oncogene, a homeobox gene, and an apoptosis inhibitor gene, wherein at least one of the oncogene and homeobox gene are under the control of the tetracycline expression induction system, and the apoptosis inhibitor gene is constitutively expressed. In a further embodiment, the hematopoietic progenitor cells have an oncogene, a homeobox gene, and an apoptosis inhibitor gene, wherein the oncogene is under the control of the tetracycline expression induction system, and the apoptosis inhibitor gene is constitutively expressed. In a still further embodiment, the hematopoietic progenitor cells have an oncogene, a homeobox gene, and an apoptosis inhibitor gene, wherein both of the oncogene and homeobox gene are under the control of the tetracycline expression induction system, and wherein the apoptosis inhibitor gene is constitutively expressed.

Hematopoietic progenitor cells may be induced from pluripotent stem cells that have an oncogene, a homeobox gene, and an apoptosis inhibitor gene. Alternatively, the oncogene, the homeobox gene, and the apoptosis inhibitor gene may be introduced into the cells at any stage of differentiation from pluripotent stem cells to hematopoietic progenitor cells. Examples of the means of introducing genes into pluripotent stem cells or the cells at any differentiation stage leading to hematopoietic progenitor cells may comprise the aforementioned methods. For example, the oncogene, the homeobox gene, and the apoptosis inhibitor gene may be introduced into the cells at any differentiation stage from pluripotent stem cells to hematopoietic progenitor cells using a transposon system. The transposon system may be, for example, the PiggyBac™ transposon system.

In one embodiment, the expression of the oncogene, the homeobox gene, and the apoptosis inhibitor gene in the pluripotent stem cells or in the cells at any differentiation stage from pluripotent stem cells to hematopoietic progenitor cells is controlled by a system that can regulate the on and off states of gene expression. The aforementioned system may be used as a system that can control the on and off states of gene expression. In particular, the tetracycline (Tet) gene expression induction system may be preferably used. For example, pluripotent stem cells or cells at any differentiation stage from pluripotent stem cells to hematopoietic progenitor cells comprise the oncogene, the homeobox gene, and the apoptosis inhibitor gene under the control of the tetracycline expression induction system.

That is, the present disclosure provides a method for producing megakaryocytes from pluripotent stem cells, which comprises the following steps of:

differentiating pluripotent stem cells having an oncogene, a homeobox gene, and an apoptosis inhibitor gene under the control of the tetracycline expression induction system, into hematopoietic progenitor cells, and culturing the obtained hematopoietic progenitor cells while forcibly expressing the oncogene, the homeobox gene, and the apoptosis inhibitor gene in the presence of doxycycline.

In another embodiment, the pluripotent stem cells have an oncogene, a homeobox gene, and an apoptosis inhibitor gene, wherein at least one of the oncogene and the homeobox gene is under the control of a tetracycline expression induction system, and wherein the apoptosis inhibitor gene is constitutively expressed. In a further embodiment, the pluripotent stem cells have an oncogene, a homeobox gene, and an apoptosis inhibitor gene, wherein the oncogene is under the control of a tetracycline expression induction system, and wherein the apoptosis inhibitor gene is constitutively expressed. In yet another embodiment, the pluripotent stem cells comprise an oncogene, a homeobox gene, and an apoptosis inhibitor gene, wherein both of the oncogene and homeobox gene are under the control of a tetracycline expression induction system, and wherein the apoptosis inhibitor gene is constitutively expressed.

That is, the present application provides a method for producing megakaryocytes from pluripotent stem cells, comprising the steps of:
differentiating pluripotent stem cells having an oncogene, a homeobox gene, and an apoptosis inhibitor gene into hematopoietic progenitor cells, wherein at least one of the oncogene and homeobox gene is under the control of the tetracycline expression induction system, and the apoptosis inhibitor gene is constitutively expressed, and
culturing the obtained hematopoietic progenitor cells while forcibly expressing the oncogene, the homeobox gene, and the apoptosis inhibitor gene in the presence of doxycycline.

The present disclosure also provides a method for producing megakaryocytes from pluripotent stem cells, comprising the steps of:

differentiating pluripotent stem cells having an oncogene, a homeobox gene, and an apoptosis inhibitor gene into hematopoietic progenitor cells, wherein the oncogene is under the control of the tetracycline expression induction system, and the apoptosis inhibitor gene is constitutively expressed, and
culturing the obtained hematopoietic progenitor cells while forcibly expressing the oncogene, the homeobox gene, and the apoptosis inhibitor gene in the presence of doxycycline.

The present disclosure also provides a method for producing megakaryocytes from pluripotent stem cells, comprising the steps of:

differentiating pluripotent stem cells having an oncogene, a homeobox gene, and an apoptosis inhibitor gene into hematopoietic progenitor cells, wherein the oncogene and the homeobox gene are under the control of the tetracycline expression induction system, and the apoptosis inhibitor gene is constitutively expressed, and
culturing the obtained hematopoietic progenitor cells under the presence of doxycycline to force expression of the oncogene, the homeobox gene, and the apoptosis inhibitor gene.

In one embodiment, the oncogene, the homeobox gene, and the apoptosis inhibitor gene are introduced into the pluripotent stem cells by the transposon system. The transposon system may be, for example, the PiggyBac™ transposon system.

In a further embodiment, a gene encoding a cytokine receptor or a cell growth promoting factor is further forcibly expressed under the control of a promoter specific to megakaryocytes or platelets in the hematopoietic precursor cells.

The promoter specific to megakaryocytes or platelets is a promoter of a gene that is specifically expressed in megakaryocytes or platelets. The promoter specific to megakaryocytes or platelets may be a promoter of genes that encodes proteins selected from the group consisting of CD41a, CD42a, CD42b, CD42c, CD42d, CD9, CD61, CD62P, GATA1, GPVI, NF-E2, β-tubulin, TSP1, and Platelet Factor 4, for example, the CD41 promoter.

The CD41 promoter is a gene consisting of the nucleic acid sequence from position 60549 to 61495 of GenBank accession number AC007722.9 (SEQ ID NO: 1). The CD41 promoter may be its homolog, and the CD41 promoter homolog refers to a gene whose cDNA sequence is composed of a sequence that is substantially identical to the nucleic acid sequence shown in SEQ ID NO: 1. The cDNA that consists of a sequence substantially identical to the nucleic acid sequence shown in SEQ ID NO: 1 is DNA that has about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity to the sequence shown in SEQ ID NO: 1, or DNA that can hybridize under a stringent condition with a DNA consisting of a sequence complementary to the nucleic acid sequence shown in SEQ ID NO: 1, which has CD41 promoter activity.

The cytokine receptor or cell growth promoting factor is a factor that promotes the proliferation of megakaryocytes, and it may be a gene that encodes a receptor that regulates cell proliferation via the JAK signaling pathway. Examples of genes that encode receptors regulating cell proliferation through the JAK signaling pathway include the MPL gene, genes encoding Type I cytokine receptors such as IL-2, IL-4, IL-6, IL-7, IL-9, IL-15, IL-21, GM-CSF, and erythropoietin, and genes encoding Type II cytokine receptors such as IFN-α, IFN-β, IFN-γ, IL-10, IL-20, IL-22, and IL-28. In one embodiment, the gene that encodes the cytokine receptor or cell growth promoting factor is the MPL gene.

The MPL gene is a gene consisting of a nucleic acid sequence represented by the NCBI accession number NM_005373.3 (SEQ ID NO:2). The MPL gene may also be its homolog, and the MPL gene homolog refers to a gene whose cDNA sequence is composed of a sequence that is substantially identical to the nucleic acid sequence represented by the NCBI accession number NM_005373.3. The cDNA that consists of a sequence substantially identical to the nucleic acid sequence represented by the NCBI accession number NM_005373.3 is DNA that has about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity with the sequence represented by the NCBI accession number NM_005373.3, or DNA that can hybridize under stringent conditions with a DNA consisting of a sequence complementary to the nucleic acid sequence represented by the NCBI accession number NM_005373.3.

The gene that encodes a cytokine receptor or cell growth promoting factor may be linked downstream of a promoter specific to megakaryocytes or platelets, so that they are under the control of the promoter. The gene that encodes the cytokine receptor or cell growth promoting factor may be directly linked downstream of the promoter specific to megakaryocytes or platelets, or it may be linked via a linker. The number of nucleotides constituting the linker is not particularly limited, but may be, for example, 2 or more, 3 or more, 4 or more, 5 or more, or 6 or more. The upper limit of the number of nucleotides constituting the linker is not particularly limited, but may be, for example, 30 or less, 25 or less, 20 or less, 19 or less, 18 or less, 17 or less, 16 or less, or 15 or less. The number of nucleotides constituting the linker may range from 2 to 30, from 2 to 25, from 2 to 20, from 3 to 19, or from 4 to 18, and may be about 12.

The method for forcibly expressing a gene that encodes a cytokine receptor or a cell growth promoting factor under the control of a promoter specific to megakaryocytes or platelets is not particularly limited. For example, a vector that expresses a gene encoding a cytokine receptor or a cell growth promoting factor may be introduced into hematopoietic progenitor cells under the control of a promoter specific to megakaryocytes or platelets. The vector that expresses the gene encoding the cytokine receptor or the cell growth promoting factor, as well as the means for introducing the vector into the hematopoietic progenitor cells, may be the aforementioned vector and means. Alternatively, the gene encoding the cytokine receptor or the cell growth promoting factor may be introduced into the hematopoietic progenitor cells by a transposon system or genome editing technology. Examples of the transposon system and genome editing technology are as mentioned above.

Genes encoding the promoters specific to megakaryocytes or platelets as well as genes encoding the cytokine receptor or the cell growth promoting factor may be incorporated into a vector that expresses the oncogene, homeobox gene, and/or apoptosis inhibitor gene, or they may be incorporated into a separate vector.

Hematopoietic progenitor cells may be those induced from pluripotent stem cells in which a vector expressing genes encoding a cytokine receptor or a cell growth promoting factor under the control of a promoter specific to megakaryocytes or platelets has been introduced. The aforementioned methods may be used as means to introduce genes into the pluripotent stem cells.

In a further embodiment, the expression of TP53 gene (protein name: p53) and/or CDKN1A gene (protein name: p21) in hematopoietic progenitor cells, or the function of their expression products, may be suppressed.

TP53 gene may be the one represented by the NCBI accession number NM_000546.6. TP53 gene may be its homolog, and a TP53 gene homolog refers to a gene whose cDNA sequence is composed of a sequence that is substantially identical to the nucleic acid sequence represented by the NCBI accession number NM_000546.6. The cDNA that consists of a sequence substantially identical to the nucleic acid sequence represented by the NCBI accession number NM_000546.6 is DNA that has about 60% or more, preferably 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity with the sequence represented by the NCBI accession number NM_000546.6, or DNA that can hybridize under stringent conditions with a sequence complementary to the nucleic acid sequence represented by the NCBI accession number NM_000546.6, wherein the proteins encoded by these DNAs are those that suppress cancer.

CDKN1A gene may be the one represented by the NCBI accession number NM_001291549.3. CDKN1A gene may be its homolog, and a CDKN1A gene homolog refers to a gene whose cDNA sequence is substantially identical to the nucleic acid sequence represented by the NCBI accession number NM_001291549.3. The cDNA that consists of a sequence substantially identical to the nucleic acid sequence represented by the NCBI accession number NM_001291549.3 is DNA that has about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity with the sequence represented by the NCBI accession number NM_001291549.3, or DNA that can hybridize under stringent conditions with a sequence complementary to the nucleic acid sequence represented by the NCBI accession number NM_001291549.3, wherein the proteins encoded by these DNAs are those that suppress cancer.

The suppression of gene expression or the inhibition of the function of the expression product may be carried out by a method known in this field. For example, small interfering RNA (siRNA), short hairpin RNA (shRNA), antisense nucleic acid, or expression vector capable of expressing said nucleic acid molecule may be introduced into the cells to specifically suppress gene expression. Alternatively, the gene may be knocked out using genome editing technologies such as the CRISPR-Cas system. In one embodiment, p53 shRNA and p21 shRNA are forcibly expressed in hematopoietic progenitor cells.

SiRNA is typically a double-stranded oligo RNA consisting of an RNA strand (the antisense strand) that has a nucleotide sequence complementary to that of the target gene's mRNA or a portion of it, and its complementary strand (the sense strand). The nucleotide sequences of these RNAs may be designed appropriately by a skilled person based on the sequence information of the gene whose expression is to be suppressed. The antisense strand does not need to be completely complementary to the target gene as long as the expression inhibition effect on the target mRNA is achieved.

shRNA is a single-stranded RNA that consists of an RNA strand (the antisense strand) having a nucleotide sequence complementary to the nucleotide sequence of the target gene's mRNA or a portion thereof, and its complementary strand (the sense strand) linked by a short spacer sequence. The nucleotide sequence of the shRNA may be designed appropriately by a skilled person based on the sequence information of the gene whose expression is to be suppressed. The antisense strand does not need to be completely complementary to the target gene as long as the expression inhibition effect on the target mRNA is achieved.

Antisense nucleic acid refers to a nucleic acid sequence that contains a nucleotide sequence capable of specifically hybridizing with the target mRNA (mature mRNA or primary transcript) under the physiological conditions of the cell expressing the target mRNA, and that can inhibit the translation of the polypeptide encoded by the target mRNA while in a hybridized state. The types of antisense nucleic acids may be either DNA or RNA, or a chimera of DNA and RNA. The nucleotide sequence of the antisense nucleic acid may be designed appropriately by a person skilled in the art based on the sequence information of the gene whose expression is to be suppressed.

The nucleotide molecules that constitute siRNA, shRNA, and antisense nucleic acids may comprise various chemical modifications to enhance their stability and activity. For example, to prevent degradation by hydrolytic enzymes such as nucleases, the phosphate residues in the molecule may be replaced with chemically modified phosphate residues such as phosphorothioate (PS), methyl phosphonate, or phosphorodithioate. Additionally, at least a portion of the molecule may be composed of nucleic acid analogs such as Peptide Nucleic Acid (PNA).

Compounds that are known to suppress gene expression may be used. For example, as p53 inhibitors, Pifithrin-$\alpha$, Natrin-3, ReACp53, and RG7388 are known.

Alternatively, in order to suppress gene expression or the function of an expression product, the target gene may be knocked out using known techniques. "Gene knockout" refers to the destruction or mutation of all or a part of a gene so that it does not exhibit its original function. A gene may be destroyed or mutated such that one of the alleles on the genome does not function. Additionally, multiple alleles may be destroyed or mutated. Knockout may be performed using known methods, such as introducing a DNA construct designed to induce genetic recombination with the target gene into cells, or utilizing genome editing technologies like the TALEN or CRISPR-Cas systems to achieve knockout through the introduction of insertions, deletions, or substitutions of bases.

Compounds that suppress the transcription of a gene and the transcription product, or substances that inhibit the binding of the produced protein to its target protein, for example a p53 binding inhibitor such as Pifithrin-$\alpha$, Natrin-3, ReACp53 and RG7388; a p21 binding inhibitor such as UC2288, Butyrolactone I, LLW10, Sorafenib and Sterigmatocystin may be used.

In one embodiment, hematopoietic progenitor cells may be cultured in a medium comprising a pyrimido-indole derivative. Examples of pyrimido-indole derivatives may comprise UM171 (1r,4r)-N1-(2-benzyl-7-(2-methyl-2H-tetrazole-5-yl)-9H-pyrido[4,5-b]indole-4-yl)cyclohexane-1,4-diamine, and UM729 (methyl 4-(3-(1-piperidyl)propylamino)-9H-pyrido[4,5-b]indole-7-carboxylate). The pyrimido-indole derivative may be a pyrido[4,5-b]indole derivative, such as UM171 or UM729.

The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

The megakaryocytes produced in this embodiment may be CD34-positive CD41-positive cells. The megakaryocytes produced in this embodiment may also be CD38-negative, CD90-positive, and/or CD49f-positive.

The megakaryocytes produced in this embodiment may have a higher mitochondrial content compared to the control megakaryocytes. The control megakaryocytes may be megakaryocytes produced by culturing hematopoietic progenitor cells in a medium that does not contain the pyrimido-indole derivative, and may be, for example, CD34-negative CD41-positive cells.

The megakaryocytes produced in this embodiment may also have a higher intracellular granule content compared to the control megakaryocytes. The control megakaryocytes may be megakaryocytes produced by culturing hematopoietic progenitor cells in a medium that does not contain pyrimido-indole derivatives, and may, for example, be CD34-negative CD41-positive cells.

The pyrimido-indole derivative may be added to the culture medium when culturing hematopoietic progenitor cells, or may be added during the culture of the pluripotent stem cells or the cells at any differentiation stage from pluripotent stem cells to hematopoietic progenitor cells. For example, the cells at a differentiation stage from pluripotent stem cells to hematopoietic progenitor cells may be cultured in a culture medium comprising pyrimido-indole derivatives.

In one embodiment, the hematopoietic progenitor cells may be cultured in suspension culture for inducing differentiation into megakaryocytes. In this application, "suspension culture" refers to the cultivation of cells in a non-adhesive state in a culture dish. While not specifically limited, it may be performed using a culture dish that has not been artificially treated to enhance adhesion, for example, coating with extracellular matrix, or a culture dish that has been artificially treated to suppress adhesion, for example, coating with polyhydroxyethyl methacrylate (poly-HEMA) or the polymer of 2-methacryloyloxyethyl phosphorylcholine (Lipidure)). For example, commercially available products such as 96-well low-adhesion plates (Sumitomo Bakelite) and 35 mm low-adhesion dishes (Sumitomo Bakelite) may be employed.

The hematopoietic progenitor cells may be cultured in the presence of feeder cells or in the absence of feeder cells (feeder-free). In one embodiment, the hematopoietic progenitor cells are cultured in the absence of feeder cells.

The culture period is not particularly limited, and it may be at least 6 days, 12 days, 18 days, 24 days, 30 days, 36 days, 42 days, 48 days, 54 days, or 60 days or more. A longer culture period is not considered problematic in the production of megakaryocytes. During the culture period, the cells may be appropriately sub-cultured. The megakaryocytes provided in this application may be maintained for a long time through subculturing the cells under a condition where the oncogene, the homeobox gene, and the apoptosis inhibitor gene are expressed, or a condition where the oncogene and the homeobox gene are expressed. In this disclosure, megakaryocytes that are subcultured under such conditions are also referred to as immortalized megakaryocytes (imMKCL).

The culture temperature is not limited and may be between about 30 and 40°C, for example, about 37°C. The cultivation is carried out in an atmosphere containing carbon dioxide ($CO_2$), and the $CO_2$ concentration may be between about 0.05 and 15%, around 3 to 7%, or about 4 to 6%, for example, about 5%.

The present application also provides a method for producing megakaryocytes from pluripotent stem cells, comprising the steps of:

culturing pluripotent stem cells in a medium comprising VEGF in the presence of feeder cells, and
culturing the obtained cells while forcibly expressing an oncogene, a homeobox gene, and an apoptosis inhibitor gene.

The present application also provides a method for producing megakaryocytes from pluripotent stem cells, comprising the steps of:

culturing the pluripotent stem cells in a medium comprising VEGF in the presence of feeder cells,
culturing the obtained cells while forcibly expressing an oncogene, homeobox gene, and apoptosis inhibitor gene in a medium comprising VEGF in the presence of feeder cells, and
culturing the obtained culture while forcibly expressing the oncogene, the homeobox gene, and the apoptosis inhibitor gene in the absence of feeder cells.

The present application also provides megakaryocytes wherein an exogenous oncogene, homeobox gene, and apoptosis inhibitor gene are integrated into their chromosome. The megakaryocytes may be produced by the method of the present application. Examples of the oncogene, the homeobox gene, and the apoptosis inhibitor gene are as mentioned above. In one embodiment, the megakaryocytes are CD34-positive CD41-positive cells. The megakaryocytes may also be CD38-negative, CD90-positive, and/or CD49f-positive. In one embodiment, the megakaryocytes have a higher mitochondrial content and/or intracellular granule content compared to control megakaryocytes. The control megakaryocytes may be megakaryocytes produced by culturing hematopoietic progenitor cells in a medium that does not contain pyrimido-indole derivatives, and may be, for example, CD34-negative CD41-positive cells.

Method for producing mature megakaryocytes

The present application also provides a method for producing mature megakaryocytes, which comprises the step of: culturing megakaryocytes obtained by the method of the present application while stopping the forced expression of all or a part of the oncogene, the homeobox gene, and the apoptosis inhibitor gene. In one aspect, the forced expression of at least one of the oncogene and the homeobox gene is stopped. In another aspect, the forced expression of the oncogene is stopped. In another aspect, the forced expression of both the oncogene and the homeobox gene is stopped. In another aspect, the forced expression of all of the oncogene, the homeobox gene, and the apoptosis inhibitor gene is stopped.

Mature megakaryocytes are fully differentiated megakaryocytes that have undergone extensive multinucleation and are capable of releasing platelets. Compared to hematopoietic progenitor cells, multinucleated mature megakaryocytes exhibit an increase in the relative number of nuclei. For example, when a hematopoietic progenitor cell has 2N nuclei, cells with 4N or more nuclei become multinucleated megakaryocytes.

In one embodiment, the expression of an oncogene, a homeobox gene, and an apoptosis inhibitor gene is controlled by a system that can regulate the on and off states of gene expression. Such a system includes the aforementioned system, preferably the tetracycline (Tet) expression induction system. When the tetracycline (Tet) expression induction system is used, forced expression may be stopped by removing doxycycline from the medium. In one embodiment, the expression of the oncogene, the homeobox gene, and the apoptosis inhibitor gene is controlled by the tetracycline expression induction system, and the medium in the process of culturing mature megakaryocytes does not contain doxycycline.

Alternatively, the forced expression of each gene may be stopped by removing the oncogene, the homeobox gene, and the apoptosis inhibitor gene from the cells. When using the PiggyBac™ vector, genes that have been integrated into the genome may be removed by the PiggyBac™ transposase. In one embodiment, this method further comprises the step of removing the oncogene, the homeobox gene, and the apoptosis inhibitor gene under the control of the tetracycline-inducible expression system from the mature megakaryocytes.

In a further embodiment, a gene encoding a cytokine receptor or a cell growth promoting factor is forcibly expressed under the control of a promoter specific to megakaryocytes or platelets. Examples of the promoter specific to megakaryocytes or platelets and the cytokine receptor or cell growth promoting factor are as mentioned above. Methods for forcibly expressing a gene encoding a cytokine receptor or a cell growth promoting factor under the control of a promoter specific to megakaryocytes or platelets can include those mentioned above.

In a further embodiment, the expression of TP53 gene and/or CDKN1A gene, or the function of their expression products, may be suppressed in the megakaryocyte. The method for suppressing the expression of the gene or the function of its expression products is as described above. In one embodiment, p53 shRNA and p21 shRNA are forcibly expressed in the megakaryocyte.

In one embodiment, the megakaryocytes may be cultured in a medium comprising a pyrimido-indole derivative. The aforementioned pyrimido-indole derivatives may be used, such as UM171 or UM729. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

The mature megakaryocytes produced in this embodiment may be CD34-positive and CD41-positive cells. The mature megakaryocytes produced in this embodiment may also be CD38-negative, CD90-positive, CD49f-positive, and/or CD201-positive.

The mature megakaryocytes produced in this embodiment may have a higher mitochondrial content compared to the control mature megakaryocytes. The control mature megakaryocytes may be megakaryocytes produced by culturing the megakaryocytes in a medium that does not contain pyrimido-indole derivatives, and may, for example, be CD34-negative CD41-positive cells.

The mature megakaryocytes produced in this embodiment may have a higher intracellular granule content compared to the control mature megakaryocytes. The control mature megakaryocytes may be megakaryocytes produced by culturing the megakaryocytes in a medium that does not contain pyrimido-indole derivatives, and may, for example, be CD34-negative CD41-positive cells.

The megakaryocytes may be cultured in suspension. The megakaryocytes may also be cultured in the presence of feeder cells or in the absence of feeder cells (feeder-free). In one embodiment, the megakaryocytes are cultured in the absence of feeder cells.

The cultivation period is not particularly limited, and may be about 2 to 9 days, for example.

The culture temperature is not particularly limited and may be between about 30 and 40°C, for example, about 37°C. The culture is conducted under an atmosphere containing carbon dioxide ($CO_2$), with the $CO_2$ concentration potentially ranging from about 0.05 to 15%, about 3 to 7%, or about 4 to 6%, for example, around 5%.

The present application also provides a method for producing mature megakaryocytes from pluripotent stem cells, which comprises the steps of:

differentiating pluripotent stem cells into hematopoietic progenitor cells,
culturing the obtained hematopoietic progenitor cells while forcibly expressing an oncogene, homeobox gene, and an apoptosis inhibitor gene in the hematopoietic progenitor cells, and
culturing the obtained culture while stopping the forced expression of all or a part of the oncogene, the homeobox gene, and the apoptosis inhibitor gene.

The pluripotent stem cells may have the oncogene, the homeobox gene, and the apoptosis inhibitor gene. For example, the oncogene, the homeobox gene, and the apoptosis inhibitor gene may be under the control of a tetracycline expression induction system. Alternatively, at least one of the oncogene and the homeobox gene may be under the control of the tetracycline expression induction system, while the apoptosis inhibitor gene may be constitutively expressed. In one embodiment, the oncogene may be under the control of the tetracycline expression induction system, while the apoptosis inhibitor gene may be constitutively expressed. In another embodiment, both the oncogene and te homeobox gene may be under the control of the tetracycline expression induction system, while the apoptosis inhibitor gene may be constitutively expressed.

The present application also provides a method for producing mature megakaryocytes from pluripotent stem cells, which comprises the steps of:

culturing the pluripotent stem cells in a medium comprising VEGF in the presence of feeder cells,
culturing the obtained culture while forcibly expressing an oncogene, a homeobox gene, and an apoptosis inhibitor gene in the cells, and
culturing the obtained culture while stopping the forced expression of all or a part of the oncogene, the homeobox gene, and the apoptosis inhibitor gene.

The present application also provides a method for producing mature megakaryocytes from pluripotent stem cells, which comprises the steps of:

culturing the pluripotent stem cells in a medium comprising VEGF in the presence of feeder cells,
culturing the obtained cells while forcibly expressing an oncogene, a homeobox gene, and an apoptosis inhibitor gene in the obtained cells in the presence of feeder cells in a medium comprising VEGF,
culturing the obtained culture in the absence of feeder cells while forcibly expressing the oncogene, the homeobox gene, and the apoptosis inhibitor gene, and
culturing the obtained culture while stopping the forced expression of all or a part of the oncogene, the homeobox gene, and the apoptosis inhibitor gene.

The present application also provides mature megakaryocytes, wherein an exogenous oncogene, a homeobox gene, and an apoptosis inhibitor gene are integrated in their chromosome. The mature megakaryocytes may be produced by the method of the present application. Examples of the oncogene, the homeobox gene, and the apoptosis inhibitor gene are as mentioned above. In one embodiment, the mature megakaryocytes are CD34-positive CD41-positive cells. The mature megakaryocytes may also be CD38-negative, CD90-positive, and/or CD49f-positive. In one embodiment, the mature megakaryocytes have a higher mitochondrial content and/or intracellular granule content compared to control mature megakaryocytes. The control mature megakaryocytes may be megakaryocytes produced by culturing megakaryocytes in a medium that does not contain pyrimido-indole derivatives, and may, for example, be CD34-negative CD41-positive cells.

Method for producing platelets

The present application also provides a method for producing platelets, which comprises the step of culturing mature megakaryocytes obtained by the method of the present application.
In one embodiment, a gene encoding a cytokine receptor or a cell growth-promoting factor is forcibly expressed in mature megakaryocytes under the control of a promoter specific for megakaryocytes or platelets. Examples of promoters specific to megakaryocytes or platelets and examples of cytokine receptors or cell growth promoting factors are as described above. The methods described above can be used to forcibly express genes encoding cytokine receptors or cell growth promoting factors under the control of promoters specific to megakaryocytes or platelets.
In a further embodiment, the expression of TP53 gene and/or CDKN1A gene, or the function of their expression products, may be suppressed in mature megakaryocytes. The method for suppressing the expression of the gene or the function of its expression products is as described above. In one embodiment, p53 shRNA and p21 shRNA are forcibly expressed in mature megakaryocytes.
In one embodiment, mature megakaryocytes may be cultured in a medium comprising a pyrimido-indole derivative. The aforementioned pyrimido-indole derivatives may be used, such as UM171 or UM729. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.
The culture condition is not particularly limited, and a suitable known medium or an equivalent medium that is appropriate for producing platelets from mature megakaryocytes may be used. The culture period is not specifically limited as long as the function of the generated platelets is maintained, and it is typically around 2 to 9 days.
Mature megakaryocytes may be cultured in suspension. The culture may be conducted while stirring or shaking. Stirring may be performed, for example, using a commercially available rotary shaker culture system that rotates the culture container in an arc shape. The stirring speed may be between 50 to 200 rpm, for example, about 100 rpm. The mature megakaryocytes may be cultured in the presence of feeder cells or in the absence of feeder cells (feeder-free). In one embodiment, the mature megakaryocytes are cultured in the absence of feeder cells.
The cultivation temperature is not limited to the following and may be between about 30 and 40°C, for example, about 37°C. The cultivation is carried out in an atmosphere containing carbon dioxide ($CO_2$), and the $CO_2$ concentration may be between about 0.05 and 15%, around 3 to 7%, or about 4 to 6%, for example, about 5%.
The present application also provides a method for producing platelets from pluripotent stem cells, comprising the steps of:

differentiating the pluripotent stem cells into hematopoietic progenitor cells,

culturing the obtained hematopoietic progenitor cells while forcibly expressing an oncogene, a homeobox gene, and an apoptosis inhibitor gene in the cells,

culturing the obtained culture while stopping the forced expression of all or a part of the oncogene, the homeobox gene, and the apoptosis inhibitor gene, and culturing the obtained culture.

Here, the pluripotent stem cells may have the oncogene, the homeobox gene, and the apoptosis inhibitor gene. For example, the oncogene, the homeobox gene, and the apoptosis inhibitor gene may be under the control of a tetracycline expression induction system. Alternatively, at least one of the oncogene and the homeobox gene may be under the control of a tetracycline expression induction system, while the apoptosis inhibitor gene may be constitutively expressed. In another embodiment, the oncogene may be under the control of a tetracycline expression induction system, while the apoptosis inhibitor gene may be constitutively expressed. In a further embodiment, both the oncogene and the homeobox gene may be under the control of a tetracycline expression induction system, while the apoptosis inhibitor gene may be constitutively expressed.

The present application also provides a method for producing platelets from pluripotent stem cells, which comprises the steps of:

culturing the pluripotent stem cells in the presence of feeder cells in a medium comprising VEGF.

culturing the obtained cells while forcibly expressing an oncogene, a homeobox gene and an apoptosis inhibitor gene in the cells.

culturing the obtained culture while stopping the forced expression of all or a part of the oncogene, the homeobox gene, and the apoptosis inhibitor gene, and

culturing the obtained culture.

The present application also provides a method for producing platelets from pluripotent stem cells, comprising the steps of:

culturing pluripotent stem cells in a medium comprising VEGF in the presence of feeder cells,

culturing the obtained culture while forcibly expressing an oncogene, a homeobox gene and an apoptosis inhibitor gene in the presence of feeder cells in a medium comprising VEGF,

culturing the obtained culture while forcibly expressing an oncogene, a homeobox gene and an apoptosis inhibitor gene in the obtained cells and culturing the cells in the presence of feeder cells in a medium comprising VEGF,

culturing the obtained culture in the absence of feeder cells while forcibly expressing the oncogene, the homeobox gene, and the apoptosis inhibitor gene,

culturing the obtained culture while stopping the forced expression of all or a part of the oncogene, the homeobox gene, and the apoptosis inhibitor gene and

culturing the obtained culture.

The platelets obtained by the method of the present application may be utilized as a platelet preparation. The platelets obtained by the method of the present application may be suspended in a solution comprising as a main ingredient, human plasma, an infusion solution, citrate-containing physiological saline, or glucose-acetate Ringer's solution, or in Platelet Additive Solution (PAS) (Gulliksson, H. et al., Transfusion, 32: 435-440, (1992)) to provide a platelet preparation and stored. The storage period may be about 3 to 7 days, for example, about 4 days. As for the storage conditions, it is desirable to store it at room temperature (20-24 degrees Celsius) with gentle agitation.

Method for producing megakaryocytes, mature megakaryocytes, and platelets (1)

The present application also provides a method for producing megakaryocytes from hematopoietic progenitor cells, characterized by forcibly expressing genes encoding a cytokine receptor or a cell growth promoting factor under the control of a megakaryocyte or platelet-specific promoter. Examples of the megakaryocyte or platelet-specific promoters, genes encoding cytokine receptors or cell growth promoting factors, and means for forcibly expressing the genes are as described above. The hematopoietic progenitor cells may be differentiated from pluripotent stem cells. The differentiation of hematopoietic progenitor cells from pluripotent stem cells may utilize the aforementioned means or known methods.

The production of megakaryocytes from hematopoietic progenitor cells may utilize the aforementioned methods or known methods. Known methods include, for example, culturing hematopoietic progenitor cells with the forced expression of one gene selected from the group consisting of genes that suppress the expression of oncogenes (such as MYC family genes), the p16 gene or the p19 gene, genes that suppress the expression of the Ink4a/Arf gene, and a polycomb gene (such as the BMI1 gene) (WO2011/034073, WO2012/157586); the process of culturing hematopoietic progenitor cells with the forced expression of an apoptosis inhibitor gene (such as the BCL2L1 gene) and an oncogene (such as the c-Myc gene) (WO2014/123242); and the process of culturing hematopoietic progenitor cells with the forced expression of one gene selected from the group consisting of genes that suppress the expression of the p16 gene or the p19 gene, genes that suppress the expression of the Ink4a/Arf gene, a polycomb gene (such as the BMI1 gene), apoptosis inhibitor genes (such as the BCL2L1 gene), and oncogenes (such as MYC family genes) (WO2014/123242). Examples of oncogenes and apoptosis inhibitor genes are as mentioned above.

One gene selected from the group consisting of genes that suppress the expression of the p16 gene or the p19 gene, genes that suppress the expression of the Ink4a/Arf gene, and a polycomb gene includes, for example, BMI1, Mel18, Ring1a/b, Phc1/2/3, Cbx2/4/6/7/8, Ezh2, Eed, Suz12, HDAC, Dnmt1/3a/3b, among others, with the BMI1 gene being preferred. The BMI1 gene is a gene consisting of a nucleic acid sequence represented by NCBI accession number NM_005180. The BMI1 gene may be its homolog, and a homolog of the BMI1 gene refers to a gene whose cDNA sequence is substantially identical to the nucleic acid sequence represented by NCBI accession number NM_005180. The cDNA consisting of a sequence substantially identical to the nucleic acid sequence represented by NCBI accession number NM_005180 is DNA that has about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% sequence identity with the sequence represented by NCBI accession number NM_005180, or DNA that can hybridize under stringent conditions with a sequence complementary to the nucleic acid sequence represented by NCBI accession number NM_005180, wherein the protein encoded by that DNA suppresses oncogene-induced cellular senescence occurring in cells expressing oncogenes such as MYC family genes and promotes the amplification of those cells.

The present application also provides a method for producing mature megakaryocytes from those obtained by the aforementioned method. Mature megakaryocytes can be produced from the megakaryocytes using the aforementioned method or a known method. Examples of known methods include the method described in WO2011/034073, which involves culturing megakaryocytes obtained by suppressing the expression of an oncogene (e.g., a MYC family gene) and genes that suppress the expression of p16 and p19, as well as one gene selected from a group of genes that suppress the expression of Ink4a/Arf and Polycomb (e.g., BMI1) by stopping forced expression. Other examples include the method described in WO2012/157586, which involves culturing megakaryocytes obtained by forcing the expression of apoptosis-suppressor genes (e.g., BCL2L1), and the method described in WO2014/123242, which involves culturing megakaryocytes obtained by stopping the forced expression of an apoptosis-suppressor gene (e.g., BCL2).The L1 gene and oncogenes, such as the c-Myc gene, are involved. The method described in WO2014/123242 involves culturing megakaryocytes obtained by stopping the forced expression of a gene selected from a group that includes genes suppressing the expression of the p16 or p19 gene, the Ink4a/Arf gene, a polycomb gene (such as the BMI1 gene), apoptosis inhibitor genes (such as the BCL2L1 gene), and oncogenes (such as a MYC family gene).

In one embodiment, a gene encoding a cytokine receptor or a cell growth-promoting factor is forcibly expressed in megakaryocytes under the control of a promoter specific to megakaryocytes or platelets. Examples of promoters specific to megakaryocytes or platelets and examples of cytokine receptors or cell growth promoting factors are as described above. The methods described above can be used to forcibly express gene encoding a cytokine receptor or a cell growth promoting factor under the control of promoters specific to megakaryocytes or platelets.

The present application also provides a method for producing platelets from the mature megakaryocytes obtained by the aforementioned method. The production of platelets from the mature megakaryocytes may utilize the aforementioned means or known methods.

In one embodiment, a gene encoding a cytokine receptor or a cell growth promoting factor is forcibly expressed under the control of a promoter specific to megakaryocytes or platelets in mature megakaryocytes. Examples of the promoter specific to megakaryocytes or platelets and the cytokine receptor or cell growth promoting factor are as mentioned above. Methods for forcibly expressing a gene encoding a cytokine receptor or a cell growth promoting factor under the control of a promoter specific to megakaryocytes or platelets can include those mentioned above.

In one aspect, the present application provides a method for producing megakaryocytes characterized by the forced expression of a gene encoding a cytokine receptor or a cell growth promoting factor under the control of a promoter specific to megakaryocytes or platelets in hematopoietic progenitor cells, which comprises the step of culturing the hematopoietic progenitor cells with the forced expression of an apoptosis-inhibiting gene and an oncogene.

Examples of the genes encoding the promoter specific to megakaryocytes or platelets, a cytokine receptor, or cell a proliferation-promoting factor, as well as an apoptosis inhibitor gene and an oncogene, may comprise those mentioned above.

The gene encoding the cytokine receptor or cell growth promoting factor, the apoptosis inhibitor gene, and the oncogene may be forcibly expressed simultaneously, or each gene may be forcibly expressed sequentially. In one embodiment, the forced expression of the gene encoding the cytokine receptor or cell growth promoting factor, the apoptosis inhibitor gene, and the oncogene is performed simultaneously. In another embodiment, the forced expression of the gene encoding the cytokine receptor or cell growth promoting factor, the apoptosis inhibitor gene, and the oncogene is performed sequentially.

Methods for forcibly expressing the aforementioned genes in hematopoietic progenitor cells may comprise the methods described above or those known to those skilled in the art. For example, a gene encoding a cytokine receptor or a cell growth promoting factor may be introduced into hematopoietic progenitor cells via vectors that express the gene under the control of promoters specific to megakaryocytes or platelets. The vectors that express a gene encoding cytokine receptor or cell growth promoting factor, as well as the means for introducing the vector into hematopoietic progenitor cells, may comprise the aforementioned vectors and means.

An apoptosis inhibitor gene and an oncogene may be introduced into hematopoietic progenitor cells via a transposon system. The aforementioned system may be used as the transposon system, for example, the PiggyBac™ transposon system may be employed. In one embodiment, the apoptosis inhibitor gene and oncogene are introduced into hematopoietic progenitor cells using the PiggyBac™ transposon system. The methods described above may be utilized for the introduction. In another embodiment, a vector expressing the apoptosis inhibitor gene and oncogene is introduced into the hematopoietic progenitor cells. The vectors for expressing the apoptosis inhibitor gene and oncogene can include the aforementioned vectors, such as transposon vectors like the PiggyBac™ vector. The methods for introducing the expression vector into the hematopoietic progenitor cells may be the methods described above.

The above genes may be incorporated into the same vector or into separate vectors. To introduce multiple genes simultaneously, a polycistronic vector with genes linked vertically may be used. To enable polycistronic expression, for example, the 2A self-cleaving peptide or IRES sequence of Thosea asigna virus, porcine teschovirus, or foot-and-mouth disease virus may be ligated between the genes that are to be forcibly expressed.

The above gene may be introduced into hematopoietic progenitor cells using a genome editing technology. Examples of genome editing technologies include the CRISPR system (such as CRISPR/Cas9), TALEN, and ZFN. The introduction of genes into cells using genome editing technology may be performed using commercially available genome editing kits, enzymes, vectors, and so on.

In one embodiment, the expression of the apoptosis inhibitor gene and the oncogene is controlled by a system that can regulate the on and off states of gene expression. The aforementioned system may be used as a system that can control the on and off states of gene expression. In particular, the tetracycline (Tet) gene expression induction system may be preferably used. In one embodiment, the expression of the apoptosis inhibitor gene and the oncogene is controlled by the tetracycline expression induction system, and the medium for culturing hematopoietic precursor cells further contains doxycycline. Doxycycline may be obtained commercially, for example, from LKT Labs. In this embodiment, the concentration of doxycycline can range from 1 ng/mL to 100 μg/mL, from 10 ng/mL to 100 μg/mL, or from 100 ng/mL to 10 μg/mL, and is about 1 μg/mL, for example.

When the introduced gene is no longer needed for expression, it may be removed from the cell. In the case of using the PiggyBac™ vector, the gene that has been integrated into the genome may be removed by the PiggyBac™ transposase. For example, this method may further include the step of removing the apoptosis inhibitor gene and oncogene in the megakaryocyte. In one embodiment, this method may further include the step of removing the apoptosis inhibitor gene and oncogene under the control of the tetracycline expression induction system in the megakaryocyte.

In a further embodiment, the expression of TP53 gene (protein name: "p53") and/or CDKN1A gene (protein name: "p21") in hematopoietic progenitor cells, or the function of their expression products, may be suppressed. Methods for suppressing the expression of TP53 gene and/or CDKN1A gene, or the function of their expression products, may be the methods described above.

In one embodiment, the hematopoietic progenitor cells have an apoptosis inhibitor gene and an oncogene under the control of a tetracycline expression induction system. In another embodiment, the hematopoietic progenitor cells have an apoptosis inhibitor gene and an oncogene, where the oncogene is under the control of the tetracycline expression induction system, and the apoptosis inhibitor gene is constitutively expressed.

The hematopoietic progenitor cells may be induced from pluripotent stem cells that have genes encoding a cytokine receptor or a cell growth promoting factor, anti-apoptotic gene, and oncogene. Alternatively, the genes encoding cytokine receptor or cell growth promoting factor, anti-apoptotic gene, and oncogene may be introduced in cells at any stage of differentiation from pluripotent stem cells to hematopoietic progenitor cells. The means of introducing genes into pluripotent stem cells or cells at any differentiation stage leading to hematopoietic progenitor cells may comprise those mentioned above. For example, the gene encoding cytokine receptor or cell growth promoting factor may be introduced into cells via a vector that expresses the gene under the control of a promoter specific to megakaryocytes or platelets. The vector expressing the gene encoding cytokine receptor or cell growth promoting factor and the means of introducing this vector into hematopoietic progenitor cells may comprise the aforementioned vector and means. The anti-apoptotic genes and oncogenes may be introduced into any cell at a differentiation stage from pluripotent stem cells to hematopoietic progenitor cells using a transposon system. The transposon system may be, for example, the PiggyBac™ transposon system.

In one embodiment, the expression of apoptosis inhibitor gene and oncogene in pluripotent stem cells or in the cells at any differentiation stage from pluripotent stem cells to hematopoietic progenitor cells is controlled by a system that can regulate the on and off states of gene expression. The aforementioned system may be used as a system that can control the on and off states of gene expression. In particular, the tetracycline (Tet) gene expression induction system may suitably be used. For example, pluripotent stem cells or cells at a differentiation stage from pluripotent stem cells to hematopoietic progenitor cells may have apoptosis inhibitor gene and oncogene under the control of the tetracycline expression induction system.

That is, the present application provides a method for producing megakaryocytes from pluripotent stem cells, which comprises the steps of:

differentiating pluripotent stem cells having a gene encoding a cytokine receptor or a cell growth promoting factor under the control of a promoter specific to megakaryocyte or platelet and an apoptosis inhibitor gene and an oncogene under the control of tetracycline expression induction system into hematopoietic progenitor cells, and
culturing the obtained hematopoietic progenitor cells while forcibly expressing the gene encoding a cytokine receptor or a cell growth promoting factor, the apoptosis inhibitor gene and the oncogene in the presence of doxycycline.

In another embodiment, the pluripotent stem cells have a gene encoding a cytokine receptor or a cell growth promoting factor, an apoptosis inhibitor gene, and an oncogene, wherein the gene encoding a cytokine receptor or a cell growth promoting factor is under the control of a promoter specific to megakaryocytes or platelets, the oncogene is under the control of a tetracycline expression induction system, and the apoptosis inhibitor gene is constitutively expressed.

The present application provides a method for producing megakaryocytes from pluripotent stem cells, which comprises the steps of:

differentiating pluripotent stem cells having a gene encoding a cytokine receptor or a cell growth promoting factor, an apoptosis inhibitor gene and an oncogene into hematopoietic progenitor cells, wherein the gene encoding a cytokine receptor or a cell growth promoting factor under the control of a promoter specific to megakaryocytes or platelets, the oncogene is under the control of the tetracycline expression induction system and the apoptosis inhibitor gene is constitutively expressed, and
culturing the obtained hematopoietic progenitor cells while forcibly expressing the gene encoding a cytokine receptor or a cell growth promoting factor, the apoptosis inhibitor gene and the oncogene in the presence of doxycycline.

In one embodiment, hematopoietic progenitor cells may be cultured in a medium comprising a pyrimido-indole derivative. The aforementioned pyrimido-indole derivatives may be used, such as UM171 or UM729. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

The megakaryocytes produced in this embodiment may be CD34-positive CD41-positive cells. The megakaryocytes produced in this embodiment may also be CD38-negative, CD90-positive, and/or CD49f-positive.

The megakaryocytes produced in this embodiment may have a higher mitochondrial content compared to the control megakaryocytes. The control megakaryocytes may be megakaryocytes produced by culturing hematopoietic progenitor cells in a medium that does not comprise pyrimido-indole derivatives, and may, for example, be CD34-negative CD41-positive cells.

The megakaryocytes produced in this embodiment may also have a higher intracellular granule content compared to the control megakaryocytes. The control megakaryocytes may be those that are produced by culturing hematopoietic progenitor cells in a medium that does not contain pyrimido-indole derivatives, and may, for example, be CD34-negative CD41-positive cells.

The pyrimido-indole derivative may be added to the culture medium when culturing hematopoietic progenitor cells, or it may be added during the culture of cells at any differentiation stage from pluripotent stem cells to hematopoietic progenitor cells. For example, cells at any differentiation stage from pluripotent stem cells to hematopoietic progenitor cells may be cultured in a culture medium comprising pyrimido-indole derivatives.

In one embodiment, hematopoietic progenitor cells may be cultured in suspension during the differentiation induction into megakaryocytes. The hematopoietic progenitor cells may be cultured in the presence of feeder cells or in the absence of feeder cells (feeder-free). The culture period is not particularly limited, and may be, for example, at least more than 6 days, more than 12 days, more than 18 days, more than 24 days, more than 30 days, more than 36 days, more than 42 days, more than 48 days, more than 54 days, or more than 60 days. A longer culture period is not considered problematic in the production of megakaryocytes. During the culture period, it is desirable to perform subculturing as appropriate.

The cultivation temperature is not limited to the following, and may be between about 30 and 40°C, for example, about 37°C. The cultivation is carried out in an atmosphere containing carbon dioxide ($CO_2$), and the CO2 concentration may be between about 0.05 and 15%, about 3 to 7%, or about 4 to 6%, for example, about 5%.

In another aspect, the present application provides a method for producing mature megakaryocytes, which comprises the steps of producing megakaryocytes by the aforementioned method; and culturing the obtained megakaryocytes while stopping the forced expression of apoptosis inhibitor genes and/or oncogenes. The aforementioned method may be used as a method for stopping the forced expression of apoptosis inhibitor genes and/or oncogenes.

In one embodiment, the expression of TP53 gene and/or CDKN1A gene, or the function of their expression products, may be suppressed in the megakaryocyte. The method for suppressing the expression of the gene or the function of its expression products is as described above. In one embodiment, p53 shRNA and p21 shRNA are forcibly expressed in the megakaryocyte.

In one embodiment, the megakaryocyte may be cultured in a medium comprising a pyrimido-indole derivative. The aforementioned pyrimido-indole derivatives may be used, such as UM171 or UM729. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

The mature megakaryocytes produced in this embodiment may be CD34-positive and CD41-positive cells. The mature megakaryocytes produced in this embodiment may also be CD38-negative, CD90-positive, and/or CD49f-positive.

The mature megakaryocytes produced in this embodiment may have a higher mitochondrial content compared to the control mature megakaryocytes. The control mature megakaryocytes may be megakaryocytes produced by culturing the megakaryocytes in a medium that does not contain pyrimido-indole derivatives, and may, for example, be CD34-negative CD41-positive cells.

The mature megakaryocytes produced in this embodiment may have a higher intracellular granule content compared to the control mature megakaryocytes. The control mature megakaryocytes may be megakaryocytes produced by culturing the megakaryocytes in a medium that does not contain pyrimido-indole derivatives, and may, for example, be CD34-negative CD41-positive cells.

The megakaryocytes may be cultured in suspension. The megakaryocytes may also be cultured in the presence of feeder cells or in the absence of feeder cells (feeder-free). In one embodiment, the megakaryocytes are cultured in the absence of feeder cells. The culture period is not particularly limited, but is, for example, about 2 to 9 days.

The cultivation temperature is not limited and may be between about 30 and 40°C, for example, about 37°C. The cultivation is carried out in an atmosphere containing carbon dioxide ($CO_2$), and the CO2 concentration may be between about 0.05 and 15%, about 3 to 7%, or about 4 to 6%, for example, about 5%.

In a further aspect, a method for producing platelets is provided, which comprises the steps of producing mature megakaryocytes by the aforementioned method; and culturing the obtained mature megakaryocytes.

In one embodiment, the expression of TP53 gene and/or CDKN1A gene, or the function of their expression products, may be suppressed in mature megakaryocytes. The method for suppressing the expression of the gene or the function of its expression products is as described above. In one embodiment, p53 shRNA and p21 shRNA are forcibly expressed in the megakaryocytes.

In one embodiment, mature megakaryocytes may be cultured in a medium comprising a pyrimido-indole derivative. The aforementioned pyrimido-indole derivatives may be used, such as UM171 or UM729. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

The culture conditions are not particularly restricted, and a suitable known medium or an equivalent medium that is appropriate for producing platelets from mature megakaryocytes may be used as needed. The culture period is not specifically limited as long as the function of the platelets is maintained, but it is between about 2 to 9 days.

Mature megakaryocytes may be cultured in suspension. The culture of the cells may be performed while stirring or shaking. Stirring may be carried out, for example, using a commercially available rotary shaker culture system to rotate the culture container in an arc shape. The stirring speed may be between 50 to 200 rpm, for example, about 100 rpm. The mature megakaryocytes may be cultured in the presence of feeder cells or in the absence of feeder cells (feeder-free). In one embodiment, the mature megakaryocytes are cultured in the absence of feeder cells.

The cultivation temperature is not limited and may be between about 30 and 40°C, for example, about 37°C. The cultivation is carried out in an atmosphere containing carbon dioxide ($CO_2$), and the $CO_2$ concentration may be between about 0.05 and 15%, between about 3 and 7%, or between about 4 and 6%, for example, about 5%.

In another aspect, the present application also provides a method for producing megakaryocytes, which comprises the step of forcibly expressing a gene encoding a cytokine receptor or a cell growth promoting factor under the control of a promoter specific to megakaryocytes or platelets in hematopoietic progenitor cells. This method comprises a step of culturing hematopoietic progenitor cells with the forced expression of one gene selected from the group consisting of genes that suppress the expression of oncogenes, as well as genes that suppress the expression of the p16 gene or the p19 gene, genes that suppress the expression of the Ink4a/Arf gene, and a polycomb gene. The hematopoietic progenitor cells may be derived from pluripotent stem cells. The present application also provides a method for step of producing megakaryocytes by the aforementioned method; and a step of culturing the obtained megakaryocytes while stopping the forced expression of one gene selected from the group consisting of genes that suppress the expression of oncogenes, as well as genes that suppress the expression of the p16 gene or the p19 gene, genes that suppress the expression of the Ink4a/Arf gene, and a polycomb gene, thus providing a method for producing mature megakaryocytes. Furthermore, the present application provides a method for producing platelets, which includes a step of producing mature megakaryocytes by the aforementioned method; and a step of culturing the obtained mature megakaryocytes.

In another aspect, the present application also provides a method for producing megakaryocytes, characterized by the forced expression of a gene encoding a cytokine receptor or a cell growth promoting factor under the control of a promoter specific to megakaryocytes or platelets in hematopoietic progenitor cells. This method includes a step of culturing the hematopoietic progenitor cells while forcing the expression of one gene selected from the group consisting of an oncogene, a gene that suppresses the expression of the p16 gene or the p19 gene, a gene that suppresses the expression of the Ink4a/Arf gene, and a polycomb family gene. The hematopoietic progenitor cells may be differentiated from pluripotent stem cells. The present application also provides a method for producing mature megakaryocytes comprising the steps of producing megakaryocytes by this method; and culturing the obtained megakaryocytes while forcing the expression of an apoptosis-suppressing gene. Furthermore, the present application provides a method for producing platelets which comprises the steps of producing mature megakaryocytes by this method; and culturing the obtained mature megakaryocytes.

The present application also provides megakaryocytes or mature megakaryocytes in which a gene encoding an exogenous cytokine receptor or cell growth promoting factor is integrated in their chromosome under the control of a promoter specific to megakaryocytes or platelets. The megakaryocytes may be those produced by the method of the present application. Examples of the promoter specific to megakaryocytes or platelets, and the gene encoding the cytokine receptor or cell growth promoting factor, are as mentioned above. In one embodiment, the megakaryocyte or mature megakaryocyte further comprises an exogenous apoptosis-inhibiting gene and an oncogene integrated in their chromosome. Examples of the apoptosis-inhibiting genes and oncogenes are as mentioned above. In one embodiment, the megakaryocytes or mature megakaryocytes are CD34-positive CD41-positive cells. In one embodiment, the megakaryocytes or mature megakaryocytes may also be CD38-negative, CD90-positive, and/or CD49f-positive. In one embodiment, the megakaryocytes or mature megakaryocytes have a higher mitochondrial content and/or intracellular granule content compared to the control megakaryocytes or mature megakaryocytes. The control megakaryocytes may be megakaryocytes produced by culturing hematopoietic progenitor cells in a medium that does not contain pyrimido-indole derivatives, for example, CD34-negative CD41-positive cells. The control mature megakaryocyte may be megakaryocytes produced by culturing the megakaryocytes in a medium that does not contain pyrimido-indole derivatives, for example, CD34-negative CD41-positive cells.

Method for producing megakaryocytes, mature megakaryocytes, and platelets (2)

The present application also provides a method for producing megakaryocytes from hematopoietic progenitor cells, characterized by culturing the hematopoietic progenitor cells in a medium comprising a pyrimido-indole derivative. The hematopoietic progenitor cells may be derived from pluripotent stem cells. The differentiation of hematopoietic progenitor cells from pluripotent stem cells may utilize the aforementioned means or known methods.

The production of megakaryocytes from hematopoietic progenitor cells may utilize the aforementioned methods or known methods. Known methods include, for example, the process of culturing hematopoietic progenitor cells with the forced expression of one gene selected from the group consisting of a gene that suppresses the expression of an oncogene (such as a MYC family gene), a gene that suppresses the expression of the p16 gene or p19 gene, a gene that suppresses the expression of the Ink4a/Arf gene, and a polycomb gene (such as the BMI1 gene) (WO2011/034073, WO2012/157586); methods that include the process of culturing hematopoietic progenitor cells with the forced expression of an apoptosis inhibitor genes (such as the BCL2L1 gene) and an oncogene (such as the c-Myc gene) (WO2014/123242); as well as a method that comprises culturing hematopoietic progenitor cells with the forced expression of one gene selected from the group consisting of a gene that suppress the expression of the p16 gene or p19 gene, a gene that suppresses the expression of the Ink4a/Arf gene, and a polycomb gene (such as the BMI1 gene), an apoptosis inhibitor gene (such as the BCL2L1 gene), and an oncogene (such as a MYC family gene) (WO2014/123242). Examples of the gene that suppresses the expression of the p16 gene or p19 gene, the gene that suppresses the expression of the Ink4a/Arf gene, and one gene selected from the group consisting of a polycomb gene, oncogenes, and apoptosis inhibitor genes are as described above.

As pyrimido-indole derivatives, the aforementioned substances, such as UM171 or UM729, may be used. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

The megakaryocytes produced by this method may be CD34-positive and CD41-positive cells. The megakaryocytes produced by this method may also be CD38-negative, CD90-positive, CD49f-positive, and/or CD201-positive.

The megakaryocytes produced by this method may have a higher mitochondrial content compared to the control megakaryocytes. The control megakaryocytes may be megakaryocytes produced by culturing hematopoietic progenitor cells in a medium that does not contain pyrimido-indole derivatives, such as CD34-negative CD41-positive cells.

The megakaryocytes produced by this method may also have a higher intracellular granule content compared to the control megakaryocytes. The control megakaryocytes may be those produced by culturing hematopoietic progenitor cells in a medium that does not contain pyrimido-indole derivatives, such as CD34-negative CD41-positive cells.

In one aspect, the present application provides a method for producing megakaryocytes, characterized by culturing hematopoietic progenitor cells in a medium comprising a pyrimido-indole derivative, which comprises forcibly expressing a gene selected from the group consisting of a gene that suppresses the expression of the p16 gene or the p19 gene, a gene that suppresses the expression of the Ink4a/Arf gene, an apoptosis inhibitor gene, and an oncogene in the hematopoietic progenitor cells and culturing the cells.

Pyrimido-indole derivatives, genes that suppress the expression of the p16 gene or the p19 gene, genes that suppress the expression of the Ink4a/Arf gene, a polycomb gene, anti-apoptotic genes, and oncogenes, as mentioned above, may be used.

One gene selected from the group consisting of a gene that suppresses the expression of the p16 gene or the p19 gene, a gene that suppresses the expression of the Ink4a/Arf gene, and a polycomb gene, an apoptosis inhibitor gene and an oncogene may be forcibly expressed simultaneously, or each gene may be forcibly expressed sequentially. In one embodiment, the forced expression of the gene that suppresses the expression of the p16 gene or the p19 gene, one gene selected from the group consisting of genes that suppress the expression of the Ink4a/Arf gene and a polycomb gene, the apoptosis inhibitor gene, and the oncogene is performed simultaneously. In another embodiment, the forced expression of the gene that suppresses the expression of the p16 gene or the p19 gene, one gene selected from the group consisting of genes that suppress the expression of the Ink4a/Arf gene and a polycomb gene, the apoptosis inhibitor gene, and the oncogene is performed sequentially.

Methods for forcibly expressing the aforementioned genes in hematopoietic progenitor cells may be the methods described above or methods known to those skilled in the art. The vectors for expressing the aforementioned genes and the means for introducing these vectors into hematopoietic progenitor cells can include the vectors and means described above.

A gene that suppresses the expression of the p16 gene or p19 gene, one gene selected from the group consisting of genes suppressing the expression of the Ink4a/Arf gene and A polycomb gene, apoptosis-suppressing genes, and oncogenes can be introduced into hematopoietic progenitor cells via a transposon system. The transposon system may be one of the systems described above, for example, the PiggyBac™ transposon system may be used. In one embodiment, a gene selected from the group consisting of a gene that suppresses the expression of the p16 gene or p19 gene, a gene that suppresses the expression of the Ink4a/Arf gene, and a Polycomb gene, an apoptosis inhibitor gene, and an oncogene are introduced into hematopoietic progenitor cells using the PiggyBac™ transposon system. The method described above can be used for the introduction. In one embodiment, a vector expressing a gene selected from the group consisting of a gene suppressing the expression of the p16 gene or p19 gene, a gene suppressing the expression of the Ink4a/Arf gene, and a Polycomb gene, an apoptosis-suppressing gene, and an oncogene is introduced into hematopoietic progenitor cells. A gene suppressing the expression of the p16 gene or the p19 gene, a gene selected from the group consisting of a gene that suppresses the expression of the Ink4a/Arf gene and a polycomb gene, an apoptosis-suppressing gene, and an oncogene. The aforementioned vectors may be used as vectors expressing these genes, and transposon vectors such as PiggyBac™ vectors may be used. The method described above may be used to introduce the expression vector into hematopoietic progenitor cells.

The above genes may be incorporated into the same vector or into separate vectors. To introduce multiple genes simultaneously, a polycistronic vector with genes linked vertically may be used. To enable polycistronic expression, for example, the 2A selfcleaving peptide from Thosea asigna virus, porcine teschovirus, or foot-and-mouth disease virus, or an IRES sequence may be ligated between the genes that are to be forcibly expressed. For example, a gene selected from a group consisting of a gene that suppresses the expression of the p16 gene or the p19 gene, a gene that suppresses the expression of the Ink4a/Arf gene, and a polycomb gene, an anti-apoptotic gene, and oncogenes may be incorporated into the same vector. Alternatively, a gene that suppresses the expression of the p16 gene or the p19 gene, a gene that suppresses the expression of the Ink4a/Arf gene, and a gene selected from the group consisting of a polycomb gene, as well as an anti-apoptotic gene, may be incorporated into the same vector, while the oncogene may be incorporated into a separate vector.

The above gene may be introduced into hematopoietic progenitor cells using genome editing technology. Examples of genome editing technologies include the CRISPR system (such as CRISPR/Cas9), TALEN, and ZFN. The introduction of genes into cells using genome editing technology may be performed using commercially available genome editing kits, enzymes, vectors, and so on.

In one embodiment, the expression of the p16 gene or the p19 gene is suppressed by a gene selected from a group consisting of a gene that suppresses the expression of the Ink4a/Arf gene, a gene selected from a group consisting of a polycomb gene, an apoptosis inhibitor gene, and the expression of oncogenes, which is controlled by a system that can regulate the on and off states of gene expression. The system that can regulate the on and off states of gene expression may comprise the aforementioned system. In particular, the tetracycline (Tet) gene expression induction system may be preferably used. In one embodiment, the expression of the p16 gene or the p19 gene is suppressed by a gene selected from a group consisting of a gene that suppresses the expression of the Ink4a/Arf gene, a gene selected from a group consisting of a polycomb gene, an apoptosis inhibitor gene, and the expression of oncogenes, which is controlled by the tetracycline expression induction system, and the medium for culturing hematopoietic progenitor cells further contains doxycycline. Doxycycline may be obtained commercially, for example, from LKT Labs. In this embodiment, the concentration of doxycycline may range from 1 ng/mL to 100 μg/mL, from 10 ng/mL to 100 μg/mL, or from 100 ng/mL to 10 μg/mL, for example, about 1 μg/mL.

When the introduced gene is no longer needed for expression, it may be removed from the cells. When the genes are introduced using the PiggyBac™ vector, the gene that has been integrated into the genome may be removed by the PiggyBac™ transposase. For example, this method may further comprise the step of removing one gene selected from the group consisting of genes that suppress the expression of the p16 gene or p19 gene in megakaryocytes, genes that suppress the expression of the Ink4a/Arf gene, apoptosis inhibitor gene and oncogene. In one embodiment, this method may further comprise the step of removing one gene selected from the group consisting of genes that suppress the expression of the p16 gene or p19 gene under the control of a tetracycline-inducible expression system in megakaryocytes, a gene that suppresses the expression of the Ink4a/Arf gene, an apoptosis inhibitor gene and an oncogene.

In a further embodiment, the expression of TP53 gene (protein name: p53) and/or CDKN1A gene (protein name: p21) in hematopoietic progenitor cells, or the function of their expression products, may be suppressed. Methods for suppressing the expression of TP53 gene and/or CDKN1A gene, or the function of their expression products, may be the methods described above.

In one embodiment, the hematopoietic progenitor cells have a gene that suppresses the expression of the p16 gene or the p19 gene under the control of the tetracycline expression induction system, a gene that suppresses the expression of the Ink4a/Arf gene, and one gene selected from the group consisting of a polycomb gene, an apoptosis inhibitor gene, and an oncogene.

The hematopoietic progenitor cells may be induced from pluripotent stem cells that have one of the genes selected from the group consisting of genes that suppress the expression of the p16 gene or the p19 gene, genes that suppress the expression of the Ink4a/Arf gene, and a polycomb gene, as well as anti-apoptotic genes and oncogenes. Alternatively, the genes that suppress the expression of the p16 gene or the p19 gene, genes that suppress the expression of the Ink4a/Arf gene, and a polycomb gene may be introduced into the cells at any differentiation stage from pluripotent stem cells to hematopoietic progenitor cells. The means for introducing genes into pluripotent stem cells or cells at any differentiation stage from pluripotent stem cells to hematopoietic progenitor cells may comprise the aforementioned methods. For example, the gene that suppresses the expression of the p16 gene or the p19 gene, gene that suppresses the expression of the Ink4a/Arf gene, and the polycomb gene may be introduced into cells at any differentiation stage from pluripotent stem cells to hematopoietic progenitor cells using a transposon system. The transposon system may be, for example, the PiggyBac™ transposon system. In one embodiment, the pluripotent stem cells or cells at any differentiation stage from pluripotent stem cells to hematopoietic progenitor cells may be cultured in a medium comprising a pyrimido-indole derivative.

In one embodiment, the expression of the p16 gene or the p19 gene in pluripotent stem cells or cells at any differentiation stage from pluripotent stem cells to hematopoietic progenitor cells is controlled by a system that can regulate the on and off states of gene expression, which includes one gene selected from the group consisting of a gene that suppresses the expression of the Ink4a/Arf gene, a gene that suppresses the expression of the p16 gene or the p19 gene, an apoptosis inhibitor gene, and an oncogene. The system that can control the on and off states of gene expression may utilize the aforementioned system. In particular, the tetracycline (Tet) gene expression induction system may be preferably used. For example, pluripotent stem cells or cells at any differentiation stage from pluripotent stem cells to hematopoietic progenitor cells have a gene that suppresses the expression of the p16 gene or the p19 gene under the control of the tetracycline expression induction system, one gene selected from the group consisting of a gene that suppresses the expression of the Ink4a/Arf gene, an apoptosis inhibitor gene, and an oncogene.

That is, the present application provides a method for producing megakaryocytes from pluripotent stem cells, which comprises the steps of:

culturing pluripotent stem cells having a gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene under the control of a tetracycline expression induction system into hematopoietic progenitor cells, and
culturing the obtained hematopoietic progenitor cells while forcibly expressing the gene that suppresses the expression of p16 gene or p19 gene, the gene that suppresses the expression of Ink4a/Arf gene, and the polycomb gene, as well as the apoptosis inhibitor gene and the oncogene in the presence of doxycycline in a medium comprising a pyrimod-indole derivative.

The hematopoietic progenitor cells may be cultured in suspension for differentiating the cells into megakaryocytes. The hematopoietic progenitor cells may be cultured in the presence of feeder cells or in the absence of feeder cells (feeder-free). The culture period is not particularly limited and for example, it may be at least more than 6 days, more than 12 days, more than 18 days, more than 24 days, more than 30 days, more than 36 days, more than 42 days, more than 48 days, more than 54 days, or more than 60 days. A longer culture period is not considered problematic in the production and maintenance of megakaryocytes. During the culture period, it is desirable to perform subculturing as appropriate.

The culture temperature is not limited and may be between about 30 and 40°C, for example, about 37°C. The culture is conducted under an atmosphere containing carbon dioxide ($CO_2$), with the $CO_2$ concentration potentially ranging from about 0.05 to 15%, about 3 to 7%, or about 4 to 6%, for example, around 5%.

The present application also provides a method for producing mature megakaryocytes from megakaryocytes obtained by the above method. The production of mature megakaryocytes from megakaryocytes may utilize the aforementioned means or known methods. As known methods, for example, the method described in WO2011/034073 which comprises obtaining megakaryocytes and culturing the obtained megakaryocytes while stopping the forced expression of the oncogene (such as MYC family genes), gene that suppress the expression of the p16 gene or p19 gene, and one gene selected from the group consisting of genes that suppress the expression of the Ink4a/Arf gene and a polycomb gene (such as BMI1 gene); the method described in WO2012/157586 which comprises obtaining megakaryocytes and forcibly expressing an apoptosis inhibitor gene (such as the BCL2L1 gene) in the megakaryocytes and culturing the cells; the method described in WO2014/123242 which comprises culturing the obtained megakaryocytes while stopping the forced expression of the apoptosis inhibitor gene (such as BCL2L1 gene) and the oncogene (such as the c-Myc gene); and the method described in WO2014/123242 may also comprise culturing the obtained megakaryocytes while stopping the forced expression of one gene selected from the group consisting of a gene that suppresses the expression of p16 gene or p19 gene, a gene that suppresses the expression of Ink4a/Arf gene, and a polycomb gene (such as BMI1 gene), an apoptosis inhibitor gene (such as BCL2L1 gene), and an oncogene (such as MYC family genes) in the megakaryocytes.

In one embodiment, the megakaryocytes are cultured in a medium comprising a pyrimido-indole derivative. As the pyrimido-indole derivatives, those mentioned above may be used, for example, UM171 or UM729. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

The mature megakaryocytes produced by this method may be CD34-positive and CD41-positive cells. The mature megakaryocytes produced by this method may also be CD38-negative, CD90-positive, and/or CD49f-positive.

The mature megakaryocytes produced by this method may have a higher mitochondrial content compared to the control mature megakaryocytes. The control mature megakaryocytes may be megakaryocytes cultured in a medium that does not contain pyrimido-indole derivatives, for example, CD34-negative CD41-positive cells.

The mature megakaryocytes produced by this method may also have a higher intracellular granule content compared to the control mature megakaryocytes. The control mature megakaryocytes may be megakaryocytes produced by culturing the megakaryocytes in a medium that does not contain pyrimido-indole derivatives, and may, for example, be CD34-negative CD41-positive cells.

In one embodiment, the expression of TP53 gene and/or CDKN1A gene, or the function of their expression products, may be suppressed in the megakaryocytes. The method for suppressing the expression of the gene or the function of its expression products is as described above. In one embodiment, p53 shRNA and p21 shRNA are forcibly expressed in the megakaryocytes.

In one embodiment, the present application provides a method for producing mature megakaryocytes from hematopoietic progenitor cells, which comprises the steps of:

culturing hematopoietic progenitor cells while forcibly expressing a gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene in a medium comprising a pyramid-indole derivative, and
culturing the obtained culture while stopping the forced expression of the gene selected from the gene that suppresses the expression of p16 gene or p19 gene, the gene that suppresses the expression of Ink4a/Arf gene, and the polycomb gene, as well as the apoptosis inhibitor gene and the oncogene.

In a further embodiment, the present application provides a method for producing mature megakaryocytes from pluripotent stem cells, which comprises the steps of:

culturing pluripotent stem cells having a gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene under the control of a tetracycline expression induction system into hematopoietic progenitor cells,
culturing the obtained hematopoietic progenitor cells while forcibly expressing the gene that suppresses the expression of p16 gene or p19 gene, the gene that suppresses the expression of Ink4a/Arf gene, and the polycomb gene, as well as the apoptosis inhibitor gene and the oncogene in the presence of doxycycline in a medium comprising a pyrimod-indole derivative, and
culturing the obtained culture while stopping the forced expression of the gene that suppresses the expression of p16 gene or p19 gene, the gene that suppresses the expression of Ink4a/Arf gene, and the polycomb gene, as well as the apoptosis inhibitor gene and the oncogene.

The megakaryocytes may be cultured in suspension. The megakaryocytes may also be cultured in the presence of feeder cells or in the absence of feeder cells (feeder-free). In one embodiment, the megakaryocytes are cultured in the absence of feeder cells. The culture period is not particularly limited, but is, for example, about 2 to 9 days.

The culture temperature is not limited and may be between about 30 and 40°C, for example, about 37°C. The cultivation is carried out in an atmosphere containing carbon dioxide ($CO_2$), and the CO2 concentration may be between about 0.05 and 15%, around 3 to 7%, or about 4 to 6%, for example, about 5%.

The present application also provides a method for producing platelets from the mature megakaryocytes obtained by the aforementioned method. The production of platelets from the mature megakaryocytes may utilize the aforementioned means or known methods.

In one embodiment, the mature megakaryocytes are cultured in a medium comprising a pyrimido-indole derivative. As pyrimido-indole derivatives, those mentioned above may be used, such as UM171 or UM729. The concentration of the pyrimido-indole derivative may be appropriately selected by those skilled in the art according to the pyrimido-indole derivative used. When the pyrimido-indole derivative is UM729, its concentration may be from 1nM to 100 $\mu$M, from 10nM to 100 $\mu$M, or from 100nM to 10 $\mu$M, for example, about 1 $\mu$M.

In one embodiment, the expression of TP53 gene and/or CDKN1A gene, or the function of their expression products, may be suppressed in mature megakaryocytes. The method for suppressing the expression of the gene or the function of its expression products is as described above. In one embodiment, p53 shRNA and p21 shRNA are forcibly expressed in the megakaryocytes.

In one embodiment, the present application provides a method for producing platelets from hematopoietic progenitor cells, which comprises the steps of:

culturing hematopoietic progenitor cells while forcibly expressing a gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene in a medium comprising a pyramid-indole derivative,
culturing the obtained culture while stopping the forced expression of the gene selected from the gene that suppresses the expression of p16 gene or p19 gene, the gene that suppresses the expression of Ink4a/Arf gene, and the polycomb gene, as well as the apoptosis inhibitor gene and the oncogene, and
culturing the obtained culture while stopping the forced expression of the gene that suppresses the expression of p16 gene or p19 gene, the gene that suppresses the expression of Ink4a/Arf gene, and the polycomb gene, as well as the apoptosis inhibitor gene and/or the oncogene.

In another embodiment, the present application provides a method for producing platelets from pluripotent stem cells, which comprises the steps of:

culturing pluripotent stem cells having a gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene under the control of a tetracycline expression induction system into hematopoietic progenitor cells,
culturing the obtained hematopoietic progenitor cells while forcibly expressing a gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene in a medium comprising a pyramid-indole derivative,
culturing the obtained culture while stopping the forced expression of the gene selected from the gene that suppresses the expression of p16 gene or p19 gene, the gene that suppresses the expression of Ink4a/Arf gene, and the polycomb gene, as well as the apoptosis inhibitor gene and the oncogene, and

culturing the obtained culture while stopping the forced expression of the gene that suppresses the expression of p16 gene or p19 gene, the gene that suppresses the expression of Ink4a/Arf gene, and the polycomb gene, as well as the apoptosis inhibitor gene and/or the oncogene.

The culture conditions are not particularly limited, and a suitable known medium or an equivalent medium that is appropriate for producing platelets from mature megakaryocytes may be used. The culture period is not specifically limited as long as the function of the platelets is maintained, but it is typically around 2 to 9 days.

Mature megakaryocytes may be cultured in suspension. The culture may be performed while stirring or shaking. Stirring may be carried out, for example, using a commercially available rotary shaker culture system to rotate the culture container in an arc shape. The stirring speed may be between 50 to 200 rpm, for example, about 100 rpm. The mature megakaryocytes may be cultured in the presence of feeder cells or in the absence of feeder cells (feeder-free). In one embodiment, the mature megakaryocytes are cultured in the absence of feeder cells.

The culture temperature is not limited and may be between about 30 and 40°C, for example, about 37°C. The cultivation is carried out in an atmosphere containing carbon dioxide ($CO_2$), and the $CO_2$ concentration may be between about 0.05 and 15%, around 3 to 7%, or about 4 to 6%, for example, about 5%.

The present application also provides megakaryocytes or mature megakaryocytes in which a gene selected from a group consisting of a gene that suppresses the expression of the exogenous p16 gene or p19 gene, a gene that suppresses the expression of the Ink4a/Arf gene, a polycomb gene, an oncogene, and an apoptosis inhibitor gene are integrated in their chromosome, and are CD34 positive and CD41 positive. The megakaryocyte or mature megakaryocyte may be produced by the method of the present application. Examples of the gene that suppresses the expression of the p16 gene or p19 gene, the gene that suppresses the expression of the Ink4a/Arf gene, a polycomb gene, an oncogene, and an apoptosis inhibitor gene are as mentioned above. The megakaryocyte or mature megakaryocyte may also be CD38" negative, CD90 positive, and/or CD49f positive. In one embodiment, the megakaryocytes or mature megakaryocytes have a higher mitochondrial content and/or intracellular granule content compared to the control megakaryocytes or mature megakaryocytes. The control megakaryocytes may be megakaryocytes produced by culturing hematopoietic progenitor cells in a medium that does not contain a pyrimido-indole derivative and are, for example, CD34 negative CD41 positive cells.

Kit for producing megakaryocytes, mature megakaryocytes, or platelets

The present application also provides a kit for producing megakaryocytes, mature megakaryocytes, or platelets, which comprises a vector having an oncogene, a homeobox gene, and an apoptosis inhibitor gene. Examples of the oncogene, homeobox gene, apoptosis inhibitor gene, and vector are as described above. The oncogene, the homeobox gene, and the apoptosis inhibitor gene may be incorporated into the same vector or into separate vectors. In one embodiment, the kit comprises a vector having the oncogene, the homeobox gene, and the apoptosis inhibitor gene under the control of a tetracycline expression induction system. In another embodiment, the kit comprises a vector having the oncogene, the homeobox gene, and the apoptosis inhibitor gene, wherein at least one of the oncogene and homeobox gene is under the control of the tetracycline expression induction system, while the apoptosis inhibitor gene is not under the control of the tetracycline expression induction system. In a further embodiment, the kit comprises a vector having the oncogene, the homeobox gene, and the apoptosis inhibitor gene, wherein the oncogene and homeobox gene are under the control of the tetracycline expression induction system, while the apoptosis inhibitor gene is not under the control of the tetracycline expression induction system.

In one embodiment, the kit comprises a vector that expresses a gene encoding a cytokine receptor or a cell growth promoting factor under the control of a promoter specific to megakaryocytes or platelets. Examples of the promoter specific to megakaryocytes or platelets, the gene encoding the cytokine receptor or cell growth promoting factor, and the vector are as described above. In further embodiments, the kit comprises a substance that suppresses the expression of TP53 gene and/or CDKN1A gene or the function of their expression products. The substance that suppresses the expression of TP53 gene and/or CDKN1A gene or the function of their expression products is, for example, a vector that includes p53 shRNA and/or p21 shRNA. Each gene may be incorporated into the same vector as a vector that includes an oncogene, a homeobox gene, and/or an apoptosis inhibitor gene, or may be incorporated into separate vectors. In the kit of the present application, multiple vectors may be packaged together or packaged separately. The kit may also comprise a pyrimido-indole derivative. The aforementioned pyrimido-indole derivatives may be used, such as UM171 or UM729.

This kit may also comprise the reagents necessary for the expression of the oncogene, the homeobox gene, and the apoptosis inhibitor gene within cells, as well as supplements for cell culture such as media, serum, and growth factors (for example, TPO, EPO, SCF, Heparin, IL-6, IL-11), and antibiotics. For example, when using cells derived from pluripotent stem cells, this kit may contain antibodies for marker verification to identify the sac-like structures prepared from these cells (for example, antibodies against Flk1, CD31, CD34, UEA-I lectin). Furthermore, it may comprise reagents for measuring the expression of KLF1 and/or FLI1 to select hematopoietic progenitor cells suitable for megakaryocyte production.

This kit may also comprise a buffer solution, a reaction vessel, an instruction manual, and so on. The reagents and antibodies contained in the kit may be supplied in any type of container that ensures the components maintain their activity effectively over a long period, are not adsorbed by the material of the container, and do not undergo degradation.

The present application also provides a kit for producing megakaryocytes, mature megakaryocytes, or platelets, which comprises a vector having a gene that encodes a cytokine receptor or a cell growth promoting factor under the control of a promoter specific to megakaryocytes or platelets. Examples of the promoter specific to megakaryocytes or platelets, the gene encoding the cytokine receptor or cell growth promoting factor, and the vector are as described above.

In one embodiment, the kit further comprises a vector having an apoptosis inhibitor gene and an oncogene. Examples of the apoptosis inhibitor gene, oncogene, and vector are as described above. In another embodiment, the kit further includes a vector having one gene selected from the group consisting of a gene that suppresses the expression of the p16 gene or the p19 gene, a gene that suppresses the expression of the Ink4a/Arf gene, and a polycomb gene, as well as an oncogene. Examples of the gene that suppresses the expression of the p16 gene or the p19 gene, the gene that suppresses the expression of the Ink4a/Arf gene, and a polycomb gene, the oncogene, and the vector are as described above.

In a further embodiment, the kit additionally comprises a substance that suppresses the expression of TP53 gene and/or CDKN1A gene or the function of their expression products. The substance that suppresses the expression of TP53 gene and/or CDKN1A gene or the function of their expression products is, for example, a vector that includes p53 shRNA and/or p21 shRNA.

This kit may also comprise a pyrimido-indole derivative. The aforementioned derivatives may be used, such as "UM171" or "UM729".

Each gene may be incorporated into the same vector that contains the gene encoding the cytokine receptor or the cell growth promoting factor, or it may be incorporated into a separate vector. In the kit of the present application, multiple vectors may be packaged together or packaged separately.

This kit may also comprise the reagents necessary for the expression of genes encoding cytokine receptors or cell growth promoting factors within cells, as well as supplements for cell culture such as media, serum, and growth factors (for example, TPO, EPO, SCF, Heparin, IL-6, IL-11), and antibiotics. For example, when using cells derived from pluripotent stem cells, this kit may contain antibodies for marker verification to identify the sac-like structures prepared from these cells (for example, antibodies against Flk1, CD31, CD34, UEA-I lectin). Furthermore, it may comprise reagents for measuring the expression of KLF1 and/or FLI1 to select hematopoietic progenitor cells suitable for megakaryocyte production.

This kit may also comprise a buffer solution, reaction vessels, and an instruction manual. The reagents and antibodies contained in the kit may be supplied in any type of container that ensures the components maintain their activity effectively over a long period, are not adsorbed by the material of the container, and do not undergo degradation.

The present application also provides a kit for producing megakaryocytes, mature megakaryocytes, or platelets that includes pyrimido-indole derivatives. The aforementioned pyrimido-indole derivatives may be used, such as UM171 or UM729.

In one embodiment, the kit further comprises a vector having a gene that suppresses the expression of the p16 gene or the p19 gene, a gene that suppresses the expression of the Ink4a/Arf gene, and one gene selected from the group consisting of a polycomb gene, apoptosis inhibitor gene and oncogene. Examples of the gene that suppresses the expression of the p16 gene or the p19 gene, the gene that suppresses the expression of the Ink4a/Arf gene, one gene selected from the group consisting of a polycomb gene, apoptosis inhibitor genes, oncogenes, and vectors are as described above. In another embodiment, the kit further includes a vector having the oncogene, the homeobox gene, and the apoptosis inhibitor gene. Examples of oncogenes, homeobox genes, apoptosis inhibitor genes, and vectors are as described above. In another embodiment, the kit further includes a vector having apoptosis inhibitor gene and oncogene. Examples of apoptosis inhibitor genes, oncogenes, and vectors are as described above.

In a further embodiment, the kit further comprises a substance that suppresses the expression of TP53 gene and/or CDKN1A gene or the function of their expression products. The substance that suppresses the expression of TP53 gene and/or CDKN1A gene or the function of their expression products is, for example, a vector that includes p53 shRNA and/or p21 shRNA.

This kit may also comprise the reagents necessary for the expression of each gene within the cells, the media for cell culture, supplements such as serum and growth factors (for example, TPO, EPO, SCF, Heparin, IL-6, IL-11), and antibiotics. For example, when using cells derived from pluripotent stem cells, this kit may contain antibodies for marker verification to identify the sac-like structures prepared from these cells (for example, antibodies against Flk1, CD31, CD34, UEA-I lectin). Furthermore, it may comprise reagents for measuring the expression of KLF1 and/or FLI1 to select hematopoietic progenitor cells suitable for megakaryocyte production.

This kit may also comprise a buffer solution, reaction vessels, instruction manuals, and so on. The reagents and antibodies contained in the kit may be supplied in any type of container that ensures the components maintain their activity effectively over a long period, are not adsorbed by the material of the container, and do not undergo degradation.

The origin of the cells described in this specification may be human and non-human animals, for example, mice, rats, hamsters, guinea pigs, cattle, horses, pigs, sheep, monkeys, orangutans, chimpanzees, dogs, cats and birds, and is not particularly limited. Preferably the cells are from primates, more preferably from humans.

This disclosure relates, for example, to the following.

[1] A method for producing megakaryocytes from hematopoietic progenitor cells, which comprises the step of culturing the hematopoietic progenitor cells while forcibly expressing an oncogene, a homeobox gene, and an apoptosis inhibitor gene in the hematopoietic progenitor cells and culturing the cells.

[2] The method according to [1], wherein the oncogene is a C-MYC gene.

[3] The method according to [2], wherein the oncogene is a C-MYC gene having an instability domain.

[4] The method according to any one of [1]-[3], wherein the homeobox gene is HOX2 gene.

[5] The method according to any one of [1]-[4], wherein the apoptosis inhibitor gene is BCL2L1 gene.

[6] The method according to any one of [1] - [5], wherein a gene encoding a cytokine receptor or a cell growth promoting factor is further forcibly expressed in hematopoietic precursor cells under the control of a promoter specific to megakaryocytes or platelets.

[7] The method according to [6], wherein the promoter specific to specific to megakaryocytes or platelets is CD41 promoter.

[8] The method according to [6] or [7], wherein the gene encoding a cytokine receptor or a cell growth promoting factor is MPL gene.

[9] The method according to any one of [1] - [8], wherein the expression of TP53 gene and/or CDKN1A gene and/or the function of their expression product in the hematopoietic progenitor cells are suppressed.

[10] The method according to [9], which comprises the step of forcibly expressing p53 shRNA and p21 shRNA in the hematopoietic progenitor cells.

[11] The method according to any one of [1]-[10], wherein the hematopoietic progenitor cells are cultured in a medium comprising a pyrimido-indole derivative.

[12] The method according to [11], wherein the pyrimido-indole derivative is UM171 or UM729.

[13] The method according to any one of [1] to [12], wherein the hematopoietic progenitor cells are human origin.

[14] The method according to any one of [1] to [13], further comprising the step of differentiating pluripotent stem cells into hematopoietic progenitor cells.

[15] The method according to [14], wherein the pluripotent stem cells are iPS cells.

[16] The method according to [14] or [15], wherein the pluripotent stem cells have an oncogene, a homeobox gene and an apoptosis inhibitor gene under the control of tetracycline expression induction system.

[17] The method according to [16], which comprises the steps of

differentiating pluripotent stem cells having an oncogene, a homeobox gene and an apoptosis inhibitor gene under the control of tetracycline expression induction system into hematopoietic progenitor cells, and

culturing the obtained hematopoietic progenitor cells while forcibly expressing the oncogene, homeobox gene and apoptosis inhibitor gene in the presence of doxycycline.

[18] The method according to [14] or [15], wherein the pluripotent stem cells have an oncogene, a homeobox gene and an apoptosis inhibitor gene, and wherein at least one of the oncogene and homeobox gene is under the control of the tetracycline expression induction system and the apoptosis inhibitor gene is constitutively expressed.

[19] The method according to [18], which comprises the steps of

differentiating pluripotent stem cells having an oncogene, a homeobox gene and an apoptosis inhibitor gene into hematopoietic progenitor cells, wherein at least one of the oncogene and homeobox gene is under the control of tetracycline expression induction system and the apoptosis inhibitor gene is constitutively expressed, and

culturing the obtained hematopoietic progenitor cells while forcibly expressing the oncogene, homeobox gene and apoptosis inhibitor gene in the presence of doxycycline.

[20] The method according to any one of [16] - [19], wherein the oncogene, homeobox gene and apoptosis inhibitor gene are incorporated into the pluripotent stem cells by a transposon system.

[21] The method according to [20], wherein the transposon system is PiggyBac™ transposon system.

[22] A method for producing mature megakaryocytes, which comprises the steps of

producing megakaryocytes by a method any one of [1]-[21],
culturing the obtained megakaryocytes while stopping the forced expression of all or a part of the oncogene, homeobox gene and apoptosis inhibitor gene.

[23] The method according to [22], which comprises the steps of

differentiating pluripotent stem cells having an oncogene, a homeobox gene and an apoptosis inhibitor gene under the control of tetracycline expression induction system into hematopoietic progenitor cells,
culturing the obtained hematopoietic progenitor cells while forcibly expressing the oncogene, homeobox gene and apoptosis inhibitor gene in the presence of doxycycline, and
culturing the obtained culture while stopping the forced expression of all or a part of the oncogene, homeobox gene and apoptosis inhibitor gene.

[24] The method according to [22], which comprises the steps of

differentiating pluripotent stem cells having an oncogene, a homeobox gene and an apoptosis inhibitor gene into hematopoietic progenitor cells, wherein at least one of the oncogene and homeobox gene is under the control of tetracycline expression induction system and the apoptosis inhibitor gene is constitutively expressed,
culturing the obtained hematopoietic progenitor cells while forcibly expressing the oncogene, homeobox gene and apoptosis inhibitor gene in the presence of doxycycline, and
culturing the obtained culture while stopping the forced expression of all or a part of the oncogene, homeobox gene and apoptosis inhibitor gene.

[25] A method for producing platelets, which comprises the steps of

producing mature megakaryocytes by a method any one of [22]-[24], and
culturing the obtained mature megakaryocytes.

[26] The method according to [25], which comprises the steps of

differentiating pluripotent stem cells having an oncogene, a homeobox gene and an apoptosis inhibitor gene under the control of tetracycline expression induction system into hematopoietic progenitor cells,
culturing the obtained hematopoietic progenitor cells while forcibly expressing the oncogene, homeobox gene and apoptosis inhibitor gene in the presence of doxycycline,
culturing the obtained culture while stopping the forced expression of all or a part of the oncogene, homeobox gene and apoptosis inhibitor gene, and
further culturing the obtained culture.

[27] The method according to [25], which comprises the steps of

differentiating pluripotent stem cells having an oncogene, a homeobox gene and an apoptosis inhibitor gene into hematopoietic progenitor cells, wherein at least one of the oncogene and homeobox gene is under the control of tetracycline expression induction system and the apoptosis inhibitor gene is constitutively expressed,
culturing the obtained hematopoietic progenitor cells while forcibly expressing the oncogene, homeobox gene and apoptosis inhibitor gene in the presence of doxycycline,
culturing the obtained culture while stopping the forced expression of all or a part of the oncogene, homeobox gene and apoptosis inhibitor gene, and
further culturing the obtained culture.

[28] Megakaryocytes or mature megakaryocytes, wherein an exogenous oncogene, an exogenous homeobox gene and an exogenous apoptosis inhibitor gene are integrated in their chromosome.
[29] A method for producing megakaryocytes from hematopoietic progenitor cells, which comprises the step of forcibly expressing a gene encoding a cytokine receptor or a cell growth promoting factor under the control of a promoter specific to megakaryocyte or platelet in the hematopoietic stem cells.
[30] The method according to [29], wherein the promoter specific to megakaryocyte or platelet is CD41 promoter.
[31] The method according to [29] or [30], wherein the gene encoding a cytokine receptor or a cell growth promoting factor is MPL gene.
[32] The method according to any one of [29]-[31], wherein the method comprises the step of culturing hematopoietic progenitor cells while forcibly expressing the apoptosis inhibitor gene and the oncogene.

[33] The method according to [32], wherein the apoptosis inhibitor gene is BCL2L1 gene.

[34] The method according to [32] or [33], wherein the oncogene is c-MYC gene.

[35] The method according to any one of [29]-[34], wherein the expression of TP53 gene and/or CDKN1A gene and/or the function of their expression product in the hematopoietic progenitor cells are suppressed.

[36] The method according to [35], which comprises the step of forcibly expressing p53 shRNA and p21 shRNA in the hematopoietic progenitor cells.

[37] The method according to any one of [29] to [36], wherein the hematopoietic progenitor cells are cultured in a medium comprising a pyrimido-indole derivative.

[38] The method according to [37], wherein the pyrimido-indole derivative is UM171 or UM729.

[39] The method according to any one of [29] to [38], wherein the hematopoietic progenitor cells are human origin.

[40] The method according to any one of [29] to [39], further comprising the step of differentiating pluripotent stem cells into hematopoietic progenitor cells.

[41] The method according to [40], wherein the pluripotent stem cells are iPS cells.

[42] The method according to [40] or [41], wherein the pluripotent stem cells have an oncogene and an apoptosis inhibitor gene under the control of tetracycline expression induction system.

[43] The method according to [42], which comprises the steps of

differentiating pluripotent stem cells having a gene encoding a cytokine receptor or a cell growth promoting factor under the control of a promoter specific to megakaryocyte or platelet and an apoptosis inhibitor gene and an oncogene under the control of tetracycline expression induction system into hematopoietic progenitor cells, and culturing the obtained hematopoietic progenitor cells while forcibly expressing the gene encoding a cytokine receptor or a cell growth promoting factor, the apoptosis inhibitor gene and the oncogene in the presence of doxycycline.

[44] The method according to [40] or [41], wherein the pluripotent stem cells have the gene encoding a cytokine receptor or a cell growth promoting factor, the apoptosis inhibitor gene and the oncogene, wherein the gene encoding a cytokine receptor or a cell growth promoting factor is under the control of a promoter specific to megakaryocyte or platelet, the oncogene is under the control of the tetracycline expression induction system and the apoptosis inhibitor gene is constitutively expressed.

[45] The method according to [44], which comprises the steps of:

differentiating pluripotent stem cells having the gene encoding a cytokine receptor or a cell growth promoting factor, the apoptosis inhibitor gene and the oncogene into hematopoietic progenitor cells, wherein the gene encoding a cytokine receptor or a cell growth promoting factor under the control of a promoter specific to megakaryocyte or platelet, the oncogene is under the control of the tetracycline expression induction system and the apoptosis inhibitor gene is constitutively expressed: and culturing the obtained hematopoietic progenitor cells while forcibly expressing the gene encoding a cytokine receptor or a cell growth promoting factor, the apoptosis inhibitor gene and the oncogene in the presence of doxycycline.

[46] The method according to any one of [42] - [45], wherein the oncogene, homeobox gene and apoptosis inhibitor gene are incorporated into the pluripotent stem cells by a transposon system.

[47] The method according to [20], wherein the transposon system is PiggyBac™ transposon system.

[48] A method for producing mature megakaryocytes, which comprises the steps of:

producing megakaryocytes according to the method any one of [32]-[47], and culturing the obtained megakaryocytes while stopping the forced expression of the apoptosis inhibitor gene and/or the oncogene.

[49] The method according to [48], which comprises the steps of

differentiating pluripotent stem cells into hematopoietic progenitor cells, culturing the hematopoietic progenitor cells while forcibly expressing a gene encoding a cytokine receptor or a cell growth promoting factor under the control of a promoter specific to megakaryocyte or platelet as well an oncogene and an apoptosis inhibitor gene, and culturing the obtained culture while stopping the forced expression of the oncogene and/or apoptosis inhibitor gene.

[50] A method for producing platelets, which comprises the steps of

producing mature megakaryocytes by the method according to [48] or [49], and
culturing the obtained mature megakaryocytes.

[51] The method according to [50], which comprises the steps of:

differentiating pluripotent stem cells into hematopoietic progenitor cells,
culturing the hematopoietic progenitor cells while forcibly expressing a gene encoding a cytokine receptor or a cell growth promoting factor under the control of a promoter specific to megakaryocyte or platelet as well an oncogene and an apoptosis inhibitor gene,
culturing the obtained culture while stopping the forced expression of the oncogene and/or apoptosis inhibitor gene,
culturing the obtained culture.

[52] A method for producing megakaryocytes from hematopoietic progenitor cells, which comprises the step of culturing the hematopoietic progenitor cells a medium comprising a pyrimido-indole derivative.
[53] The method according to [52], wherein the pyrimido-indole derivative is UM171 or UM729.
[54] The method according to [54], wherein the hematopoietic progenitor cells are cultured while forcibly expressing an apoptosis inhibitor gene and an oncogene.
[55] The method according to any one of [52]-[55], which comprises the step of culturing hematopoietic progenitor cells while forcibly expressing a gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene.
[56] The method according to [55], wherein the gene selected from the group consisting of a gene that suppresses the expression of p16 gene or p19 gene, a gene that suppresses the expression of Ink4a/Arf gene, and a polycomb gene is BMI1.
[57] The method according to [55] or [56], wherein the apoptosis inhibitor gene is BCL2L1 gene.
[58] The method according to any one of [55] - [57], wherein the oncogene is c-MYC gene.
[59] The method according to any one of [52]-[58], wherein the hematopoietic progenitor cells are human origin.
[60] The method according to [52] - [59], wherein the megakaryocytes are CD34-positive and CD41-positive cells.
[61] The method according to [61], wherein the megakaryocytes are CD38-negative, CD90-positive and/or CD49f-positive cells.
[62] The method according to any one of [52]-[61], wherein the megakaryocytes have a higher mitochondrial content or intracellular granule content compared to control megakaryocytes.
[63] The method according to [62], wherein the control megakaryocytes are CD34-negative and CD41-positive cells.
[64] The method according to any one of [52]-[63], further comprising the step of differentiating pluripotent stem cells into hematopoietic progenitor cells.
[65] The method according to [64], wherein the pluripotent stem cells are iPS cells.
[66] The method according to [64] or [65], wherein the pluripotent stem cells have a gene selected from the group consisting of a gene that suppresses the expression of p16 gene or p19 gene, a gene that suppresses the expression of Ink4a/Arf gene, and a polycomb gene as well as an apoptosis inhibitor gene and an oncogene under the tetracycline expression induction system.
[67] The method according to [66], which comprises the steps of

differentiating the pluripotent stem cells have a gene selected from the group consisting of a gene that suppresses the expression of p16 gene or p19 gene, a gene that suppresses the expression of Ink4a/Arf gene, and a polycomb gene as well as an apoptosis inhibitor gene and an oncogene under the tetracycline expression induction system into hematopoietic progenitor cells, and
culturing the obtained hematopoietic progenitor cells while forcibly expressing the gene selected from the group consisting of a gene that suppresses the expression of p16 gene or p19 gene, a gene that suppresses the expression of Ink4a/Arf gene, and a polycomb gene as well as an apoptosis inhibitor gene and an oncogene in the presence of doxycycline in a medium comprising a pyrimido-indole derivative.

[68] The method according to [66] or [67], wherein the gene selected from the group consisting of a gene that suppresses the expression of p16 gene or p19 gene, a gene that suppresses the expression of Ink4a/Arf gene, and a polycomb gene as well as an apoptosis inhibitor gene and an oncogene are incorporated into the pluripotent stem cells by a transposon system.

[69] The method according to [68], wherein the transposon system is PiggyBac™ transposon system.

[70] A method for producing mature megakaryocytes, which comprises the steps of

producing megakaryocyte by the method according to any one of [52] to [69], and

culturing the megakaryocytes while stopping the forced expression of the gene selected from the group consisting of a gene that suppresses the expression of p16 gene or p19 gene, a gene that suppresses the expression of Ink4a/Arf gene, and a polycomb gene as well as the apoptosis inhibitor gene and/or the oncogene.

[71] The method according to [70], which comprises the steps of

differentiating pluripotent stem cells into hematopoietic progenitor cells,

culturing the obtained hematopoietic progenitor cells while forcibly expressing the gene selected from the group consisting of a gene that suppresses the expression of p16 gene or p19 gene, a gene that suppresses the expression of Ink4a/Arf gene, and a polycomb gene as well as an apoptosis inhibitor gene and an oncogene in a medium comprising a pyrimidine-indole derivative, and

culturing the obtained culture while stopping the forced expression of the gene selected from the group consisting of a gene that suppresses the expression of p16 gene or p19 gene, a gene that suppresses the expression of Ink4a/Arf gene, and a polycomb gene as well as the apoptosis inhibitor gene and/or the oncogene.

[72] A method for producing platelets, which comprises the steps of

producing mature megakaryocytes by a method according to [69] or [70], and

culturing the obtained mature megakaryocytes.

[73] A method according to [72], which comprises the steps of

differentiating pluripotent stem cells into hematopoietic progenitor cells,

culturing the obtained hematopoietic progenitor cells while forcibly expressing the gene selected from the group consisting of a gene that suppresses the expression of p16 gene or p19 gene, a gene that suppresses the expression of Ink4a/Arf gene, and a polycomb gene as well as an apoptosis inhibitor gene and an oncogene in a medium comprising a pyrimido-indole derivative,

culturing the obtained culture while stopping the forced expression of the gene selected from the group consisting of a gene that suppresses the expression of p16 gene or p19 gene, a gene that suppresses the expression of Ink4a/Arf gene, and a polycomb gene as well as the apoptosis inhibitor gene and/or the oncogene, and

culturing the obtained culture.

[74] Megakaryocytes or mature megakaryocytes, wherein an exogenous gene selected from the group consisting of a gene that suppresses the expression of p16 gene or p19 gene, a gene that suppresses the expression of Ink4a/Arf gene, and a polycomb gene as well as an oncogene and an apoptosis inhibitor gene are integrated in their chromosome, and wherein the cells are CD34-positive and CD41-positive cells.

[75] The megakaryocytes or mature megakaryocytes according to [74], wherein the cells are CD38-negative, CD90-positive and/or CD49f-positive.

[76] The megakaryocytes or mature megakaryocytes according to [74] or [75], wherein the megakaryocytes have a higher mitochondrial content or intracellular granule content compared to control megakaryocytes.

[77] The method according to [62], wherein the control megakaryocytes are CD34-negative and CD41-positive cells.

[78] A kit for producing megakaryocytes, mature megakaryocytes or platelets, which comprises a vector having an oncogene and an apoptosis inhibitor gene.

[79] The kit according to [78], wherein the vector is a transposon vector.

[80] A kit for producing megakaryocytes, mature megakaryocytes or platelets, which comprises a vector having a gene encoding a cytokine receptor or a cell growth promoting factor under the control of a promoter specific for megakaryocytes or platelets.

[81] The kit according to [81], further comprises a vector having an apoptosis inhibitor gene and an oncogene.

[82] A kit for producing megakaryocytes, mature megakaryocytes or platelets, which comprises a pyrimido-indole derivative.

[83] The kit according to [82], wherein the pyrimido-indole derivative is UM171 or UM729.

[84] The kit according to [82] or [83], further comprises a vector having a gene that suppresses the expression of p16 gene or p19 gene, a gene that suppresses the expression of Ink4a/Arf gene, and a polycomb gene as well as an oncogene and an apoptosis inhibitor gene.

[EXAMPLES]

This application will be explained further with the examples below. The examples are just for examples and should not be used to limit the scope of the application.

iPS cells

In the examples, the following human iPS cell lines were obtained from Kyoto University iPS Cell Research Foundation (CiRA Foundation): 585A1, Ff-I01s01 (I01s01), Ff-MH23s01 (MH23S01), TkDN-sev2, 15M41, Ff-MH09S01 (MH09S01), Ff-MH15S01 (MH15S01), and Ff-MH15S02 (MH15S02). Among these, I01s01 and 15M41 cell lines were derived from peripheral blood of donors who had the Japan's most frequent HLA and were homozygous for their HLAs. MH09S01, MH15S01, MH15S02, and MH23S01were derived from peripheral blood of donors who had the Japan's second most frequent HLA and were homozygous for their HLAs.

[Example 1]

Production of megakaryocytes by forced expression of an oncogene, a homeobox gene, and an apoptosis inhibitor gene

Materials and Methods

1. iPS cell culture

The iPS cells were seeded on a 6-well plate coated with iMatrix-511 silk or iMatrix-511MG (Nippon) in StemFit AK02N or StemFit AK03N medium (Ajinomoto) under the culture condition of 37°C and 5% $CO_2$. The medium supplemented with the ROCK inhibitor Y-27632 (Nacalai Tesque, Code No. 18188-04) was added when the cells were passaged. Then, the medium was replaced on the next day to remove Y-27632. The medium was removed from the iPS cell culture and the cells were washed with PBS, and then, a 1:1 mixture of TrypLE™ Select Enzyme (Thermo Fisher Scientific, Code No. 12563011) and 0.5 mmol/L EDTA/PBS solution (Nacalai Tesque, Code No. 13567-84) was added to the cells. The cells were incubated for 7 minutes in an incubator (37°C, 5% $CO_2$). The StemFit AK02N or StemFit AK03N medium was then added to the cells, and the cells were detached and collected using a scraper. The cell number was counted using trypan blue and the cells were seeded at a density of 0.5 - 3 $\times$ $10^4$ cells per well for passaging.

A cell suspension collected during subculturing was seeded in a 6-well plate at a cell density of 0.3 - 1$\times$$10^5$ cells per well for introducing genes.

2. Gene introduction into the iPS cells

The introduction of target genes into iPS cells was performed using the PiggyBac™ system, which utilizes the transposase enzyme. HOXA2 gene, instability domain-fused c-MYC gene (MYCdd gene), and BCL2L1 gene (3 genes: MYCdd/HOXA2/BCLxL) were introduced under the control of the TRE (Tet Response Element). The TRE induces the expression of the introduced genes by the addition of doxycycline (Nakamura S., et al., Expandable megakaryocyte cell lines enable clinically applicable generation of platelets from human induced pluripotent stem cells, Cell Stem Cell, 14 (4), 535-548, 2014). Unless otherwise indicated in the following examples, the oncogene, homeobox gene, and apoptosis-inhibiting gene were introduced to be under the control of the TRE. In some conditions, the HOXA2 gene was excluded, and the introduced genes consisted of the instability domain-fused c-MYC gene (MYCdd gene) and the BCL2L1 gene. Additionally, in some conditions, the expression of the BCL2L1 gene was induced by doxycycline under the control of the TRE system, or it was expressed constitutively under the control of the ubiquitin promoter. Genes under the control of the ubiquitin promoter are expressed continuously, regardless of the presence of doxycycline.

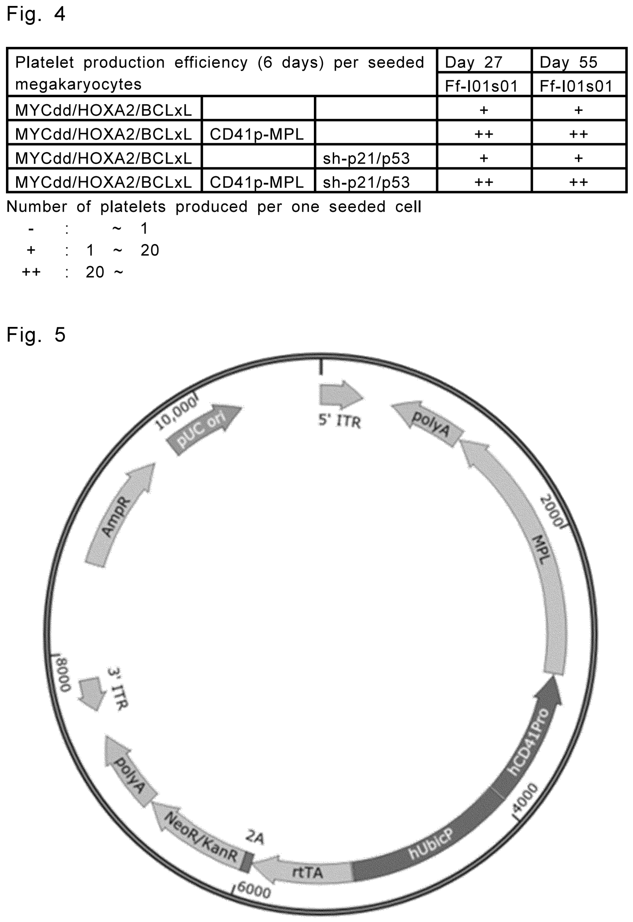

Furthermore, under certain conditions, a shRNA expression vector to knock down p21 and p53 was additionally introduced (Sone M., et al., Silencing of p53 and CDKN1A establishes sustainable immortalized megakaryocyte progenitor cells from human iPSCs, Stem Cell Reports, 16 (12), 2861-2870, 2021). Additionally, under certain conditions, a vector having human MPL gene under the control of human CD41 promoter was also introduced. The vector map of the vector having human MPL gene under the control of human CD41 promoter used in this example is shown in Figure 5. The human CD41 promoter-human MPL gene sequence, which includes a 12bp linker, is shown as SEQ ID NO:3. In the control group, BMI1 gene was introduced under the control of the TRE (tet Responsive Element) instead of HOXA2 gene (conventional three factors: MYCdd/BMI1/BCLxL).

In addition, to purify the iPS cells with the introduced genes through drug treatment, each of the gene expression vectors was incorporated one of drug resistance gene selected from the puromycin resistance gene, the hygromycin resistance gene, and the neomycin resistance gene.

The introduction of the vector into the cells was performed using the Lipofectamine™ Stem Transfection Reagent (ThermoFisher Scientific, Code No. STEM00008) according to the provider's manual. The transposase expression vector was mixed with each gene expression vector and incubated for 10 minutes, after which the mixture was added to the medium in which the iPS cells were seeded. The following day, the medium was replaced with a drug-containing media of puromycin/hygromycin/G418, and the gene-introduced iPS cells were cultured in the drug-containing medium until they proliferated sufficiently.

3. Differentiation of iPS cells into megakaryocytes via hematopoietic progenitor cells derived from iPS cells

The gene-modified iPS cells were treated with a GSK-3β inhibitor CHIR-99021 and Y-27632 for three days, and were dissociated into cell aggregate fragments. The feeder cells used were the human fibroblast cell line C3H/10T1/2 (ATCC number: CCL-226), and the gene-modified iPS cell aggregate fragments were added onto the mitomycin C-treated C3H/10T1/2 cells that had been pre-seeded in a 10 cm dish.

As the basal medium, IMDM(Iscove's Modified Dulbecco's Medium)(Sigma-Aldrich, Code No.I3390)supplemented with 15% Fetal Bovine Serum(FBS)(Life Technologies, Code No.10270-106), 2mM Glutamine(Life Technologies, Code No. 25030-081), 1% Insulin / Transferrin / Selenium Solution(ITS-G)(Life Technologies, Code No.41400-045), 0.45 mM 1-Thioglycerol(Sigma-Aldrich,Code No. A6145), 50 μg/mL L-Ascorbic Acid(Sigma-Aldrich, Code No. A4544)was prepared.

The basal medium supplemented with 20 ng/mL of VEGF (Fujifilm Wako Pure Chemical Corporation, Code No. 226-01786) was used for the start culture of the genetically modified iPS cells, and the cells were cultured under conditions of 37°C, 5% O2, and 5% CO2.

On the 4th day of culture, the medium was replaced with the basal medium supplemented with 20 ng/mL of VEGF, 10 U/mL of Heparin (Aiwai Pharma), 50 ng/mL of bFGF (Wako Pure Chemical Industries, Code No. 068-04544), and 10 μM of SB431542 (Wako Pure Chemical Industries, Code No. 037-24293), and the cells were cultured under conditions of 37°C, 5% O2, and 5% CO2.

On the 7th day of culture, the medium was replaced with the basal medium supplemented with 20 ng/mL of VEGF, 0.2 μg/mL of a TPO mimetic compound, 50 ng/mL of Human Stem Cell Factor (SCF) (Fujifilm Wako Pure Chemical Corporation, Code No. 193-15513), 1 μg/mL of Doxycycline (TAKARA Bio Inc., Code No. 631311), and 10 U/mL of Heparin. The cells were cultured under conditions of 37°C, 20% O2, and 5% CO2.

On the 11th day of culture, the basal medium supplemented with 20 ng/mL of VEGF, 0.2 μg/mL of TPO mimetic compound, 50 ng/mL of SCF, and 1 μg/mL of doxycycline was added or replaced. The cells were cultured under the conditions of 37°C, 20% O2, and 5% CO2.

On the 14th day of culture, the floating cells were collected from the feeder cells and resuspended in the basal medium supplemented with 0.2 μg/mL TPO mimetic compound, 50 ng/mL SCF, and 1 μg/mL doxycycline. The culture was started under feeder-free conditions of 37°C, 20% $O_2$ and 5% $CO_2$. By continuing the subculture using the same medium, the cells were differentiated into megakaryocytes. The start date of the feeder-free culture was designated as Day 0, and in some conditions, UM729 (MERCK Code No. ATEH97ECEE61) was added to the basal medium at a final concentration of 1 μM, UM171 (APExBIO Cat. No. A8950) at a final concentration of 35nM, or SR-1 at a final concentration of 0.75 μM, starting from Day 0 or Day 7, to continue the subculture.

4. Evaluation of the proliferative capacity of the megakaryocytes

The start date of culture under feeder-free conditions was designated as Day 0. Cell counts were measured periodically, and detection of the megakaryocyte-specific surface marker CD41 was performed periodically by flow cytometry.

The cell number was counted using trypan blue, and the cumulative proliferation rate from Day 0 was calculated based on the proliferation factor derived from the number of the seeded cells. Simultaneously, the proliferation rate of the megakaryocytes was determined by multiplying the cumulative proliferation rate of the megakaryocytes by the ratio of megakaryocyte presence calculated from the measurement results of the specific surface markers for megakaryocytes. The cumulative proliferation rate of the megakaryocytes at passage n was calculated using the formula below. cumulative proliferation rate of the megakaryocytes at the nth passage n

$$= (\prod_{k=1}^{n} \frac{live\ cell\ density\ collected\ at\ the\ kth\ passage}{cell\ dencity\ seeded\ at\ the\ k\text{-}1\ th\ passage}) \times (\frac{ratio\ of\ CD41\ positive\ cells\ at\ the\ nth\ passag}{ratio\ of\ CD41\ positive\ cells\ on\ day\ 0})$$

The seeding density of the 0th passage is the seeding density on Day 0.

5. Induction of mature megakaryocytes, evaluation of platelet production capacity of the mature megakaryocytes and evaluation of the function of the produced platelets

The cells in the proliferation culture of the gene-introduced megakaryocytes were washed to remove doxycycline and then, cultured in a medium with no doxycycline thereby the forced expression of the genes controlled by the TRE (tet Response Element) was terminated and the maturation of the megakaryocytes and the production of platelets were induced. In the case the cells express MPL gene under the control of CD41 promoter and BCL2L1 gene under the control of ubiquitin promoter, the expression of the genes was maintained even after the washing step. IMDM media supplemented with 5% human plasma, 2 mM glutamine, 1% ITS-G, 0.45 mM 1-thioglycerol, 50 $\mu$g/mL L-ascorbic acid, 50 ng/mL SCF, 0.2 $\mu$g/mL TPO mimetic compound, 15 $\mu$M KP-457 (Kaken Pharmaceutical) and 10 $\mu$M Y-27632 (Nacalai Tesque, Code No. 18188-04), as well as 0.75 $\mu$M SR-1 (Stemregenin-1) or 0.1 $\mu$M AhR inhibitor (the concentrations are final concentrations) were prepared. Platelet production culture was conducted by seeding megakaryocytes at a density of 1 x $10^5$ cells/mL 25 mL/flask in a 125 mL culture flask, and performing shaking culture. The shaking culture was conducted using the LT-X (Lab-Therm) (Kuhner) or S41i (Eppendorf) under conditions of 37°C and 5% $CO_2$ at a shaking speed of 100 rpm.

The cells were cultured under the above conditions for 6 days, then, a portion of the cell suspension was analyzed by BD LSRFortessa™ (BD Biosciences) to measure the number of produced platelets and to evaluate platelet function.

The following antibodies were used to stain the cells to measure the number of produced platelets:

APC-labeled anti-CD41 antibody (BioLegend, Code No. 303710)
PE-labeled anti-CD42b antibody (BioLegend, Code No. 303906)
eFluor™ 450-labeled anti-CD42a antibody (Thermo Fisher, Code No. 48-0428-42)

The cells were stained with the antibodies and after 30 minutes' staining, the platelet count (gated as microparticles by FSC/SSC, either CD41/CD42b double positive or CD41/CD42a/CD42b positive) was measured using TruCOUNT ™ (BD Biosciences, Code No. 340334). Flow cytometry data was analyzed using FlowJo ™ Version 10 (BD Biosciences) to calculate the number of the produced platelets. The data was calculated as the number of platelets produced per megakaryocyte, referencing the number of megakaryocytes seeded at the start of the platelet production culture.

To evaluate the function of the platelets, the platelets were stimulated under the following two types of stimulants.

Stimulant 1: 0.02 to 0.4 $\mu$M PMA
Stimulant 2: 100 $\mu$M ADP/40 $\mu$M Thrombin Receptor Activator Peptide 6(TRAP6)

After the addition of the stimulant, the samples were stained with the following antibodies:

APC-labeled anti-CD41 antibody (BioLegend, Code No. 303710)
PE-labeled anti-CD62P antibody (BioLegend, Code No. 304906)
FITC-labeled anti-PAC-1 antibody (BD Bioscience, Code No. 34507)

Measurements were conducted using the flow cytometer after 30 minutes.

(Example 1-1)

Megakaryocytes induced from iPS cell line 585A1

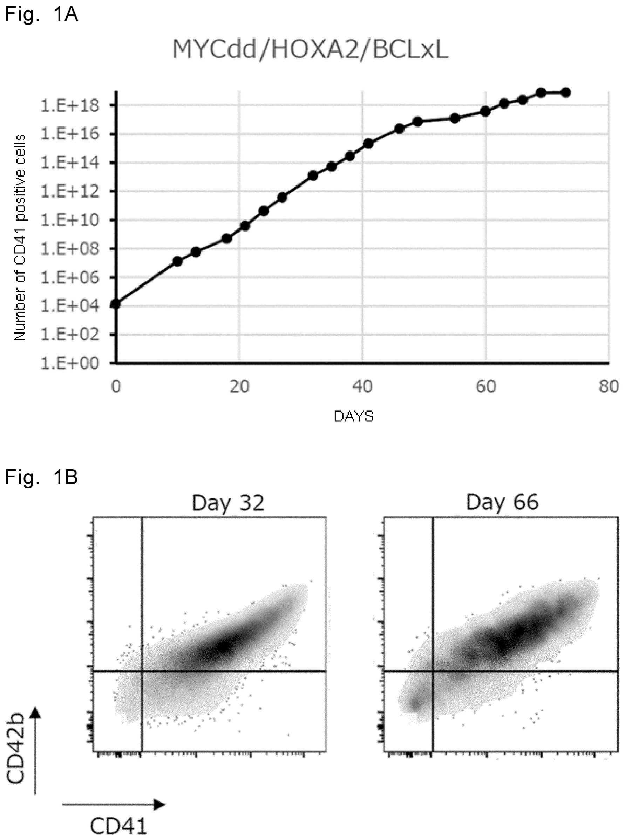

iPS cells incorporated with the expression vector having the three genes (MYCdd gene, HOXA2 gene, and BCL2L1 gene) were cultured on feeder cells for 14 days to induce differentiation into megakaryocytes via hematopoietic progenitor cells. Seven days after the initiation of the differentiation culture, doxycycline was added to induce the expression of the exogenous genes. The cells were collected from the feeder cells, and transferred to a feeder free condition under the presence of doxycycline. The day the feeder-free culture began was designated as Day 0, and the cells were sub-cultured in the presence of doxycycline. The cell number was counted using trypan blue. Simultaneously, the megakaryocyte specific surface marker CD41 was detected, and the percentage of the CD41 positive cells were calculated. The number of megakaryocytes was calculated by multiplying the measured cell count and the parcentage of the CD41 positive cells. The cumulative cell count of megakaryocytes on each measurement day was calculated based on the number of the seeded cells at the start of passaging and is shown in Figure 1A.

It was confirmed that the megakaryocytes induced by the three factors (MYCdd/HOXA2/BCLxL) have sustained cell proliferation ability for over 60 days (Figure 1A). The establishment of the immortalized megakaryocytes with long-term proliferative capacity by the three factors was confirmed.

On Day 32 and Day 66, a part of the proliferative immortalized megakaryocytes obtained above was collected. The cells were stained with anti-human CD41 antibody and anti-human CD42b antibody, and the surface markers specific to megakaryocytes were measured using a flow cytometer. The results on Day 32 and Day 66 are shown in Figure 1B. It was confirmed that on Day 32, megakaryocytes were generated from iPS cell line 585A1 introduced with the three factors (MYCdd/HOXA2/BCLxL). Further, it was confirmed that the characteristics of the megakaryocytes were maintained on Day 66, i.e. the culture period was extended. Therefore, it has been clarified that the three factors can establish immortalized megakaryocytes.

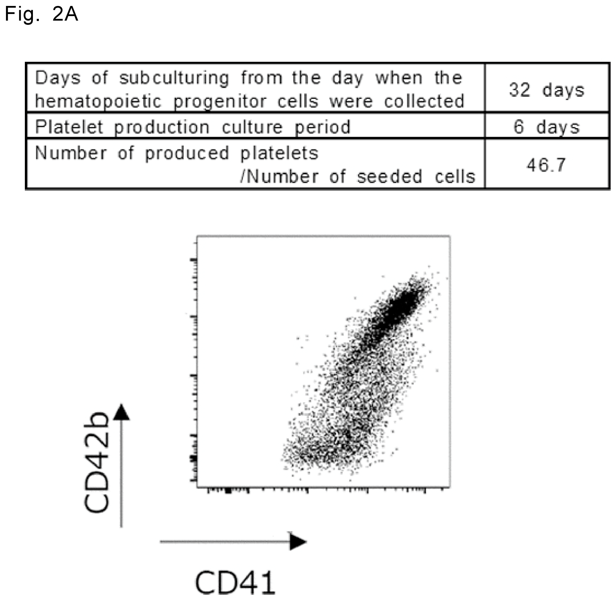

Additionally, on Day 32, a part of the proliferative immortalized megakaryocytes obtained above was recovered, and the cells were washed to remove doxycycline and further cultured in the absence of doxycycline to start the platelet induction culture. After six days of the platelet induction culture, a part of the culture medium was collected and stained with anti-human CD41 antibody and anti-human CD42b antibody to measure the number of double-positive microparticles as platelets using a flow cytometer. Based on the number of the seeded cells at the start of the platelet induction culture, the number of platelets per seeded cell was calculated and is shown in Figure 2A.

As shown in Figure 2A, platelets were produced from immortalized megakaryocytes established using the three factors (MYCdd/HOXA2/BCLxL). Furthermore, the number of platelets produced was increased compared to the case wherein the megakaryocytes were established using the conventional three factors (MYCdd/BMI1/BCLxL). The immortalized megakaryocytes established using the three factors (MYCdd/HOXA2/BCLxL) were confirmed to retain the important function of platelet production.

The CD41-positive platelets obtained above were evaluated for the responsibilities to the platelet activating stimulant(s). The stimulants used were PMA or a mixture of ADP/TRAP6. For the CD41-positive platelets, the PAC1+/CD62P+ fraction was identified as activated platelets, and its ratio was analyzed. The unstimulated condition served as the negative control.

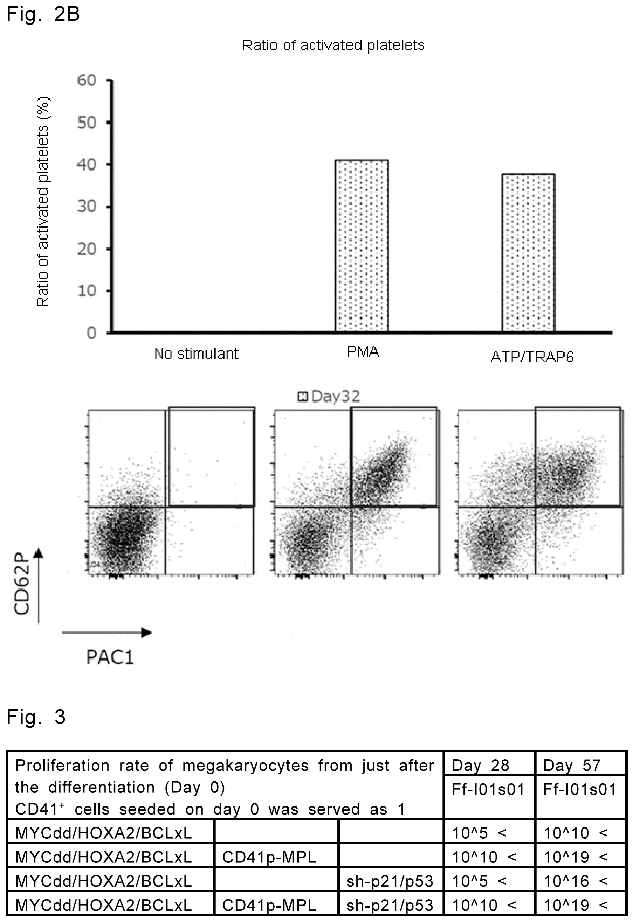

As a result, platelets produced from immortalized megakaryocytes established using the three factors (MYCdd/HOXA2/BCLxL) were responsive to the stimulant (Figure 2B). The ratio of responsive platelets to the total produced platelets was higher compared to the platelets produced from megakaryocytes established using the conventional three factors (MYCdd/BMI1/BCLxL). Therefore, the platelets produced from the immortalized megakaryocytes established using the three factors (MYCdd/HOXA2/BCLxL) are responsive to the platelet activating stimulant. The responsibility to the stimulant relates to the hemostat ability, the important function of platelets. In addition, the production efficiency of functional platelets was higher than megakaryocytes established using the conventional three factors (MYCdd/BMI1/BCLxL).

(Example 1-2)

Megakaryocytes differentiated from iPS cell lines Ff-I01s01 and Ff-MH23s01

iPS cells incorporated with the expression vector having the three genes (MYCdd gene, HOXA2 gene, and BCL2L1 gene) were cultured on feeder cells for 14 days to induce differentiation into megakaryocytes via hematopoietic progenitor cells. Seven days after the initiation of the differentiation culture, doxycycline was added to induce the expression of the exogenous genes. The cells were collected from the feeder cells, and transferred to a feeder free condition in the presence of doxycycline. The day the feeder-free culture began was designated as Day 0, and the cells were sub-cultured in the presence of doxycycline. The cell number was counted using trypan blue. Simultaneously, the megakaryocyte specific surface marker CD41 was detected, and the percentage of the CD41 positive cells were calculated. The number of megakaryocytes was calculated by multiplying the measured cell count and the percentage of the CD41 positive cells. The cumulative cell growth rates of the megakaryocytes until Day 28 and Day 57 were calculated based on the number of the seeded cells at the start of passaging and are shown in Figure 3.

As shown in Figure 3, the megakaryocytes induced using the three factors (MYCdd/HOXA2/BCLxL) could proliferate for about 60 days. Therefore, it has been confirmed that immortalized megakaryocytes with long-term proliferation ability were established from iPS cell lines other than that used in Example 1-1 using the three factors (MYCdd/HOXA2/BCLxL).

The introduction of the CD41 promoter-MPL cassette in addition to the three factors (MYCdd/HOXA2/BCLxL) in the iPS cells resulted in an increased proliferation rate in the megakaryocyte cell line established therefrom. Furthermore, even in the presence of the sh-p21/p53 cassette, the additional introduction of the CD41 promoter-MPL cassette enhanced the proliferation rate of the megakaryocyte cell line (Figure 3).

On Days 27 and 55, a part of the megakaryocytes obtained above was recovered, the cells were washed to remove doxycycline and further cultured under an additive free condition to start the platelet induction culture. After six days of the platelet induction culture, a part of the culture medium was collected and stained with anti-human CD41 antibody, anti-human CD42b antibody, and anti-human CD42a antibody. The number of CD41, CD42b, and CD42a all-positive microparticles as platelets was counted by a flow cytometer. The platelet production efficiency based on the number of the seeded megakaryocytes at the start of the platelet induction culture is shown in Figure 4. "-" indicates less than one, "+" indicates one to twenty, and "++" indicates more than twenty platelets were produced per seeded cell.

As shown in Figure 4, platelets were produced from immortalized megakaryocytes established using the three factors (MYCdd/HOXA2/BCLxL). It has been confirmed that immortalized megakaryocytes retaining platelet production capability were established from iPS cell line different from that in Example 1-1 using the same three factors.

Introduction of the CD41 promoter-MPL cassette in addition to the three factors (MYCdd/HOXA2/BCLxL) in the iPS cells resulted in an increased number of platelets generation per the seeded megakaryocytes. Furthermore, even in the presence of the sh-p21/p53 cassette, the additional introduction of the CD41 promoter-MPL cassette enhanced number of platelets generation per the seeded megakaryocytes. (Figure 4).

Further, megakaryocytes were established from iPS cell line Ff-MH23s01 in the similar manner as above and confirmed that immortalized megakaryocytes with long-term proliferative capacity were established using the three factors (MYCdd, HOXA2, and BCLxL), just as with the iPS cell lines 585A1 and Ff-I01s01. These immortalized megakaryocytes produced platelets. Thus, this disclosure's method of establishing immortalized megakaryocytes with platelet production capacity does not rely on clones of the pluripotent stem cells used as the starting material.

A part of CD41-positive platelets produced by the immortalized megakaryocytes derived from iPS cell lines Ff-I01s01 and Ff-MH23s01 using the three factors (MYCdd/HOXA2/BCLxL) were recovered and washed to remove doxycycline. The platelets were cultured under a non-additive condition. After six days of platelet induction culture, a portion of the culture medium was collected and stained with anti-human CD42b antibody and anti-human CD42a antibody, identifying the CD42b and CD42a double-positive microparticles as platelets. Platelet activation responsiveness was evaluated using a flow cytometer with PMA or ADP as stimulants. For CD41-positive platelets, the PAC1+/CD62P+ fraction was defined as activated platelets, and its ratio was analyzed. The unstimulated condition served as a negative control.

As a result, the platelets produced from the immortalized megakaryocytes established using the three factors (MYCdd/HOXA2/BCLxL) were responsive to the platelet stimulant. Furthermore, the ratio of responsive platelets to the total produced platelets was higher compared to the platelets produced from megakaryocytes established using the conventional three factors (MYCdd/BMI1/BCLxL). The platelets produced from the immortalized megakaryocyte cell line established using the three factors (MYCdd/HOXA2/BCLxL) have a higher stimulation responsiveness compared to those produced using the conventional three factors, regardless of the clones of the pluripotent stem cells used as the starting material.

[Example 2]

[Materials and Methods]

This example was conducted in accordance with the materials and methods in Example 1.

(Example 2-1)

Proliferation assay of megakaryocytes established from iPS cell line Ff-I01s01

An expression vector that incorporates two genes (MYCdd and HOXA2) under the control of the TRE (tet responsive element) and BCL2L1 gene under the control of ubiquitin promoter was introduced in the iPS cells (Ff-I01s01), and the cells were cultured on feeder cells for 14 days to differentiate into megakaryocytes via hematopoietic progenitor cells. In some groups, human CD41 promoter-human MPL gene was also introduced.

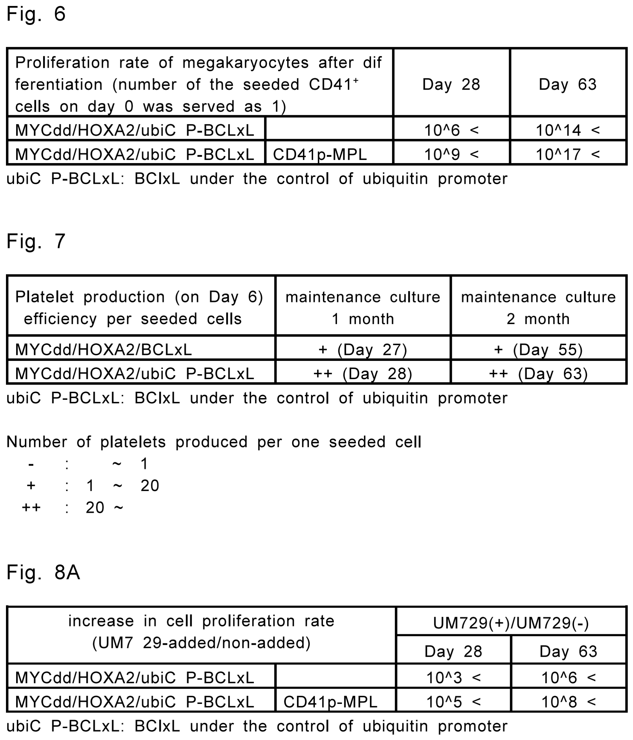

Seven days after the initiation of the differentiation culture, doxycycline was added to the iPS cells incorporated with the vector to induce the expression of the exogenous genes under the control of the TRE (tet response element) system. The cells were recovered from the culture on the feeder cells, and sub-cultured under a feeder-free condition. The day when the feeder-free culture started was designated as Day 0, and the cells were passaged in the presence of doxycycline. The cell number was counted using trypan blue. Simultaneously, the megakaryocyte-specific surface marker CD41 was detected using a flow cytometer, and the ratio of the CD41 positive cells was calculated and multiplied by the measured cell count to determine the number of megakaryocytes. The cumulative cell proliferation rate on Day 28 and Day 63 versus the cells seeded at the start of the passage is shown in Figure 6.

As shown in Figure 6, megakaryocytes induced from iPS cells using the doxycycline-induced expression system to express MYCdd and HOXA2, while expressing the BCL2L1 gene under the control of the ubiquitin promoter (MYCdd/HOXA2/UbiC P BCLxL) exhibited characteristics of immortalized megakaryocytes that could proliferate up to two months in the maintenance culture. In addition, addition of CD41 promoter-MPL gene enhanced the proliferation rate of the megakaryocyte cell line.

(Example 2-2)

Platelet production of megakaryocytes differentiated from iPS cell line Ff-I01s01

iPS cells (Ff-I01s01) incorporated with the expression vector having the three genes (MYCdd gene, HOXA2 gene, and BCL2L1 gene) were cultured on feeder cells for 14 days to induce differentiation into megakaryocytes via hematopoietic progenitor cells. In some groups, BCL2L1 gene was incorporated under the control of a ubiquitin promoter. Seven days after the initiation of the differentiation culture, doxycycline was added to induce the expression of the exogenous genes under the control of TRE. The cells were collected from the culture on feeder cells, and transferred to a feeder free condition under the presence of doxycycline. The day the feeder-free culture began was designated as Day 0, and the cells were sub-cultured in the presence of doxycycline. On Day 27 or 28 and on Day 55 or 63 of the megakaryocytes passaging culture, doxycycline was washed out and removed to induce platelet production under an additive-free condition. After six days of platelet induction culture, a part of the culture medium was collected, stained with anti-human CD41 antibody and anti-human CD42b antibody, and the number of CD41 and CD42b double positive microparticles was measured as platelets using a flow cytometer. The platelet production efficiency based on the number of seeded megakaryocytes at the start of the platelet induction culture is shown in Figure 7. "-" indicates less than one, "+" indicates one to twenty, and "++" indicates more than twenty platelets produced per seeded cell.
As shown in Figure 7, megakaryocytes (MYCdd/HOXA2/BCLxL) which were produced from iPS cells by expressing the MYCdd, HOXA2, and BCL2L1 genes using the doxycycline-inducible gene expression system as well as megakaryocytes (MYCdd/HOXA2/UbiC P BCLxL) which were produced from iPS cells by expressing MYCdd and HOXA2 genes using the doxycycline-inducible gene expression system and BCL2L1 gene under the control of the ubiquitin promoter were confirmed to produce platelets for up to two months of maintenance culture.

(Example 2-3)

Proliferation of megakaryocytes with a pyrimido-indole derivative

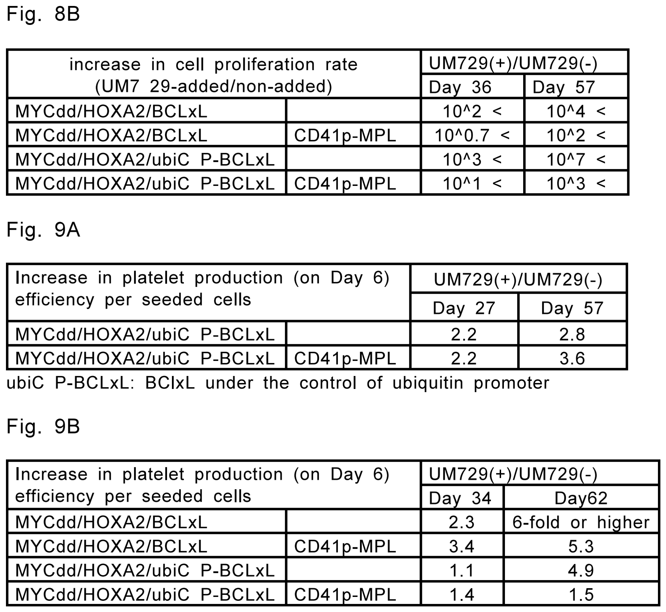

An expression vector that incorporates three genes (MYCdd, HOXA2, and BCLxL), two genes (MYCdd and BCL2L1) or in some groups, human CD41 promoter-human MPL gene was introduced in the iPS cells (Ff-I01s01), and the cells were cultured on feeder cells for 14 days to differentiate into megakaryocytes via hematopoietic progenitor cells. Seven days after the initiation of the differentiation culture, doxycycline was added to the iPS cells incorporated with the vector to induce the expression of the foreign genes under the control of the TRE (tet response element) system. The cells were recovered from the culture on the feeder cells, and sub-cultured under a feeder-free condition. The day when the feeder-free culture started was designated as Day 0, and the cells were passaged in the presence of doxycycline. About one week after the start of the subculturing, the cells were divided into two groups: one treated with UM729 at a final concentration of 1 μM and one untreated. Subculture of both groups were continued for about two months.
The cell number was counted using trypan blue. Simultaneously, the megakaryocyte-specific surface marker CD41 was detected using a flow cytometer, and the ratio of CD41 positive cells was calculated. By multiplying this ratio by the measured cell count, the megakaryocyte cell count was determined. The cumulative cell proliferation rate from Day 28 or 36 to Day 57 or 63 was calculated based on the number of the seeded cells at the start of subculturing. Figure 8 shows the increase ratio calculated by dividing the cell proliferation of the UM7 29-added group by that of the non-added group.
As shown in Figure 8A, addition of UM729 to the megakaryocyte cell line (MYCdd/HOXA2/UbiC P BCLxL) produced by expressing the MYCdd gene and HOXA2 gene, and the BCL2L1 gene under the control of ubiquitin promoter resulted in increased cell proliferation on both Day 28 and Day 63. This effect was further confirmed in a megakaryocyte cell line produced by incorporating human MPL gene under the control of the CD41 promoter.
In addition, the addition of UM729 resulted in increased cell proliferation on both Day 36 and Day 57 in megakaryocytes (MYCdd/BCLxL) produced by inducing the expression of MYCdd and the BCL2L1 genes using the doxycycline-induced expression system wherein the megakaryocytes do not include HOXA2, as well as in the megakaryocyte cells (MYCdd/UbiC P BCLxL) where the BCL2L1 gene was expressed under the control of the ubiquitin promoter (Figure 8B). This effect was also observed in the megakaryocyte cell line produced by incorporating human MPL gene under the control of CD41 promoter.

(Example 2-4)

Promotion of platelet production by a pyrimido-indole derivative

A part of the megakaryocyte cells shown in Figure 8 on Days 27, 34, 57, and 62 of subculture were collected. Cells were washed to remove doxycycline and cultured under an additive-free condition to induce the production of platelets.

After six days of the platelet induction culture, a part of the culture medium was collected and stained with anti-human CD41 antibody and anti-human CD42b antibody to measure the number of CD41 and CD42b positive microparticles as platelets using a flow cytometer. The platelet production efficiency was calculated based on the number of seeded megakaryocytes at the start of the platelet induction culture. The change in platelet production efficiency was calculated by dividing the platelet production efficiency in the UM729-added group by that in the non-added group, and is shown in Figure 9.

As shown in Figure 9, the rate of change in platelet production efficiency was greater than 1, confirming that platelet production increased in the UM729-treated group regardless of the method used to establish the megakaryocytes.

[Example 3]

Production of megakaryocytes using pyrimido-indole derivatives

[Materials and Methods]

1. The culture of iPS cells

iPS cells (Kyoto University iPS Cell Research Foundation) were seeded in StemFit AK02N medium (Ajinomoto) supplemented with iMatrix-511 silk (Nippi) at a final concentration of 0.5 ng/$\mu$L in 6-well plates under the culture condition of 37°C and 5% $CO_2$. The medium was changed the following day to remove iMatrix-511 silk. For subsequent passages, culture was continued using medium supplemented with the ROCK inhibitor Y-27632 (Nacalai Tesque, Code No. 18188-04). Medium was removed from the iPS cell culture, cells were washed with PBS, and TrypLETM Select Enzyme (Thermo Fisher Scientific, Code No. 12563011)/ 0.5 mol/L EDTA solution (pH 8.0) (Nacalai Tesque, Code No. 06894-14) in a 1:1 mixture for 5-7 minutes in an incubator (37°C, 5% $CO_2$). StemFit AK02N or StemFit AK03N medium was then added, and the cells were detached and collected using a scraper. The cell number was counted using trypan blue, and the cells were seeded at 0.5-3 $\times$ $10^4$ cells/well for passage.

For gene introduction, a cell suspension collected during subculturing was seeded in a 6-well plate at a cell density of 0.3 - 1$\times$$10^5$ cells per well.

2. Introduction of genes into iPS cells

The introduction of target genes into iPS cells was performed using the PiggyBac™ system, which utilizes transposase enzymes. The target genes introduced were three genes (three factors: MYCdd/BMI1/BCLxL): BMI1 gene, instability domain fused c-MYC gene (MYCdd gene), and BCL2L1 gene. Further, the expression induction system triggered by the addition of doxycycline was incorporated (Nakamura S., et al., Expandable megakaryocyte cell lines enable clinically applicable generation of platelets from human induced pluripotent stem cells, Cell Stem Cell, 14 (4), 535-548, 2014). Furthermore, in some groups, a shRNA expression vector targeting p21 and p53 knockdown was additionally introduced (Sone M., et al., Silencing of p53 and CDKN1A establishes sustainable immortalized megakaryocyte progenitor cells from human iPSCs, Stem Cell Reports, 16 (12), 2861-2870, 2021). To purify the iPS cells comprising the inserted genes by drug treatment, each gene expression vector was additionally equipped with one of drug resistance genes of puromycin resistance gene, hygromycin resistance gene, and neomycin resistance gene.

Introduction of the vectors into the cells was performed by using Lipofectamine™ Stem Transfection Reagent (ThermoFisher Scientific, Code No. STEM00008) according to the supplier's manual. The transposase expression vector and the other gene expression vectors were mixed and incubated for 10 minutes, and then the mixture was added to the medium in which the iPS cells were seeded. The medium was replaced with puromycin/hygromycin/G418-containing medium on the next day, and the genetically modified iPS cells were cultured in this medium until they proliferated sufficiently.

3. Differentiation of iPS cells into megakaryocytes via hematopoietic progenitor cells

The gene-modified iPS cells treated with GSK-3$\beta$ inhibitors CHIR-99021 and Y-27632 for three days were dissociated into cell aggregate fragments. The feeder cells used here were the human fibroblast cell line C3H/10T1/2 (ATCC number: CCL-226), and the gene-modified iPS cell aggregate fragments were added onto the mitomycin-treated C3H/10T1/2 cells that had been pre-seeded in a 10 cm dish.

As the basal medium, IMDM (Iscove's Modified Dulbecco's Medium) (Sigma-Aldrich, Code No.I3390) supplemented with 15% Fetal Bovine Serum(FBS)(Life Technologies, Code No.10270-106), 2mM Glutamine(Life Technologies, Code No. 25030-081), 1% Insulin / Transferrin / Selenium Solution(ITS-G)(Life Technologies, Code No.41400-045), 0.45 mM 1-Thioglycerol(Sigma-Aldrich, Code No. A6145), 50 $\mu$g/mL L-Ascorbic Acid(Sigma-Aldrich, Code No. A4544) was prepared.

The genetically modified iPS cells were cultured in the basal medium supplemented with 20 ng/mL VEGF (Fujifilm Wako Pure Chemical Industries, Ltd., Code No. 226-01786) under conditions of 37°C, 5-8% $O_2$, and 5% $CO_2$.

On the fourth day of culture, the medium was replaced with the basal medium supplemented with 20 ng/mL of VEGF, 10 U/mL of Heparin (Aiwai Pharma), 50 ng/mL of bFGF (Wako Pure Chemical Industries, Code No. 068-04544), and 10 $\mu$M of SB431542 (Wako Pure Chemical Industries, Code No. 037-24293), and the cells were cultured under the condition of 37°C, 5-8% $O_2$, and 5% $CO_2$.

On the seventh day of culture, the medium was replaced with the basal medium supplemented with 20 ng/mL of VEGF and 10 U/mL of Heparin, and cultured under the condition of 37°C, 20% $O_2$, and 5% $CO_2$.

On the 11th day of culture, the basal medium supplemented with 20 ng/mL of VEGF and 0.75 $\mu$M of SR-1 (Stemregenin-1) was added or replaced, and the cells were cultured under the condition of 37°C, 20% $O_2$, and 5% $CO_2$.

On the 14th day, the floating cells were collected from the culture on feeder cells and suspended in the basal medium supplemented with 0.2 $\mu$g/mL TPO mimetic compound, 50 ng/mL SCF, 1 $\mu$M UM729 (MERCK Code No. ATEH97ECEE61), and 1 $\mu$g/mL doxycycline. Then the cells were cultured under a feeder-free condition at 37°C, with 20% $O_2$ and 5% $CO_2$. The cells were continuously subcultured using the same medium to differentiate into imMKCL.

4. Evaluation of the proliferative capacity of megakaryocytes

The start date of the feeder-free culture was designated as Day 0, and the cell count measurement and the detection of the megakaryocyte-specific surface marker CD41 by flow cytometry were regularly performed.

The cell count was measured over time using trypan blue, and the cumulative proliferation rate from Day 0 was calculated based on the proliferation rate derived from the number of the seeded cells. Simultaneously, the proliferation rate of the megakaryocytes was determined by multiplying the cumulative proliferation rate of the megakaryocytes by the ratio of megakaryocyte in the total cells calculated from the cells positive for the surface marker specific to megakaryocytes. The cumulative proliferation rate of the megakaryocytes at the nth passage was calculated using the formula below.

[Math 2]

cumulative proliferation rate of the megakaryocytes at the nth passage

$$= \left( \prod_{k=1}^{n} \frac{live\ cell\ density\ collected\ at\ the\ kth\ passage}{cell\ dencity\ seeded\ at\ the\ k\text{-}1\ th\ passage} \right) \times ratio\ of\ CD41\ positive\ cells\ at\ the\ nth\ passage$$

In the above formula, the density for the first generation (0th passage) is the cell density on Day 0.

5. Evaluation of platelet production ability and function of the generated platelets

The cells in the proliferation culture of the gene-introduced megakaryocytes were washed to remove doxycycline and then, cultured in a medium with no doxycycline thereby the forced expression of BM11 gene, MYCdd gene and BCL2L1 gene controlled by the TRE (tet Response Element) was stopped and the maturation of the megakaryocytes and the production of platelets were induced. IMDM media supplemented with 5% human plasma, 2 mM glutamine, 1% ITS-G, 0.45 mM 1-thioglycerol, 50 $\mu$g/mL L-ascorbic acid, 50 ng/mL SCF, 0.2 $\mu$g/mL TPO mimetic compound, 15 $\mu$M KP-457 (Kaken Pharmaceutical) and 10 $\mu$M Y-27632 (Nacalai Tesque, Code No. 18188-04), as well as 0.75 $\mu$M SR-1 (Stemregenin-1) or 0.1 $\mu$M AhR inhibitor (the concentrations are final concentrations) were prepared. Platelet production culture was conducted by seeding megakaryocytes at a density of 1 x $10^5$ cells/mL in a 125 mL culture flask, with 25 mL/flask, and performing shaking culture. The shaking culture was conducted using the LT-X (Lab-Therm) (Kuhner) or S41i (Eppendorf) under conditions of 37°C and 5% $CO_2$ at a shaking speed of 100 rpm.

The cells were cultured under the above conditions for 6 days, then, a part of the cell suspension was analyzed by BD FACSVerse™ (BD Biosciences) to measure the number of produced platelets and to evaluate the platelet function.

The following antibodies were used to stain the particles to measure the number of produced platelets: APC-labeled anti-CD41 antibody (BioLegend, Code No. 303710)

The PE-labeled anti-CD42b antibody (BioLegend, Code No. 303906) eFluor™ 450-labeled anti-CD42a antibody (Thermo Fisher, Code No. 48-0428-42) The particles were stained with the antibodies and after 30 minutes' staining, the platelet count (gated as micro-particles by FSC/SSC, either CD41/CD42b double positive or CD41/CD42a/CD42b positive) was measured using TruCOUNT ™ (BD Biosciences, Code No. 340334). Flow cytometry data was analyzed using FlowJo ™ Version 10 (BD Biosciences) to calculate the number of the produced platelets. The data was calculated as the number of platelets produced per megakaryocyte, referencing the number of megakaryocytes seeded at the start of the platelet production culture.

To evaluate the function of the platelets, the platelets were stimulated by the following two types of stimulants.

Stimulant 1: 0.02 to 0.4 $\mu$M PMA
Stimulant 2:100 $\mu$M ADP/40 $\mu$M Thrombin Receptor Activator Peptide 6(TRAP6)

After the addition of the stimulant, the samples were stained with the following antibodies:

APC-labeled anti-CD41 antibody (BioLegend, Code No. 303710)
The PE-labeled anti-CD62P antibody (BioLegend, Code No. 304906)
FITC-labeled anti-PAC-1 antibody (BD Bioscience, Code No. 34507)

Measurements were conducted using BD FACSVerse™ after 30 minutes.

The three factors MYCdd/BMI1/BCLxL were introduced under the control of the TRE (tet responsive expression) in iPS cells (TkDN-sev2) (Kyoto University iPS Cell Research Foundation) using the PiggyBac™ system which utilizes transposase enzyme, and the cells were differentiated into immortalized megakaryocytes (imMKCL) according to a previously reported method. Then, the Yamanaka factors were introduced into the obtained immortalized megakaryocytes to generate iPS cells (Re MK iPS) (Seo et al., Blood Advances, 2(17):2262-2272 (2018)). The Re MK iPS cells have the three genes MYCdd, BMI1, and BCL2L1 under the control of the TRE (tet expression induction system), and the addition of doxycycline induces the expression of these genes.

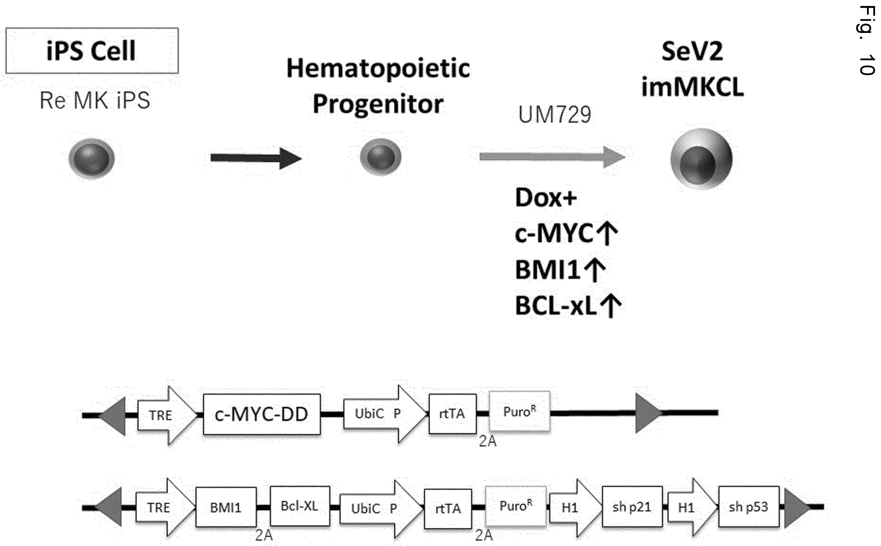

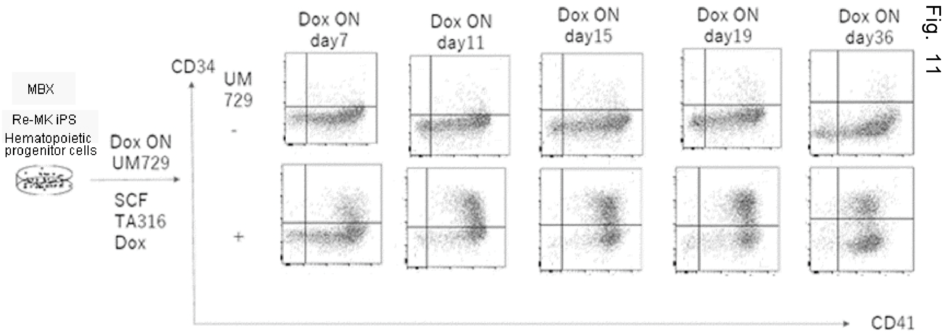

The induced pluripotent stem cells (Re MK iPS) having the three genes c-MYC, BMI1, and BCL2L1 were differentiated into hematopoietic progenitor cells (Re MK iPS hematopoietic progenitor cells). The obtained progenitor cells were then cultured in a differentiation medium supplemented with 50 ng/ml SCF, 200 ng/ml TA-316, 1 $\mu$g/ml doxycycline, and (+/-) 1 $\mu$M UM729 to induce differentiation into immortalized megakaryocytes (imMKCL). A schematic diagram showing the differentiation from induced pluripotent stem cells to immortalized megakaryocytes via hematopoietic progenitor cells, as well as a schematic diagram of the constructs introduced into the cells, are shown in Figure 10. Cells were collected on days 7, 11, 15, 19, and 36 after the start of feeder-free culture in the presence of doxycycline, stained with CD41a-APC and CD34-FITC antibodies, and the surface markers were analyzed by flow cytometry. As a result, in the UM729-treated group, a population of CD34/CD41 positive cells was observed (Figure 11). It is known that CD34/CD41 double-positive megakaryocytes have a high platelet production capacity (Strassel C et al., Blood (2016) 127 (18): 2231-2240). Therefore, the addition of UM729 during the differentiation of hematopoietic progenitor cells into immortalized megakaryocytes leads the induction of CD34/41 double-positive cells.

Next, the established imMKCL (60-70 days after the initiation of the megakaryocyte induction culture) was cultured for 7 days in the differentiation medium supplemented with 50 ng/ml SCF, 200 ng/ml TA-316, 1 $\mu$g/ml doxycycline, and 1 $\mu$M UM729. Cells were harvested on days 3 and 7 of culture, stained with CD41a-APC and CD34-FITC antibodies, and analyzed by flow cytometry for the surface markers. The results showed that the cells were not differentiated into the CD34/CD41-positive cell population (Figure 12). In the group wherein the imMKCL were induced under the condition of without UM729, and then, UM729 was added to the imMKCL after 60 days of the start of the induction of megakaryocytes, neither 3-day nor 6-day induction induced CD34/41 double-positive cells.

iPS cells (Re MK iPS) into which the three genes -c-MYC, BMI1, and BCL2L1- were introduced under TRE (tet expression induction system) regulation were differentiated into hematopoietic progenitor cells (Re MK iPS hematopoietic progenitor cells). These progenitor cells were then cultured in the differentiation medium supplemented with 50 ng/ml SCF, 200ng/ml TA-316, and 1$\mu$g/ml doxycycline, supplemented with (+/)0.75$\mu$M SR-1, (+/-)35nM a UM729 analog UM171, and (+/-)1$\mu$M UM729, to induce differentiation into imMKCL. Cells were harvested on day 7 of feeder-free culture, stained with CD41a-APC and CD34-FITC antibodies, and analyzed by flow cytometry for surface markers. The results showed that the groups supplemented with UM171 and UM729 were induced to CD34/CD41-positive cells, whereas SR-1 failed to induce them (Figure 13). This indicates that the addition of SR-1 instead of UM729 does not induce CD34/CD41 double-positive cells.

Next, cells were harvested on day 11 of imMKCL induction, stained with CD41a-APC and CD34-FITC antibodies, and the CD34/41-positive population was FACS sorted. When cultured with (+/-) 1$\mu$M UM729, the CD34/41 cell population was maintained in the UM729-added group (Figure 14). In contrast, the CD34/41-positive cell population could not be maintained in the UM729-free group. UM729 was shown to contribute to the maintenance of CD34- and CD41- double positive cells.

The platelet production capacities of imMKCL in the UM729(+/-) groups were evaluated. imMKCL on day 11 of megakaryocyte induction culture were cultured in a maturation medium using T125 flasks. On day 6 of megakaryocyte maturation culture, the culture supernatant was collected, stained with CD41a-APC and CD42b-PE, and platelet counts were measured by flow cytometry (Figure 15). The results showed that platelet production capacity increased more than two-fold compared to the UM729-free group (Figure 16 and Figure 17, left panel). Further, the platelet production capacity of megakaryocytes on days 11, 19, 27, 49, and 91 of megakaryocyte induction culture were measured and confirmed that no significant decrease in the platelet production capacity was observed even after long-term culture. This result indicates that the capacity was maintained over extended periods (Figure 17, right panel). CD34/41 double-positive cells obtained by the differentiation using a UM729-containing medium exhibited high platelet production capacity, and this high capacity is maintained over the long term.

imMKCL on Day 60 of UM729(+/-) megakaryocyte induction culture (feeder-free culture) and megakaryocytes on Day 3 of maturation culture were collected and observed using BSE-SEM electron microscopy. CD34/41-positive imMKCL treated with UM729 retained more mitochondria compared to the UM729-untreated group (Figure 18). Furthermore, CD34/41-positive megakaryocytes cultured for 3 days in UM729-containing medium retained more intracellular granules compared to the UM729-free group (Figure 19). Therefore, CD34/41 double-positive cells induced to differentiate in UM729-containing medium were shown to have higher levels of mitochondria and intracellular granules.

CD34/41 positive megakaryocytes treated with UM729 were stained with CD45RA-PerCP, CD38-PerCP, CD34-FITC, CD90-BV420, CD49f-PE, and CD41a APC, and the surface markers were examined by flow cytometry. The CD34/41 positive megakaryocytes retained the hematopoietic stem cell markers CD34+CD38-CD90+CD49f+CD201+ (Figure 20). The CD34/41 double positive cells obtained by differentiation in a medium comprising UM729 also express hematopoietic stem cell markers.

Reproducibility experiments were conducted to determine whether other iPS cell lines could also be differentiated into imMKCL. Hematopoietic progenitor cells were induced from various iPS cell lines, 15M41, MH09S01, MH15S01, MH15S02 and MH23S01. The three genes-c-MYC, BMI1, and BCL2L1-were introduced in the iPS cells. The obtained progenitor cells were then cultured in a differentiation medium supplemented with SCF 50 ng/ml, TA-316 200ng/ml, doxycycline 1µg/ml, and (+/-)UM729 1µM to induce differentiation into megakaryocytes (imMKCL). Cells on day 7 of megakaryocyte induction were stained with CD41a-APC and CD34-FITC antibodies, and surface markers were analyzed by flow cytometry. The results showed that a CD34/41 cell population was observed in all iPS cell lines (Figure 21). Therefore, it was experimentally demonstrated that other iPS cell lines were differentiated similarly into CD34/41 double-positive cells.

Additionally, cells on day 7 of megakaryocyte induction were stained with CD41a-APC and CD34-FITC antibodies. The CD34/41-positive population was sorted by FACS and cultured with (+/-) UM729 at 1µM. Cells on day 21 of megakaryocyte induction were harvested, stained with CD41a-APC and CD34-FITC antibodies, and analyzed by flow cytometry for surface markers. The results showed that a CD34/41-positive cell population was observed in all iPS cell lines (Figure 22). Culturing in UM729-supplemented medium successfully maintained this population in other iPS cells as well. Thus, UM729 was shown to contribute to the maintenance of CD34/41 double-positive cells in various iPS cell lines.

Furthermore, the obtained imMKCLs were cultured in a maturation medium, and their platelet production capacity was measured. On day 6 of megakaryocyte maturation culture, the culture supernatant was collected, stained with CD41a-APC and CD42b-PE, and platelet counts were measured by flow cytometry (Figures 23 and 24). Compared to the UM729-free group, the UM729-added group showed increased platelet production capacity in all iPS clones (Figure 25). Therefore, it was demonstrated that CD34/41 double-positive cells obtained by differentiation induction in UM729-containing medium also exhibit high platelet production capacity in other iPS cell lines.

**Claims**

1. A method for producing megakaryocytes from hematopoietic progenitor cells, which comprises the step of culturing the hematopoietic progenitor cells while forcibly expressing in the hematopoietic progenitor cells an oncogene, a homeobox gene, and an apoptosis inhibitor gene.

2. The method according to claim 1, wherein a gene encoding a cytokine receptor or a cell growth promoting factor under the control of a promoter specific to megakaryocytes or platelets is also forcibly expressed in the hematopoietic progenitor cells.

3. The method according to claim 1 or 2, wherein the hematopoietic progenitor cells are cultured in a medium comprising a pyrimido-indole derivative.

4. The method according to any one of claims 1-3, further comprises the step of differentiating pluripotent stem cells into hematopoietic progenitor cells.

5. A method for producing mature megakaryocytes, which comprises the steps of:

producing megakaryocytes by the method according to any one of claims 1-4, and
culturing the megakaryocytes while stopping the forced expression of the oncogene, the homeobox gene and the apoptosis inhibitor gene.

6. A method for producing platelets, which comprises the steps of
producing mature megakaryocytes by the method according to claim 5, and culturing the obtained mature megakaryocytes.

7. Megakaryocytes or mature megakaryocytes, wherein an exogenous oncogene, an exogenous homeobox gene and an exogenous apoptosis inhibitor gene are integrated in their chromosome.

8. A method for producing megakaryocytes from hematopoietic progenitor cells, **characterized by** forcibly expressing in the hematopoietic progenitor cells a gene encoding a cytokine receptor or a cell growth promoting factor under the control of a promoter specific to megakaryocyte or platelet.

9. The method according to claim 8, wherein the hematopoietic progenitor cells are cultured in a medium comprising a pyrimido-indole derivative.

10. The method according to claim 8 or 9, which comprises the step of culturing hematopoietic progenitor cells while forcibly expressing an apoptosis inhibitor gene and an oncogene in the hematopoietic progenitor cells.

11. A method for producing mature megakaryocytes, which comprises the steps of:

producing megakaryocytes by the method according to claim 10, and
culturing the megakaryocytes while stopping the forced expression of the apoptosis inhibitor gene and/or the oncogene.

12. A method for producing platelets, which comprises the steps of:

producing mature megakaryocytes by the method according to claim 11, and
culturing the obtained mature megakaryocytes.

13. A method for producing megakaryocytes form hematopoietic progenitor cells, which comprises the step of culturing the hematopoietic progenitor cells in a medium comprising a pyrimido-indole derivative.

14. The method according to claim 13, which comprises the step of
culturing the hematopoietic progenitor cells while forcibly expressing in the hematopoietic progenitor cellsa gene selected from the group consisting of a gene that suppresses the expression of p16 gene or p19 gene, a gene that suppresses the expression of Ink4a/Arf gene, and a polycomb gene, as well as an apoptosis inhibitor gene and an oncogene.

15. A method for mature megakaryocytes, which comprises the steps of:

producing megakaryocytes by the method according to claim 14, and
culturing the obtained megakaryocytes while stopping the forced expression of the gene selected from the group consisting of a gene that suppresses the expression of p16 gene or p19 gene, a gene that suppresses the expression of Ink4a/Arf gene, and a polycomb gene, as well as an apoptosis inhibitor gene and/or an oncogene in the megakaryocytes.

16. A method for producing platelets, which comprises the steps of
producing mature megakaryocytes by the method according to claim 15, wnd culturing the obtained mature megakaryocytes.

17. A kit for megakaryocytes, mature megakaryocytes or platelets, which comprises a vector having an oncogene, a homeobox gene and an apoptosis inhibitor gene.

Fig. 1A

MYCdd/HOXA2/BCLxL

Fig. 1B

Fig. 2A

| Days of subculturing from the day when the hematopoietic progenitor cells were collected | 32 days |
|---|---|
| Platelet production culture period | 6 days |
| Number of produced platelets /Number of seeded cells | 46.7 |

Fig. 2B

Ratio of activated platelets

Fig. 3

| Proliferation rate of megakaryocytes from just after the differentiation (Day 0) CD41+ cells seeded on day 0 was served as 1 | | | Day 28 Ff-I01s01 | Day 57 Ff-I01s01 |
|---|---|---|---|---|
| MYCdd/HOXA2/BCLxL | | | 10^5 < | 10^10 < |
| MYCdd/HOXA2/BCLxL | CD41p-MPL | | 10^10 < | 10^19 < |
| MYCdd/HOXA2/BCLxL | | sh-p21/p53 | 10^5 < | 10^16 < |
| MYCdd/HOXA2/BCLxL | CD41p-MPL | sh-p21/p53 | 10^10 < | 10^19 < |

## Fig. 4

| Platelet production efficiency (6 days) per seeded megakaryocytes | | | Day 27 Ff-I01s01 | Day 55 Ff-I01s01 |
|---|---|---|---|---|
| MYCdd/HOXA2/BCLxL | | | + | + |
| MYCdd/HOXA2/BCLxL | CD41p-MPL | | ++ | ++ |
| MYCdd/HOXA2/BCLxL | | sh-p21/p53 | + | + |
| MYCdd/HOXA2/BCLxL | CD41p-MPL | sh-p21/p53 | ++ | ++ |

Number of platelets produced per one seeded cell

- : ~ 1
+ : 1 ~ 20
++ : 20 ~

## Fig. 5

Fig. 6

| Proliferation rate of megakaryocytes after differentiation (number of the seeded CD41+ cells on day 0 was served as 1) | | Day 28 | Day 63 |
|---|---|---|---|
| MYCdd/HOXA2/ubiC P-BCLxL | | 10^6 < | 10^14 < |
| MYCdd/HOXA2/ubiC P-BCLxL | CD41p-MPL | 10^9 < | 10^17 < |

ubiC P-BCLxL: BClxL under the control of ubiquitin promoter

Fig. 7

| Platelet production (on Day 6) efficiency per seeded cells | maintenance culture 1 month | maintenance culture 2 month |
|---|---|---|
| MYCdd/HOXA2/BCLxL | + (Day 27) | + (Day 55) |
| MYCdd/HOXA2/ubiC P-BCLxL | ++ (Day 28) | ++ (Day 63) |

ubiC P-BCLxL: BClxL under the control of ubiquitin promoter

Number of platelets produced per one seeded cell

  -  :    ~ 1
  +  : 1 ~ 20
 ++  : 20 ~

Fig. 8A

| increase in cell proliferation rate (UM7 29-added/non-added) | | UM729(+)/UM729(-) | |
|---|---|---|---|
| | | Day 28 | Day 63 |
| MYCdd/HOXA2/ubiC P-BCLxL | | 10^3 < | 10^6 < |
| MYCdd/HOXA2/ubiC P-BCLxL | CD41p-MPL | 10^5 < | 10^8 < |

ubiC P-BCLxL: BClxL under the control of ubiquitin promoter

Fig. 8B

| increase in cell proliferation rate (UM7 29-added/non-added) | | UM729(+)/UM729(-) | |
|---|---|---|---|
| | | Day 36 | Day 57 |
| MYCdd/HOXA2/BCLxL | | 10^2 < | 10^4 < |
| MYCdd/HOXA2/BCLxL | CD41p-MPL | 10^0.7 < | 10^2 < |
| MYCdd/HOXA2/ubiC P-BCLxL | | 10^3 < | 10^7 < |
| MYCdd/HOXA2/ubiC P-BCLxL | CD41p-MPL | 10^1 < | 10^3 < |

Fig. 9A

| Increase in platelet production (on Day 6) efficiency per seeded cells | | UM729(+)/UM729(-) | |
|---|---|---|---|
| | | Day 27 | Day 57 |
| MYCdd/HOXA2/ubiC P-BCLxL | | 2.2 | 2.8 |
| MYCdd/HOXA2/ubiC P-BCLxL | CD41p-MPL | 2.2 | 3.6 |

ubiC P-BCLxL: BClxL under the control of ubiquitin promoter

Fig. 9B

| Increase in platelet production (on Day 6) efficiency per seeded cells | | UM729(+)/UM729(-) | |
|---|---|---|---|
| | | Day 34 | Day62 |
| MYCdd/HOXA2/BCLxL | | 2.3 | 6-fold or higher |
| MYCdd/HOXA2/BCLxL | CD41p-MPL | 3.4 | 5.3 |
| MYCdd/HOXA2/ubiC P-BCLxL | | 1.1 | 4.9 |
| MYCdd/HOXA2/ubiC P-BCLxL | CD41p-MPL | 1.4 | 1.5 |

Fig. 10

Fig. 11

Fig. 12

Fig. 13

Fig. 14

Fig. 15

UM+ 34/41 sort
imMKCL

Gene off

Count platelets

Fig. 16

UM+ 34/41 sort
platelet

CD42b

−     +

CD41

Fig. 17

Platelet count

Fig. 18

Dox ON

UM+          UM-

Fig. 19

Dox OFF day3

UM+ UM−

Fig. 20

Fig. 21

Fig. 22

Fig. 23

Fig. 24

15M41 proplatelet

Fig. 25

PLT/MGK

UM-
UM+

# EP 4 692 332 A1

## INTERNATIONAL SEARCH REPORT

| | International application No. |
|---|---|
| | **PCT/JP2024/013612** |

### A. CLASSIFICATION OF SUBJECT MATTER

*C12N 5/10*(2006.01)i; *C12N 5/078*(2010.01)i; *C12N 15/12*(2006.01)i; *C12N 15/19*(2006.01)i; *C12N 15/63*(2006.01)i
FI:   C12N5/10; C12N5/078 ZNA; C12N15/12; C12N15/19; C12N15/63 Z

According to International Patent Classification (IPC) or to both national classification and IPC

### B. FIELDS SEARCHED

Minimum documentation searched (classification system followed by classification symbols)

C12N5/10; C12N5/078; C12N15/12; C12N15/19; C12N15/63

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2014/123242 A1 (KYOTO UNIVERSITY) 14 August 2014 (2014-08-14) claims, paragraphs [0074], [0079], [0092], examples | 1-17 |
| Y | VIEILLE-GROSJEAN, Isabelle et al., Lineage and stage specific expression of HOX1 genes in the human hematopoietic system, Biochemical and Biophysical Research Communications, 1992, vol. 183, no. 3, pages 1124-1130 abstract, page 1125, lines 1-17, fig. 2 | 1-7, 17 |
| Y | SUNOHARA, Masataka et al., Role of promoter element in c-mpl gene expression induced by TPO, Okajimas Folia Anatomica Japonica, 2013, vol. 89, no. 4, pages 131-135 abstract | 1-12, 17 |
| Y | WO 2016/143836 A1 (MEGAKARYON CORPORATION) 15 September 2016 (2016-09-15) claims, paragraph [0013] | 1-12, 17 |
| X | JP 2018-516089 A (HEMA-QUEBEC) 21 June 2018 (2018-06-21) paragraphs [0003], [0008], [0050], examples 2, 3, table 1 | 13 |
| Y | paragraphs [0003], [0008], [0050], examples 2, 3, table 1 | 1-17 |

☐ Further documents are listed in the continuation of Box C.        ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **10 June 2024** | **25 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**65**

**INTERNATIONAL SEARCH REPORT**

| International application No. |
| --- |
| **PCT/JP2024/013612** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/JP2024/013612**

| Patent document cited in search report | | | Publication date (day/month/year) | Patent family member(s) | | | Publication date (day/month/year) |
|---|---|---|---|---|---|---|---|
| WO | 2014/123242 | A1 | 14 August 2014 | US | 2016/0002599 | A1 | |
| | | | | claims, paragraphs [0152], [0157], [0175], examples | | | |
| | | | | EP | 2955223 | A1 | |
| WO | 2016/143836 | A1 | 15 September 2016 | US | 2018/0044634 | A1 | |
| | | | | claims, paragraph [0052] | | | |
| | | | | EP | 3296393 | A1 | |
| | | | | CN | 107532145 | A | |
| JP | 2018-516089 | A | 21 June 2018 | US | 2018/0147239 | A1 | |
| | | | | paragraphs [0003], [0006], [0081], examples 2, 3, table 1 | | | |
| | | | | EP | 3303570 | A1 | |
| | | | | WO | 2016/191879 | A1 | |
| | | | | KR | 10-2018-0023947 | A | |
| | | | | CN | 107922926 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011034073 A
- WO 2012157586 A
- WO 2014123242 A
- WO 2021075568 A
- WO 2007069666 A
- WO 2011007900 A

**Non-patent literature cited in the description**

- *Cell Stem Cell*, 03 April 2014, vol. 144, 535-48
- *Cell*, 26 July 2018, vol. 174 (3), 636-648
- **TAKAYAMA N. et al.** *J Exp Med.*, 2010, 2817-2830
- **CHADWICK et al.** *Blood*, 2003, vol. 102, 906-15
- **VIJAYARAGAVAN et al.** *Cell Stem Cell*, 2009, vol. 4, 248-62
- **SAEKI et al.** *Stem Cells*, 2009, vol. 27, 59-67
- **NIWA A et al.** *J Cell Physiol.*, vol. 221 (2), 367-77
- **J. A. THOMSON et al.** *Science*, 1998, vol. 282, 1145-1147
- **J. A. THOMSON et al.** *Proc. Natl. Acad. Sci. USA*, 1995, vol. 92, 7844-7848
- **J. A. THOMSON et al.** *Biol. Reprod.*, 1996, vol. 55, 254-259
- **J.A. THOMSON ; V.S. MARSHALL.** *Curr. Top. Dev. Biol.*, 1998, vol. 38, 133-165
- **T. WAKAYAMA et al.** *Science*, 2001, vol. 292, 740-743
- **S. WAKAYAMA et al.** *Biol. Reprod.*, 2005, vol. 72, 932-936
- **J. BYRNE et al.** *Nature*, 2007, vol. 450, 497-502
- **M. KANATSU-SHINOHARA et al.** *Biol. Reprod.*, 2003, vol. 69, 612-616
- **K. SHINOHARA et al.** *Cell*, 2004, vol. 119, 1001-1012
- **Y. MATSUI et al.** *Cell*, 1992, vol. 70, 841-847
- **J. L. RESNICK et al.** *Nature*, 1992, vol. 359, 550-551
- **K. TAKAHASHI ; S. YAMANAKA.** *Cell*, 2006, vol. 126, 663-676
- **K. TAKAHASHI et al.** *Cell*, 2007, vol. 131, 861-872
- **J. YU et al.** *Science*, 2007, vol. 318, 1917-1920
- **NAKAGAWA, M. et al.** *Nat. Biotechnol.*, 2008, vol. 26, 101-106
- **BANASZYNSKI et al.** *Cell*, 2006, vol. 126, 995
- **FRASER MJ et al.** *Insect Mol Biol.*, May 1996, vol. 5 (2), 141-51
- **WILSON MH et al.** *Mol Ther.*, January 2007, vol. 15 (1), 139-45
- **NAKAZAWA Y et al.** *J Immunother*, 2009, vol. 32, 826-836
- **NAKAZAWA Y et al.** *J Immunother*, 2013, vol. 6, 3-10
- **IVICS Z ; HACKETT PB ; PLASTERK RH ; IZSVAK Z.** *Cell*, 1997, vol. 91, 501-510
- **MISKEY C ; IZSVAK Z ; PLASTERK RH ; IVICS Z.** *Nucleic Acids Res*, 2003, vol. 31, 6873-6881
- **KOGA A ; INAGAKI H ; BESSHO Y ; HORI H.** *Mol Gen Genet.*, 10 December 1995, vol. 249 (4), 400-5
- **KOGA A ; SHIMADA A ; KUROKI T ; HORI H ; KUSUMI J ; KYONO-HAMAGUCHI Y ; HAMAGU-CHI S.** *J Hum Genet.*, 07 June 2007, vol. 52 (7), 628-35
- **KOGA A ; HORI H ; SAKAIZUMI M.** *Mar Biotechnol*, 2002, vol. 4, 6-11
- **JOHNSON HAMLET MR ; YERGEAU DA ; KU-LIYEV E ; TAKEDA M ; TAIRA M ; KAWAKAMI K ; MEAD PE.** *Genesis*, 2006, vol. 44, 438-445
- **CHOO BG ; KONDRICHIN I ; PARINOV S ; EMELYANOV A ; GO W ; TOH WC ; KORZH V.** *BMC Dev Biol*, 2006, vol. 6, 5
- **SAHA S ; NAKAZAWA Y ; HUYE LE ; DOHERTY JE ; GALVAN DL ; ROONEY CM ; WILSON MH.** *J Vis Exp*, 05 November 2012, vol. 69, e4235
- **URLINGER, S. et al.** *Proc. Natl. Acad. Sci. USA*, 2000, vol. 97 (14), 7963-7968
- **GULLIKSSON, H. et al.** *Transfusion*, 1992, vol. 32, 435-440

- **NAKAMURA S. et al.** Expandable megakaryocyte cell lines enable clinically applicable generation of platelets from human induced pluripotent stem cells. *Cell Stem Cell*, 2014, vol. 14 (4), 535-548

- **SONE M. et al.** Silencing of p53 and CDKN1A establishes sustainable immortalized megakaryocyte progenitor cells from human iPSCs. *Stem Cell Reports*, 2021, vol. 16 (12), 2861-2870
- **SEO et al.** *Blood Advances*, 2018, vol. 2 (17), 2262-2272
- **STRASSEL C et al.** *Blood*, 2016, vol. 127 (18), 2231-2240